# EUROPEAN PATENT SPECIFICATION

(11) **EP 2 625 195 B1**
(45) Date of publication and mention of the grant of the patent: **06.04.2016**
(21) Application number: 11779194.7
(22) Date of filing: 07.10.2011
(51) Int. Cl.: C07K 14/47, G01N 33/50, C12Q 1/68, C12N 15/113, A61K 38/17, A61K 39/395, A61K 48/00

(54) **CANCER TARGETS**
KREBSZIELMOLEKÜLE
CIBLES POUR LE TRAITEMENT DE CANCERS

(30) Priority: 08.10.2010 GB 201016995
(43) Date of publication of application: 14.08.2013
(73) Proprietor: University Of Dundee, Dundee DD1 4HN (GB)
(72) Inventor: SOUTH, Andrew, Dundee DD1 9SY (GB); POURREYRON, Celine, Dundee DD1 9SY (GB); WATT, Stephen, Dundee DD1 9SY (GB); FOERSTER, John, Dundee DD1 9SY (GB)
(74) Representative: Gibbs, Richard
(86) International application number: PCT/GB2011/001455
(87) International publication number: WO 2012/046006

(56) References cited:
- US-A1- 2006 147 930
- NINDL INGO ET AL: "Identification of differentially expressed genes in cutaneous squamous cell carcinoma by microarray expression profiling", MOLECULAR CANCER, BIOMED CENTRAL, LONDON, GB, vol. 5, no. 1, 8 August 2006 (2006-08-08), page 30, XP021018292, ISSN: 1476-4598, DOI: 10.1186/1476-4598-5-30 cited in the application
- DANG CHANTIP ET AL: "Identification of dysregulated genes in cutaneous squamous cell carcinoma.", ONCOLOGY REPORTS SEP 2006 LNKD- PUBMED:16865251, vol. 16, no. 3, September 2006 (2006-09), pages 513-519, XP002667334, ISSN: 1021-335X
- HAIDER ASIFA S ET AL: "Genomic analysis defines a cancer-specific gene expression signature for human squamous cell carcinoma and distinguishes malignant hyperproliferation from benign hyperplasia.", THE JOURNAL OF INVESTIGATIVE DERMATOLOGY APR 2006 LNKD- PUBMED:16470182, vol. 126, no. 4, April 2006 (2006-04), pages 869-881, XP002667335, ISSN: 0022-202X cited in the application
- ONDO ET AL: "Topical combination therapy for cutaneous squamous cell carcinoma in situ with 5-fluorouracil cream and imiquimod cream in patients who have failed topical monotherapy", JOURNAL OF THE AMERICAN ACADEMY OF DERMATOLOGY, C.V. MOSBY, ST. LOUIS, MO, US, vol. 55, no. 6, 14 November 2006 (2006-11-14), pages 1092-1094, XP005725747, ISSN: 0190-9622, DOI: 10.1016/J.JAAD.2006.06.031
- K YAMAGUCHI ET AL: "C20orf20 (MRG-binding protein) as a potential therapeutic target for colorectal cancer", BRITISH JOURNAL OF CANCER, vol. 102, no. 2, 19 January 2010 (2010-01-19), pages 325-331, XP055016595, ISSN: 0007-0920, DOI: 10.1038/sj.bjc.6605500 cited in the application
- CAI YONG ET AL: "Identification of new subunits of the multiprotein mammalian TRRAP/TIP60-containing histone acetyltransferase complex.", THE JOURNAL OF BIOLOGICAL CHEMISTRY 31 OCT 2003 LNKD- PUBMED:12963728, vol. 278, no. 44, 31 October 2003 (2003-10-31), pages 42733-42736, XP002667336, ISSN: 0021-9258 cited in the application
- DATABASE EMBL [Online] 26 August 2010 (2010-08-26), "Sequence 1062030 from Patent EP2213738.", XP002667337, retrieved from EBI accession no. EMBL:HH185315 Database accession no. HH185315
- S A WATT ET AL: "Integrative mRNA profiling comparing cultured primary cells with clinical samples reveals PLK1 and C20orf20 as therapeutic targets in cutaneous squamous cell carcinoma", ONCOGENE, vol. 30, no. 46, 23 May 2011 (2011-05-23), pages 4666-4677, XP055016610, ISSN: 0950-9232, DOI: 10.1038/onc.2011.180

## Description

### FIELD OF THE INVENTION

The present invention provides novel targets potentially useful in the treatment of cancer - in particular cutaneous squamous cell carcinoma (cSCC). Furthermore, the invention provides compounds useful in the treatment of cSCC as well as methods for identifying other potential therapeutic targets.

### BACKGROUND OF THE INVENTION

Keratinocyte skin cancers are the most common neoplasm in Caucasian populations with an estimated incidence of over 100,000 per year in the UK (littp://info.cancerresearchuk.org/cancerstats/incidence/commoncancers/index.htm) and a cumulative risk of 70% in a 70y old Australian male (Staples et al 2002). Cutaneous SCC (cSCC) is the most common skin cancer with malignant potential and patients presenting with regional metastasis have a poor outcome: 5 year survival in this group is 25-50% (Epstein et al, 1968; Veness et al, 2005). In the UK, greater than 1 in 4 skin cancer deaths can be attributed to non-melanoma skin cancer, principally cSCC (ISD Scotland, http://www.isdscotland.org/isd/183.html). High risk groups exist where cSCC is a major complication leading to considerable morbidity and mortality. Organ transplant patients are at a greater than 100 fold increased chance of developing cSCC leading to a high burden of malignancy (Euvrard et al, 2003), while patients with the genetic skin blistering disease, recessive dystrophic epidermolysis bullosa, suffer from unprecedented terminal metastasis with over 80% mortality making cSCC the usual cause of death for this patient group (Fine et al, 2009).

Treatment is always required for cSCC and principally consists of excision and/or radiotherapy for local disease control with a paucity of options for recurrent and metastatic disease. Pursuit of targeted therapies capable of halting the growth and spread of cSCC remains a clear research goal. Recent success with targeted therapies for the treatment of chronic myeloid leukemia, HER2 amplified and *BRCA* mutated tumors demonstrate that this goal is achievable and holds great potential (Druker et al., 2001; Piccart-Gebhart et al., 2005; Fong et al., 2009). Screening for cancer targets is hampered by tumor complexity and heterogeneity coupled with difficulties in distinguishing between drivers of tumor characteristics and the characteristics themselves (Merlo et al., 2006). Furthermore, targets of cancer pathways are frequently inherent to normal cell function resulting in clinically limiting side effects when these pathways are successfully targeted (Cheng and Force, 2010).

RNA profiling has been widely used as readout of tumor characteristics and numerous targets have been identified through its use both *in vitro* and *in vivo* (Ross et al., 2000; van de Vijver et al., 2002). However, inconsistencies between data sets attributed to differences in technologies, analysis and sample collection or preparation have led to both speculation over the validity of such approaches and measures to improve data collection (Michiels et al., 2007; Shi et al., 2006). Certainly, it is clear that although valid potential targets can be identified, either in cSCC or other cancers, they rarely show ubiquitous properties (Gallegos Ruiz et al., 2008; Green et al., 2006).

### SUMMARY OF THE INVENTION

The present invention is based on the identification of genes associated with the neoplastic condition, cutaneous squamous cell carcinoma (cSCC).

The present invention is directed to a polynucleotide for use in the treatment and/or prevention of cSCC according to claims 1 and 2, a polypeptide for use in the treatment and/or prevention of cSCC according to claims 3 and 4, a compound for use in treating cSCC according to claims 5 to 7, a pharmaceutical composition according to claim 8, the use of oligonicleotride/polypeptide probes and/or primers according to claim 9, a method of diagnosing cSCC or a predisposition or susceptibility thereto according to claims 10 and 11 and the use of a kit according to claim 12.

Accordingly and in a first aspect, the present invention provides the polynucleotides and/or polypeptides described herein for use in the treatment and/or prevention of cSCC.

In a second aspect, there is provided the use of polynucleotides and/or polypetides described herein for the manufacture of a medicament for the treatment and/or prevention of cSCC.

In a third aspect, there is provided a method of treating cSCC comprising administering to a patient in need thereof a therapeutically effective amount of one or more of the polynucleotides and/or polypeptides described herein.

The term "polynucleotides" as used above may encompass one or more polynucleotides encoding the genes selected from the group consisting of:
(i) Polo-like kinase-1 (*PLK*1);
(ii) Chromosome 20 open reading frame 20 (*c20orf20*);
(iii) Germ cell-specific gene 2 (Haspin: *GSG*2);
(iv) Bradykinin receptor B1 (*BDKRB*1); and
(v) serine protease 21 (testisin: *PRSS*21).

While the skilled man may be familiar with these genes, exemplary sequences may be retrieved from the Entrez Gene database (www.ncbi.nlm.nih.gov/gene) using the following Gene ID numbers:
(i) PLK1 - Gene ID: 5347
   **SEQ** ID NO: 1: **PLK1** (Homo sapiens) 
[?]
gagcggtgcg gaggctctgc tcggatcgag gtctgcagcg cagcttcggg agcatgagtg
ctgcagtgac tgcagggaag ctggcacggg caccggccga ccctgggaaa gccggggtcc
ccggagttgc agctcccgga gctccggcgg cggctccacc ggcgaaagag atcccggagg
 
tcctagtgga cccacgcagc cggcggcgct atgtgcgggg ccgctttttg ggcaagggcg
gctttgccaa gtgcttcgag atctcggacg cggacaccaa ggaggtgttc gcgggcaaga
ttgtgcctaa gtctctgctg ctcaagccgc accagaggga gaagatgtcc atggaaatat
ccattcaccg cagcctcgcc caccagcacg tcgtaggatt ccacggcttt ttcgaggaca
acgacttcgt gttcgtggtg ttggagctct gccgccggag gtctctcctg gagctgcaca
agaggaggaa agccctgact gagcctgagg cccgatacta cctacggcaa attgtgcttg
gctgccagta cctgcaccga aaccgagtta ttcatcgaga cctcaagctg ggcaaccttt
tcctgaatga agatctggag gtgaaaatag gggattttgg actggcaacc aaagtcgaat
atgacgggga gaggaagaag accctgtgtg ggactcctaa ttacatagct cccgaggtgc
tgagcaagaa agggcacagt ttcgaggtgg atgtgtggtc cattgggtgt atcatgtata
ccttgttagt gggcaaacca ccttttgaga cttcttgcct aaaagagacc tacctccgga
tcaagaagaa tgaatacagt attcccaagc acatcaaccc cgtggccgcc tccctcatcc
agaagatgct tcagacagat cccactgccc gcccaaccat taacgagctg cttaatgacg
agttctttac ttctggctat atccctgccc gtctccccat cacctgcctg accattccac
caaggttttc gattgctccc agcagcctgg accccagcaa ccggaagccc ctcacagtcc
tcaataaagg cttggagaac cccctgcctg agcgtccccg ggaaaaagaa gaaccagtgg
ttcgagagac aggtgaggtg gtcgactgcc acctcagtga catgctgcag cagctgcaca
gtgtcaatgc ctccaagccc tcggagcgtg ggctggtcag gcaagaggag gctgaggatc
ctgcctgcat ccccatcttc tgggtcagca agtgggtgga ctattcggac aagtacggcc
ttgggtatca gctctgtgat aacagcgtgg gggtgctctt caatgactca acacgcctca
tcctctacaa tgatggtgac agcctgcagt acatagagcg tgacggcact gagtcctacc
tcaccgtgag ttcccatccc aactccttga tgaagaagat caccctcctt aaatatttcc
gcaattacat gagcgagcac ttgctgaagg caggtgccaa catcacgccg cgcgaaggtg
atgagctcgc ccggctgccc tacctacgga cctggttccg cacccgcagc gccatcatcc
tgcacctcag caacggcagc gtgcagatca acttcttcca ggatcacacc aagctcatct
tgtgcccact gatggcagcc gtgacctaca tcgacgagaa gcgggacttc cgcacatacc
gcctgagtct cctggaggag tacggctgct gcaaggagct ggccagccgg ctccgctacg
cccgcactat ggtggacaag ctgctgagct cacgctcggc cagcaaccgt ctcaaggcct
cctaatagct gccctcccct ccggactggt gccctcctca ctcccacctg catctggggc
ccatactggt tggctcccgc ggtgccatgt ctgcagtgtg ccccccagcc ccggtggctg
ggcagagctg catcatcctt gcaggtgggg gttgctgtgt aagttatttt tgtacatgtt
 
cgggtgtggg ttctacagcc ttgtccccct ccccctcaac cccaccatat gaattgtaca

gaatatttct attgaattcg gaactgtcct ttccttggct ttatgcacat taaacagatg

tgaatattca aaaaaaaaaa aaaaaaaaaa aaaaaaaaaa aaaa
 (NCBI Reference Sequence: NM_005030.3)
(ii) c20orf20 - Gene ID: **55257**
   **SEQ ID NO: 2: C20orf20 (Homo sapiens)** 
agtgcgcctg cgcggagctc gtggccgcgc ctgctcccgc cgggggctcc ttgctcggcc

gggccgcggc catgggagag gccgaggtgg gcggcggggg cgccgcaggc gacaagggcc

cgggggaggc ggccaccagc ccggcggagg agacagtggt gtggagcccc gaggtggagg

tgtgcctctt ccacgccatg ctgggccaca agcccgtcgg tgtgaaccga cacttccaca

tgatttgtat tcgggacaag ttcagccaga acatcgggcg gcaggtccca tccaaggtca

tctgggacca tctgagcacc atgtacgaca tgcaggcgct gcatgagtct gagattcttc

cattcccgaa tccagagagg aacttcgtcc ttccagaaga gatcattcag gaggtccgag

aaggaaaagt gatgatagaa gaggagatga aagaggagat gaaggaagac gtggaccccc

acaatggggc tgacgatgtt ttttcatctt caggagtttt ggggaaagca tcagaaaaat

ccagcaaaga caaagagaag aactcctcag acttggggtg caaagaaggc gcagacaagc

ggaagcgcag ccgggtcacc gacaaagtcc tgaccgcaaa cagcaaccct tccagtccca

gtgctgccaa gcggcgccgc acgtagaccc tcagccctgg tggcggcaga gaagcgggcg

aggcactgtg gtcgctgagg gggttggctg ggtctgagtg ccacccccca ggccacagtg

ataccatccc agtgccatga gcccacactg cccgccctca ggctctcagg tgaacgtggc

cgtcagcggg gaaacgtgtg tgtcagttgg accatgtggg accctgatgg acctgaaaga

ccaggatcgg tccagctcag atattgaggg ctctgaagcc tagttctgtc ttctctggag

cagctgtggc ttccccgtgg ctgcttggtg acatggatta gcgctacgtg ggctgcagca

tttgggatcc aggctaccta gaggggcatc gggccaggga aaacctcgga ttagcaagca

ataaaaacat gacctcactc ttcctcaaag gagcccctgg tcttccctgt gtgactcagt

tctttccatc tgtttgtccc gctgcaagcc tctttctgcg ctgactgtga cattggaacg

tggccttcct gtcaccccct ccgtgccacg cactgaaggc cacccccacc cacctgggaa

actaagaact ggatattttg cctcattcac ttgtactgta acaatgtata taatttggtt

ggtatttcac tatttaattt ttaagaagcc tattttacta gtgttttata tgaacaaagt

actgcagaag ttaaacctgt gttgtatttt ttctgagatg ttttgcttta agagatactt
 
tttgctcagt ttttatatgc cagatacaga gaatttgtag cggttatttt tgtatgatct

agtaacttgc aaacagacca aatggatgag aggcggggac cgtgcagctg tcggctgatg

aggaggcggc cgccccagtg ctgatggaga tgccactttc gtgtgactgc gaacattaaa

gcacaaaaaa atccaaaaaa aaaaaaaaaa aaaaaaaaa
 NCBI Reference Sequence: N_018270.4
(iii) GSG2 - Gene ID: **83903**
   **SEQ ID NO: 3: GSG2 (Homo sapiens)** 
gtttgcgttt gaacctcttg gcgggtgccg gccatggcgg cttcgctccc gggacctggg
agccggcttt tccgcacata tggggctgcg gacggcagga gacagcggcg gccgggccgg
gaagccgcgc agtggttccc gccgcaggac cggaggcgtt tcttcaacag cagcggcagc
agcgacgcca gcatcggcga cccctcgcag tccgacgatc ctgacgatcc cgacgacccc
gacttccccg gcagcccggt gaggcggcgg cggaggcgtc ccggcggccg agtgcccaag
gaccggccca gcctgaccgt gaccccaaag cgctggaagc tgcgagctcg cccaagccta
accgtgaccc caagacgcct ggggctgcga gctcggcccc cgcagaagtg cagcacaccc
tgcggcccgc tccgacttcc gcccttcccc agccgcgact ccggccgcct cagcccggac
ctcagcgtgt gcggccagcc cagggacggc gacgagctgg gcatcagtgc ctccctgttc
agctctctgg cctcgccctg ccccgggtcc ccaacgccaa gggacagtgt catctcgatc
ggcacctccg cctgtctggt tgcagcctca gccgtcccga gcggcctcca cctcccagaa
gtctccctgg accgagcatc tctcccctgc tcccaggagg aagcgacagg aggagccaag
gacaccagga tggtccacca aacccgcgcc agcctcaggt cagttctctt tggccttatg
aactcaggaa cccctgagga ttctgagttt cgggcagatg ggaagaatat gagagagtcc
tgctgtaaaa ggaaactggt ggtgggaaat ggaccagagg gtccaggtct gtcaagcaca
ggcaagagga gggccacagg ccaggactct tgtcaagaga gagggcttca agaggccgtc
cggagagagc atcaggaggc cagtgttccc aagggccgca ttgtgccaag ggaatagac
aggctggaga gaactagatc aagccggaag agcaaacatc aggaggcaac ggaaacctct
ctcctccatt cccaccgctt taaaaagggc caaaagctgg gaaaagattc gttccccacc
caggacctga ctcctttaca gaatgtctgc ttttggacca aaaccagggc ttccttcagt
ttccacaaga agaaaattgt gactgatgtg tcagaggtct gcagcatcta taccactgcc
acttctctct ctggatccct cctatcagaa tgttcaaacc ggcctgtcat gaacagaaca
agtggtgctc cgtcctcttg gcactcctcc tctatgtatt tgctaagccc cttaaacact
 
ctaagtattt caaacaaaaa ggcatctgat gctgaaaagg tttatgggga atgcagtcag

aagggtcctg tcccctttag ccattgcctt cccacagaaa aactgcaacg ctgtgagaag

attggggaag gggtgtttgg cgaagtgttt caaacaattg ctgatcacac acccgtagcc

ataaaaatca ttgctattga aggaccagat ttagtcaatg gatcccatca gaaaaccttt

gaggaaatcc tgccagagat catcatctcc aaagagttga gcctcttatc cggtgaagtg

tgcaaccgca cagaaggctt tatcgggctg aactcagtgc actgtgtcca gggatcttac

cctcccttgc tcctcaaagc ctgggatcac tataattcaa ccaaaggctc tgcaaatgac

cggcctgatt tttttaaaga cgaccagctc ttcattgtgc tggaatttga gtttggaggg

attgacttag agcaaatgcg aaccaagttg tcttccttgg ctactgcaaa gagcattcta

caccagctca cagcctccct cgcagtggca gaggcatcac tgcgctttga gcaccgagac

ttacactggg ggaacgtgct cttaaagaaa accagcctca aaaaactcca ctacaccctc

aatgggaaga gcagcactat ccccagctgt gggttgcaag tgagcatcat tgactacacc

ctgtcgcgct tggaacggga tgggattgtg gttttctgtg acgtttccat ggatgaggac

ctgtttaccg gtgacggtga ctaccagttt gacatctaca ggctcatgaa gaaggagaat

aacaaccgct ggggtgaata tcacccttat agtaatgtgc tctggttaca ttacctgaca

gacaagatgc tgaaacaaat gaccttcaag actaaatgta acactcctgc catgaagcaa

attaagagaa aaatccagga gttccacagg acaatgctga acttcagctc tgccactgac

ttgctctgcc agcacagtct gtttaagtaa gctaaatgta tcttactgcc ccgaaatgag

aggagactgg tcttgaagcc tctggtgctg tttcaacctc catccccaca ggagggtgga

actcccattc tcacaggttt ccagtcagct tttcaaacaa gaattttgtt tccaaatgga

aactgaaata tttgttgaaa tgtttaaatt tgctgataac aaatgttctg aaagaagtaa

actagccggg cacagtggcg tgcgcctgta gtcccagcta ctcgggaggc tgaggcagga

ggatcgcttg agcccaagag ttcatatcta gcctggtcaa catagcaaga cccctgtctc

tattttttta aataaataaa ctacatgtga aaacaaa
 NCBI Reference Sequence: NM_031965.2
(iv) BDKRB1 - Gene ID: **623**
   **SEQ ID NO: 4: BDKRB1 (Homo sapiens)** 
aagagaaaac tcctccaaaa gcagctctca ctatcagaaa acccaactac agttgtgaac
gccttcattt tctgcctgag gtctcagtcc gtcggcccag actgaagtgc agtggcacaa
tcatagctcg ctgcagcctc gaccttccag gcttaaacga ttctcccacc tcagcctctc
gagttgctgg gaccacaggt cactgtgcat ggcatcatcc tggccccctc tagagctcca
 
atcctccaac cagagccagc tcttccctca aaatgctacg gcctgtgaca atgctccaga
agcctgggac ctgctgcaca gagtgctgcc aacatttatc atctccatct gtttcttcgg
cctcctaggg aacctttttg tcctgttggt cttcctcctg ccccggcggc aactgaacgt
ggcagaaatc tacctggcca acctggcagc ctctgatctg gtgtttgtct tgggcttgcc
cttctgggca gagaatatct ggaaccagtt taactggcct ttcggagccc tcctctgccg
tgtcatcaac ggggtcatca aggccaattt gttcatcagc atcttcctgg tggtggccat
cagccaggac cgctaccgcg tgctggtgca ccctatggcc agccggaggc agcagcggcg
gaggcaggcc cgggtcacct gcgtgctcat ctgggttgtg gggggcctct tgagcatccc
cacattcctg ctgcgatcca tccaagccgt cccagatctg aacatcaccg cctgcatcct
gctcctcccc catgaggcct ggcactttgc aaggattgtg gagttaaata ttctgggttt
cctcctacca ctggctgcga tcgtcttctt caactaccac atcctggcct ccctgcgaac
gcgggaggag gtcagcagga caaggtgcgg gggccgcaag gatagcaaga ccacagcgct
gatcctcacg ctcgtggttg ccttcctggt ctgctgggcc ccttaccact tctttgcctt
cctggaattc ttattccagg tgcaagcagt ccgaggctgc ttttgggagg acttcattga
cctgggcctg caattggcca acttctttgc cttcactaac agctccctga atccagtaat
ttatgtcttt gtgggccggc tcttcaggac caaggtctgg gaactttata aacaatgcac
ccctaaaagt cttgctccaa tatcttcatc ccataggaaa gaaatcttcc aacttttctg
gcggaattaa aacagcattg aaccaagaaa aaaaaaaaaa aaaaaaa
 NCBI Reference Sequence: NM_000710.2
(v) PRSS21 - Gene ID: **10942**
   **SEQ ID NO: 5: PRSS21 (Homo sapiens)** 
gctgggagta gagggcagag ctcccacccc gccccgcccc cagggggcgc cccgggcccg
gcgcgagagg aggcagaggg ggcgtcaggc cgcgggagag gaggccatgg gcgcgcgcgg
ggcgctgctg ctggcgctgc tgctggctcg ggctggactc aggaagccgg agtcgcagga
ggcggcgccg ttatcaggac catgcggccg acgggtcatc acgtcgcgca tcgtgggtgg
agaggacgcc gaactcgggc gttggccgtg gcaggggagc ctgcgcctgt gggattccca
cgtatgcgga gtgagcctgc tcagccaccg ctgggcactc acggcggcgc actgctttga
aacctatagt gaccttagtg atccctccgg gtggatggtc cagtttggcc agctgacttc
catgccatcc ttctggagcc tgcaggccta ctacacccgt tacttcgtat cgaatatcta
tctgagccct cgctacctgg ggaattcacc ctatgacatt gccttggtga agctgtctgc
acctgtcacc tacactaaac acatccagcc catctgtctc caggcctcca catttgagtt
tgagaaccgg acagactgct gggtgactgg ctgggggtac atcaaagagg atgaggcact
gccatctccc cacaccctcc aggaagttca ggtcgccatc ataaacaact ctatgtgcaa
 
ccacctcttc ctcaagtaca gtttccgcaa ggacatcttt ggagacatgg tttgtgctgg
caatgcccaa ggcgggaagg atgcctgctt cggtgactca ggtggaccct tggcctgtaa
caagaatgga ctgtggtatc agattggagt cgtgagctgg ggagtgggct gtggtcggcc
caatcggccc ggtgtctaca ccaatatcag ccaccacttt gagtggatcc agaagctgat
ggcccagagt ggcatgtccc agccagaccc ctcctggccg ctactctttt tccctcttct
ctgggctctc ccactcctgg ggccggtctg agcctacctg agcccatgca gcctggggcc
actgccaagt caggccctgg ttctcttctg tcttgtttgg taataaacac attccagttg
atgccttgca gggcattctt caaaa
 NCBI Reference Sequence: NM_006799.2

As stated, the inventors have determined that each of the genes encoded by SEQ ID NOS: 1-5 above, are associated with cSCC and as such, hereinafter, these genes (and others described herein or identified by methods provided by this invention) shall be referred to as "cSCC genes". The cSCC genes described herein represent possible therapeutic targets for treating and/or preventing neoplastic diseases such as, for example, cSCC.

In this regard, one of skill will appreciate that where a condition such as cSCC results from the modulated (particularly increased) expression, function and/or activity of one or more cSCC genes (particularly those described above) a cSCC gene sequence (or fragment thereof or sequence complementary to any portion thereof), may be used to restore wild type function, expression and/or activity. As such, any of the sequences given as SEQ ID NOS: 1-5 above (or fragments thereof or or sequences complementary thereto of to fragments thereof) may be used to restore wild type expression, function and/or activity of the corresponding genes. Such an approach may result in the treatment and/or prevention of cSCC.

In one part of the disclosure the polynucleotides comprise the complete cSCC gene sequences provided above as SEQ ID NOS: 1-5. However, other embodiments of this disclosure relate to polynucleotide fragments derived from any of SEQ ID NOS: 1-5. It should be understood that the term "polynucleotide fragments" may encompass fragments comprising at least 5-150, at least 5-100, at least 5-75, at least 5-50, at least 5-40, at least 5-30, at least 5-20, at least 5-15 and at least 5-10 nucleotides of any of the sequences described herein (for example SEQ ID NOS: 1-5. Typically, the fragments may comprise at least 5, at least 10, at least 20, at least 30, at least 40, at least 50, at least 75, at least 100 or at least 150 nucleotides of any of the sequences described herein. The skilled man will appreciate that the polynucleotide fragments described herein may comprise consecutive nucleotide sequences from any of SEQ ID NOS 1-5.

The term "polynucleotides" may also relate to sequences which are complementary to any of the sequences described herein (or to fragment sor portions thereof) and which hybridise thereto under conditions of high, medium or low stringency (see below for further discussion of such conditions).

In addition, the term "polynucleotides" may include the sequences of genes identified in the various tables presented in this application (For example Tables S2, S5 and S6).

As stated, the disclosure further relates to polypetides (including proteins or peptides) encoded by the cSCC genes or polynucleotides (including polynucleotide fragments) of this invention. Accordingly, the polypeptides of this disclosure may be referred to as "cSCC polypeptides. In one part of the disclosure the cSCC polypeptides are the protein products of the sequences encoded by SEQ ID NOS: 1-5 above - i.e. the products of the cSCC genes.

As above, the inventors have determined that each of the genes encoded by SEQ ID NOS: 1-5 above encode proteins associated with cSCC; as such, these cSCC proteins represent possible therapeutic targets for treating and/or preventing neoplastic diseases such as, for example, cSCC.

In particular, the disclosure relates to uses or methods exploiting one or more of the polypeptides provided below:
SEQ ID NO: 6: Human PLK1 sequence 
MSAAVTAGKLARAPADPGKAGVPGVAAPGAPAAAPPAKEIPEVLVDPRSRRRYVRGRF
LGKGGFAKCFEISDADTKEVFAGKIVPKSLLLKPHQREKMSMEISIHRSLAHQHVVGF
HGFFEDNDFVFVVLELCRRRSLLELHKRRKALTEPEARYYLRQIVLGCQYLHRNRVIH
RDLKLGNLFLNEDLEVKIGDFGLATKVEYDGERKKTLCGTPNYIAPEVLSKKGHSFEV
DVWSIGCIMYTLLVGKPPFETSCLKETYLRIKKNEYSIPKHINPVAASLIQKMLQTDP
TARPTINELLNDEFFTSGYIPARLPITCLTIPPRFSIAPSSLDPSNRKPLTVLNKGLE
NPLPERPREKEEPVVRETGEVVDCHLSDMLQQLHSVNASKPSERGLVRQEEAEDPACI
IFWVSKWVDYSDKYGLGYQLCDNSVGVLFNDSTRLILYNDGDSPLQYIERDGTESYLT
VSSHPNSLMKKITLLKYFRNYMSEHLLKAGANITPREGDELARLPYLRTWFRTRSAII
HLSNGSVQINFFQDHTKLILCPLMAAVTYIDEKRDFRTYRLSLLLEEYGCCKELASRL
RYARTMVDKLLSSRSASNRLKAS
SEQ ID NO: 7: Human C20orf20 sequence 
MGEAEVGGGGAAGDKGPGEAATSPAEETVVWSPEVEVCLFHAMLGHKPVGVNRHFHM
ICIRDKFSQNIGRQVPSKVIWDHLSTMYDMQALHESEILPFPNPERNFVLPEEIIQE
VREGKVMIEEEMKEEMKEDVDPHNGADDVFSSSGSLGKASEKSSKDKEKNSSDLGCK
EGADKRKRSRVTDKVLTANSNPSSPSAAKRRRT
SEQ ID NO: 8: Human GSG2 sequence 
MAASLPGPGSRLFRTYGAADGRRQRRPGREAAQWFPPQDRRRFFNSSGSSDASIGDP
SQSDDPDDPDDPDFPGSPVRRRRRRPGGRVPKDRPSLTVTPKRWKLRARPSLTVTPR
RLGLRARPPQKCSTPCGPLRLPPFPSRDSGRLSPDLSVCGQPRDGDEGISASLFSSL
ASPCPGSPTPRDSVISIGTSACLVAASAVPSGLHLPEVSLDRASLPCSQEEATGGAK
DTRMVHQTRASLRSVLFGLMNSGTPEDSEFRADGKNMRESCCKRKLVVGNGPEGPGL
SSTGKRRATGQDSCQERGLQEAVRREHQEASVPKGRIVPRGIDRLERTRSSRKSKHQ
EATETSLLHSHRFKKGQKLGKDSFPTQDLTPLQNVCFWTKTRASFSFHKKKIVTDVS
EVCSIYTTATSLSGSLLSECSNRPVMNRTSGAPSSWHSSSMYLLSPLNTLSISNKKA
SDAEKVYGECSQKGPVPFSHCLPTEKLQRCEKIGEGVFGEVFQTIADHTPVAIKIIA
IEGPDLVNGSHQKTFEEILPEIIISKELSLLSGEVCNRTEGFIGLNSVHCVQGSYPP
LLLKAWDHYNSTKGSANDRPDFFKDDQLFIVLEFEFGGIDLEQMRTKLSSLATAKSI
LHQLTASLAVAEASLRFEHRDLHWGNVLLKKTSLKKLHYTLNGKSSTIPSCGLQVSI
IDYTLSRLERDGIVVFCDVSMDEDLFTGDGDYQFDIYRLMKKENNNRWGEYHPYSNV
LWLHYLTDKMLKQMTFKTKCNTPAMKQIKRKIQEFHRTMLNFSSATDLLCQHSLFK
SEQ ID NO: 9: Human BDKRB1 sequence 
MASSWPPLELQSSNQSQLFPQNATACDNAPEAWDLLHRVLPTFIISICFFGLLGNLF
VLLVFLLPRRQLNVAEIYLANLAASDLVFVLGLPFWAENIWNQFNWPFGALLCRVIN
GVIKANLFISIFLVVAISQDRYRVLVHPMASRRQQRRRQARVTCVLIWVVGGLLSIP
 
TFLLRSIQAVPDLNITACILLLPHEAWHFARIVELNILGFLLPLAAIVFFNYHILAS
LRTREEVSRTRCGGRKDSKTTALILTLVVAFLVCWAPYHFFAFLEFLFQVQAVRGCF
WEDFIDLGLQLANFFAFTNSSLNPVIYVFVGRLFRTKVWELYKQCTPKSLAPISSSH
RKEIFQLFWRN
SEQ ID NO: 10: Human PRSS21 sequence

MGARGALLLALLLARAGLRKPESQEAAPLSGPCGRRVITSRIVGGEDAELGRWPWQG
SLRLWDSHVCGVSLLSHRWALTAAHCFETYSDLSDPSGWMVQFGQLTSMPSFWSLQA
YYTRYFVSNIYLSPRYLGNSPYDIALVKLSAPVTYTKHIQPICLQASTFEFENRTDC
WVTGWGYIKEDEALPSPHTLQEVQVAIINNSMCNHLFLKYSFRKDIFGDMVCAGNAQ
GGKDACFGDSGGPLACNKNGLWYQIGVVSWGVGCGRPNRPGVYTNISHHFEWIQKLM
AQSGMSQPDPSWPLLFFPLLWALPLLGPV

In a further embodiment, "polypeptides" may encompass proteins encoded by the sequences provided as SEQ ID NOS: 6-10. However, other embodiments relate to polypeptide or peptide fragments derived from any of SEQ ID NOS: 6-10. Typically, polypeptide/peptide fragments encompass fragments comprising at least 5-150, at least 5-100, at least 5-75, at least 5-50, at least 5-40, at least 5-30, at least 5-20, at least 5-15 and at least 5-10 amino acids of any of the sequences described herein (for example SEQ ID NOS: 6-10. Typically, the fragments may comprise at least 5, at least 10, at least 20, at least 30, at least 40, at least 50, at least 75, at least 100 or at least 150 amino acids of any of the sequences described herein. The skilled man will appreciate that the polypeptide/peptide fragments described herein may comprise consecutive amino acids from any of SEQ ID NOS 6-10. Methods for creating polpeptide fragments are well known and may include the use of PCR techniques to amplified select parts of relevant nucleic acid sequences and subsequent cloning and protein expression techniques. Such techniques may involve the use of cloning and expression vectors. Further information regarding the cloning, expression and purification of recombinant proteins (including fragments) may be found in Molecular Cloning: A Laboratory Manual (Third Edition) Sambrook, MacCallum & Russell (CSHL, 2001) and Basic Methods in Protein Purification and Analysis: A Laboratory Manual: Simpson, Adams & Golemis (CSHL, 2009).

It should be understood that the disclosure may further relate to the polypeptides encoded by the genes cited in the Tables (for example Tables S2, S5 and S6) provided herein.

In addition to the above, the disclosure may encompass mutants, variants, derivatives and/or homologs/orthologues of any of the polynucleotides/polypeptides provided by this invention.

Typically, fragments, mutants, variants, derivatives and/or homologs/orthologues described herein are functional - that is to say, they retain the function and/or activity of the wild type genes (PLK1, c20orf20, GSG2, BDKRB1 and PRSS21) from which they are derived.

The term "mutants" may encompass naturally occurring mutant sequences and or those artificially created using, for example, recombinant techniques. "Mutant" sequences may comprise one or more nucleotide/amino acid additions, deletions, substitutions and/or inversions.

One of skill will readily understand that polynucleotide and/or polypeptide sequences homologous or identical to any of the human sequences described herein, may be found in a number of species, including, for example, other mammalian species. According to this disclosure, homologous and/or identical sequences may exhibit as little as approximately 20% or 30% sequence homology or identity however, in other cases, homologous/identical sequences may exhibit at least 40, 50, 60, 65, 70, 75, 80, 85, 90, 91, 92, 93, 94, 95, 96, 97, 98 and 99% homology/identity to the various polynucleotide/polypeptide sequences given above. As such, the present disclosure may further relate to homologous and/or identical sequences described above.

For the various polynucleotide and/or polypeptide sequences described herein (for example SEQ ID NOS: 1-10 and fragments thereof), natural variations, due to, for example, polymorphisms, may exist between those sequences given as SEQ ID NOS: 1-10 above and the same gene/protein sequences isolated from any given species or from other members of the same species. These variants may comprise polynucleotide/polypeptide sequences that comprise one or more nucleotide or amino acid substitutions, additions, deletions and/or inversions relative to a reference sequence (for example any of the sequences described above as SEQ ID NOS: 1-10). It is also known that the degeneracy of the genetic code permits substitution of one or more bases in a codon without changing the primary amino acid sequence. Consequently, although the sequences described in this application are known to encode specific genes, the degeneracy of the code may be exploited to yield variant nucleic acid sequences which encode the same primary amino acid sequences.

Where a condition such as cSCC results from the modulated or aberrant (reduced or increased) expression, function and/or activity of one or more cSCC proteins (particularly those described above) a cSCC protein sequence (or fragment thereof), may be used to restore wild type function, expression and/or activity. As such, any of the sequences given as SEQ ID NOS: 6-10 above (or fragments thereof) may be used to restore wild type expression, function and/or activity of the corresponding cSCC protein. Such an approach may result in the treatment and/or prevention of cSCC.

By way of example, where the increased expression, function and/or activity of a cSCC protein (such as those described above) contributes to or causes cSCC, the cSCC sequences provided above may be used to further modulate the aberrant (i.e. increased) function, expression and/or activity fo the cSCC protein. One of skill will appreciate that the whole or fragments of the polypeptide sequences described herein may be used to replicate or antagonise (inhibit) the function and/or activity of the native and modulated (for example aberrantly expressed) cSCC protein.

In a further aspect, the disclosure relates to compounds, for example, proteins, peptides, amino acids, carbohydrates, small organic molecules, antibodies and/or nucleic acids, which modulate the activity, function and/or expression of any of the genes identified herein as being associated with cSCC. In other embodiments, the compounds provided by this disclosure may modulate the activity, function and/or expression of any of the protein products of the genes described herein.

Accordingly, a second aspect of this disclosure provides compounds for use in treating cSCC, wherein said compounds modulate the expression, function and/or activity of any of the cSCC genes/proteins described herein. Further aspects may provide uses of said compounds of the manufacture of medicaments for treating and/or preventing cSCC as well as methods of treating cSCC, comprising the administration of one or more of the compounds described herein, to a patient in need thereof.

In one embodiment, the present disclosure provides nucleic acids, for example compounds comprising DNA and/or RNA, which may be used to regulate the expression, function and/or activity of any of the genes described herein. Such compounds will be referred to hereinafter as "oligonucleotide compounds". Oligonucleotide compounds of this type may comprise those for use in gene therapy, where, for example, any of the sequences described as SEQ ID NOS: 1-5 above (or fragments thereof) may be used to restore wild-type gene expression. For example, where disease results from a complete absence gene expression, an appropriate gene sequence may be used to restore gene expression.

In other embodiments, the oligonucleotide compounds provided by this invention may comprise antisense, silencing and/or interfering nucleic acids. The skilled man will be familiar with antisense nucleic acids (known as antisense oligonucleotides) which may comprise DNA or RNA and may comprise sequences complementary to mRNA sequences encoding the protein products of the cSCC genes identified herein. The skilled man is also familiar with silencing and/or small interfering (si) RNA molecules which may be used to modulate the function, activity and/or expression of targeted genes such as those described herein.

The oligonucleotide compounds provided by this disclosure may be designed to modulate the function, activity and/or expression of any of the genes identified as being associated with cSCC. By analysing wild type gene sequences, such as the sequences identified above and provided as SEQ ID NOS: 1-5 and with the aid of algorithms such as BIOPRED*si* and/or siDesign center, one of skill could readily determine or computationally predict oligonucleotide sequences that have an optimal knock-down effect for these genes (see for example: http://www.dharmacon.com/DesignCenter/DesignCenterPage.aspx). Furthermore, the skilled man may generate and test an array or library of different oligonucleotides to determine whether or not they are capable of modulating the expression, function and/or activity of any of the genes described herein.

As such, one part of the disclosure provides oligonucleotide compounds for use in (i) treating or preventing cSCC (ii) the manufacture of a medicament for the treatment and/or prevention of cSCC and (iii) in methods of treating subjects suffereing from or susceptible to, cSCC; wherein said oligonucleotide compounds modulate the expression, function and/or activity of the cSCC genes described herein.

In certain embodiments, the oligonucleotide compounds for use in (or the manufacture of medicaments for) treating or preventing cSCC may be selected from the group consisting of:
(i) CUCAGAUAUUGAGGGCUCUdTdT
   AGAGCCCUCAAUAUCUGAGdTdT
(ii) GGGACAAGUUCAGCCAGAAdTdT
   UUCUGGCUGAACUUGUCCCdTdT
wherein said oligonucleotides are particularly useful in controlling aberrant c20orf20 expression.

One of skill will appreciate that were upregulation of a particular gene or genes is found to be associated with (or causative of) cSCC, the compounds provided by this invention may be used to reduce (perhaps selectively) the expression of such a gene or genes. Where the down regulation of a gene or genes is/are found to be associated with cSCC, the oligonucleotide compounds provided by this disclosure (including the whole sequences (or fragments thereof) listed as SEQ ID NOS: 1-5 above) may be used to restore wild-type function and/or activity by upregulating or replicating/mimicking the expression, function and/or activity of the gene or gene(s) in question.

In addition, antibodies (or antigen/epitope binding fragments thereof) capable of binding to the protein products of the cSCC genes described herein may be useful in the treatment and/or prevention of cSCC. Antibodies which block or neutralise the cSCC proteins described herein, may be particularly useful where cSCC results from the over expression of a cSCC proteins - such as for example a cSCC protein described above. Antibodies useful in the treatment and/or prevention of cSCC may be either polyclonal or monoclonal antibodies and the techniques used to generate either are well known to one of skill in this field. As such, the skilled man is well placed to be able to raise antibodies (either monoclonal or polyclonal) which exhibit specificity and/or selectivity for any of the cSCC proteins described herein.

In one embodiment, the invention provides antibodies specific for one or more epitopes contained within the following peptides which peptides comprise sequences derived from the C20orf20 protein:
(a) CNPSSPSAAKRRRT
(b) GEAEVGGGGAAGDKGC
(c) CGKASEKSSKDKEKNSSD

With regards c20orf20, the inventors have surprisingly found that inhibition of this gene in cSCC cells results in apoptosis without altering cell cycle parameters. In contrast, inhibition of the same gene in colon carcinoma cell lines only results in inhibited proliferation. One of skill will readily understand that treatments which result in tumour cell apoptosis may be considered as significantly more useful than treatments which inhibit cell proliferation only. Accordingly, compounds which are able to inhibit the expression function and/or activity of c20orf20, may find particular application in the treatment of cSCC. Compounds of this type may include oligonucleotide compounds described above and/or antibodies against the product of the c20orf20 gene.

Without wishing to be bound by theory, the inventors have further discovered that cSCC cell survival may depend, not only on the function, activity and/or expression of the C20orf20 gene or protein(s) encoded thereby, but on functional interactions with components of the TIP60 HAT complex. As such, this disclosure may further extend to methods, uses and/or medicaments for treating cSCC, which methods, uses and/or medicaments modulate the function activity and/or expression of VPS72 (Vacuolar protein sorting-associated protein 72), EPC1 (Enhancer of polycomb homolog 1), DMAP1 (DNMT1-associated protein 1) and/or TRRAP (transformation/transcription domain associated protein) proteins and/or genes encoding the same.

In one part the disclosure provides polynucleotides and/or polypeptides encoding VPS72, EPC1, DMAP1 and/or TRRAP proteins and/or genes (i) for use in treating cSCC; (ii) for use in the manufacture of a medicament for treating cSCC; (iii) a method of treating cSCC comprising administering a patient in need thereof a therapeutically effective amount of one or more polynucleotides/polypeptides encoding VPS72, EPC1, DMAP1 and/or TRRAP; a method of diagnosing cSCC, comprising probing a sample for a level of VPS72, EPC1, DMAP1 and/or TRRAP expression, function and/or activity.

The disclosure further relates to fragments, homologues, mutants, variants and/or derivatives of any of the VPS72, EPC1, DMAP1 and/or TRRAP proteins/genes; the definitions of fragments, homologues, mutants, variants and/or derivatives being provided above and elsewhere in this specification.

It should be understood that the successful treatment and/or prevention of cSCC may depend on the use of combinations of the various compounds and polynucleotide sequences/polypeptides described herein.

In one aspect, the present disclosure provides pharmaceutical compositions comprising one or more compounds selected from the group consisting of polynucleotides provided by this disclosure (including cSCC genes); polypeptides of the disclosure (including the cSCC proteins) and compounds which modulate the function, activity and/or expression of one or more of the cSCC genes/proteins described herein, together or in asscociation with, a pharmaceutically acceptable excipient, carrier or diluent. Such compositions may find application in the treatment or prevention of (or methods of treating or preventing) cSCC.

Pharmaceutical formulations include those suitable for oral, topical (including dermal, buccal and sublingual), rectal or parenteral (including subcutaneous, intradermal, intramuscular and intravenous), transdermal, nasal and pulmonary (for example by inhalation) administration. The formulation may, where appropriate, be conveniently presented in discrete dosage units and may be prepared by any of the methods well known in the art of pharmacy. Methods typically include the step of bringing into association an active compound with liquid carriers or finely divided solid carriers or both and then, if necessary, shaping the product into the desired formulation.

Pharmaceutical formulations suitable for oral administration wherein the carrier is a solid are most preferably presented as unit dose formulations such as boluses, capsules or tablets each containing a predetermined amount of active compound. A tablet may be made by compression or moulding, optionally with one or more accessory ingredients. Compressed tablets may be prepared by compressing in a suitable machine an active compound in a free-flowing form such as a powder or granules optionally mixed with a binder, lubricant, inert diluent, lubricating agent, surface-active agent or dispersing agent. Moulded tablets may be made by moulding an active compound with an inert liquid diluent. Tablets may be optionally coated and, if uncoated, may optionally be scored. Capsules may be prepared by filling an active compound, either alone or in admixture with one or more accessory ingredients, into the capsule shells and then sealing them in the usual manner. Cachets are analogous to capsules wherein an active compound together with any accessory ingredient(s) is sealed in a rice paper envelope. An active compound may also be formulated as dispersible granules, which may for example be suspended in water before administration, or sprinkled on food. The granules may be packaged, e.g., in a sachet. Formulations suitable for oral administration wherein the carrier is a liquid may be presented as a solution or a suspension in an aqueous or non-aqueous liquid, or as an oil-in-water liquid emulsion.

Formulations for oral administration include controlled release dosage forms, e.g., tablets wherein an active compound is formulated in an appropriate release-controlling matrix, or is coated with a suitable release-controlling film. Such formulations may be particularly convenient for prophylactic use.

Pharmaceutical formulations suitable for rectal administration wherein the carrier is a solid are most preferably presented as unit dose suppositories. Suitable carriers include cocoa butter and other materials commonly used in the art. The suppositories may be conveniently formed by admixture of an active compound with the softened or melted carrier(s) followed by chilling and shaping in moulds.

Pharmaceutical formulations suitable for parenteral administration include sterile solutions or suspensions of an active compound in aqueous or oleaginous vehicles.

Injectable preparations may be adapted for bolus injection or continuous infusion. Such preparations are conveniently presented in unit dose or multi-dose containers, which are sealed after introduction of the formulation until required for use. Alternatively, an active compound may be in powder form that is constituted with a suitable vehicle, such as sterile, pyrogen-free water, before use.

An active compound may also be formulated as long-acting depot preparations, which may be administered by intramuscular injection or by implantation, e.g., subcutaneously or intramuscularly. Depot preparations may include, for example, suitable polymeric or hydrophobic materials, or ion-exchange resins. Such long-acting formulations are particularly convenient for prophylactic use.

Formulations suitable for pulmonary administration *via* the buccal cavity are presented such that particles containing an active compound and desirably having a diameter in the range of 0.5 to 7 microns are delivered in the bronchial tree of the recipient.

As one possibility such formulations are in the form of finely comminuted powders which may conveniently be presented either in a pierceable capsule, suitably of, for example, gelatin, for use in an inhalation device, or alternatively as a self-propelling formulation comprising an active compound, a suitable liquid or gaseous propellant and optionally other ingredients such as a surfactant and/or a solid diluent. Suitable liquid propellants include propane and the chlorofluorocarbons, and suitable gaseous propellants include carbon dioxide. Self-propelling formulations may also be employed wherein an active compound is dispensed in the form of droplets of solution or suspension.

Such self-propelling formulations are analogous to those known in the art and may be prepared by established procedures. Suitably they are presented in a container provided with either a manually-operable or automatically functioning valve having the desired spray characteristics; advantageously the valve is of a metered type delivering a fixed volume, for example, 25 to 100 microlitres, upon each operation thereof.

As a further possibility an active compound may be in the form of a solution or suspension for use in an atomizer or nebuliser whereby an accelerated airstream or ultrasonic agitation is employed to produce a fine droplet mist for inhalation.

Formulations suitable for nasal administration include preparations generally similar to those described above for pulmonary administration. When dispensed such formulations should desirably have a particle diameter in the range 10 to 200 microns to enable retention in the nasal cavity; this may be achieved by, as appropriate, use of a powder of a suitable particle size or choice of an appropriate valve. Other suitable formulations include coarse powders having a particle diameter in the range 20 to 500 microns, for administration by rapid inhalation through the nasal passage from a container held close up to the nose, and nasal drops comprising 0.2 to 5% w/v of an active compound in aqueous or oily solution or suspension.

It should be understood that in addition to the aforementioned carrier ingredients the pharmaceutical formulations described above may include, an appropriate one or more additional carrier ingredients such as diluents, buffers, flavouring agents, binders, surface active agents, thickeners, lubricants, preservatives (including anti-oxidants) and the like, and substances included for the purpose of rendering the formulation isotonic with the blood of the intended recipient.

Pharmaceutically acceptable carriers are well known to those skilled in the art and include, but are not limited to, 0.1 M and preferably 0.05 M phosphate buffer or 0.8% saline. Additionally, pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Preservatives and other additives may also be present, such as, for example, antimicrobials, antioxidants, chelating agents, inert gases and the like.

Formulations suitable for topical formulation may be provided for example as gels, creams or ointments.

Liquid or powder formulations may also be provided which can be sprayed or sprinkled directly onto the site to be treated, e.g. a wound or ulcer. Alternatively, a carrier such as a bandage, gauze, mesh or the like can be impregnated, sprayed or sprinkled with the formulation and then applied to the site to be treated.

Therapeutic formulations for veterinary use may conveniently be in either powder or liquid concentrate form. In accordance with standard veterinary formulation practice, conventional water-soluble excipients, such as lactose or sucrose, may be incorporated in the powders to improve their physical properties. Thus particularly suitable powders of this invention comprise 50 to 100% w/w and preferably 60 to 80% w/w of the active ingredient(s) and 0 to 50% w/w and preferably 20 to 40% w/w of conventional veterinary excipients. These powders may either be added to animal feedstuffs, for example by way of an intermediate premix, or diluted in animal drinking water.

Liquid concentrates of this disclosure suitably contain the compound or a derivative or salt thereof and may optionally include an acceptable water-miscible solvent for veterinary use, for example polyethylene glycol, propylene glycol, glycerol, glycerol formal or such a solvent mixed with up to 30% v/v of ethanol. The liquid concentrates may be administered to the drinking water of animals.

In general, a suitable dose of the one or more compounds of the invention may be in the range of about 1 µg to about 5000 µg /kg body weight of the subject *per* day, *e.g.,* 1, 5, 10, 25, 50, 100, 250, 1000, 2500 or 5000 µg/kg per day. Where the compound(s) is a salt, solvate, prodrug or the like, the amount administered may be calculated on the basis the parent compound and so the actual weight to be used may be increased proportionately.

Transdermal administration may be achieved with the use of impregnated coverings dressings, bandages or the like or via the use of some form of transdermal delivery device. Such devices are advantageous, particularly for the administration of a compound useful in the treatment of a cutaneous disease, such as for example neoplastic diseases such as cSCC, as they may allow a prolonged period of treatment relative to, for example, an oral or intravenous medicament. Furthermore, transdermal administration of any of the compositions/medicaments described herein may be particularly advantageous for the treatment fo cSCC as it permits direct contact between the neoplastic lesion and the therapeutic moiety for prolonged periods of time.

Examples of transdermal delivery devices may include, for example, a patch, dressing, bandage or plaster adapted to release a compound or substance through the skin of a patient. A person of skill in the art would be familiar with the materials and techniques which may be used to transdermally deliver a compound or substance and exemplary transdermal delivery devices are provided by GB2185187, US3249109, US3598122, US4144317, US4262003 and US4307717.

By way of example, any of the compounds provided by this invention may be combined with some form of matrix or substrate, such as a non-aqueous polymeric carrier, to render it suitable for use in a bandage, dressing, covering or transdermal delivery system. The compound/matrix or substrate mixture may be further strengthened by the use of a woven or knit, non-woven, relatively open mesh fabric, to produce a patch, bandage, plaster or the like which may be reversibly attached to a particular region of a patient's body. In this way, while in contact with a patient's skin, the transdermal delivery device releases the compound or substance through the skin.

Based upon the inventor's finding that certain genes and/or proteins are associated with cSCC, the invention may provide nucleotide/peptide probes or primers for use in detection, diagnostic and/or expression methods/studies. Typical detection studies may include, for example, Polymerase chain reaction (PCR) hybridisation studies, sequencing protocols and immunological and/or Sothern/Northern blotting detection techniques.

Advantageously a polypeptide and/or polynucleotide for use as a probe and/or primer may comprise 10-30 nucleotides and/or 5-30 amino acids (although the exact length (perhaps shorter or longer than the lengths suggested above) will depend on the application). Furthermore, the probes or primers should exhibit some specificity for a particular sequence and limited or no binding to unrelated sequences. In order to reduce incidences of non-specific/selective binding, polynucleotide (oligonucleotide) and/or polypeptide probes/primers provided by this disclosure may have at least 50%, at least 60%, at least 70%, at least 75%, at least 80%, at least 85%, at least 90%, at least 95%, at least 99% or even 100% complementarity to all or part of the polynucleotide/polypeptide sequences described herein.

Thus, one of skill will appreciate that a combination of probe/primer design and the use of stringent (low, moderate and high) conditions, can greatly reduce incidences of non-specific binding.

Hybridisation between a probe/primer and a nucleic acid sequence (such as any described herein) may be effected at a temperature of about 40°C-75°C in 2-6 x SSC (i.e. 2-6 x NaCl 17.5g/l and sodium citrate (SC) at 8.8g/l) buffered saline containing 0.1% sodium dodecyl sulphate (SDS). Of course, depending on the degree of similarity between the probe/primer and the sequence, buffers with a reduced SSC concentration (i.e. 1xSSC containing 0.1% SDS, 0.5xSSC containing 0.1% SDS and 0.1xSSC containing 0.1% SDS).

As such, the present disclosure extends to the provision of oligonucleotide/polypeptide probes and/or primers designed to hybridise to all or part of a sequence selected from the group consisting of SEQ ID NOS: 1-10.

In further aspect, the disclosure provides a method of diagnosing cSCC or a susceptibility or predisposition thereto, said method comprising determining if one or more of the cSCC genes and/or cSCC proteins described herein exhibits a modulated function or aberrant (for example modulated) expression or activity. The diagnostic methods described herein may also be used to stage or assess a cSCC tumour.

The methods of diagnosis described herein may require the provision of a sample to be tested, said sample being provided by a subject, wherein said subject may have or may be suspected of suffereing from, cSCC; in other cases the subject may appear healthy and may be subjected to a diagnostic test simply to determine whether they are suffering from cSCC or whether they are susceptible or predisposed to developing cSCC.

Accordingly, the method of diagnosing cSCC or a predisposition or susceptibility thereto may comprise:
(a) providing a sample from a subject; and
(b) identifying a level of expression or activity of one or more cSCC genes and/or proteins;
wherein the detection of aberrant levels of cSCC gene expression/activity and/or cSCC protein expression/activity indicates that the subject is suffering from and/or susceptible/predisposed to cSCC.

The term "sample" should be understood as including samples of bodily fluids such as whole blood, plasma, serum, saliva, sweat and/or semen. In other instances "samples" such as tissue biopsies and/or scrapings may be used. In particular, cutaneous (i.e. skin) tissue biopsies and/or scrapings may be used. Advantageously such biopsies may comprise keratinocyte cells and in some embodiments, the keratinocytes and/or biopsy as a whole, may be obtained from lesions suspected of comprising cSCC - in other cases the biopsy or keratinocytes may be derived from tissue which does not exhibit pathology indicative of cSCC or from healthy tissue. In addition, a sample may comprise a tissue or gland secretion and washing protocols may be used to obtain samples of fluid secreted into or onto various tissues, including, for example, the skin. One of skill in this field will appreciate that the samples described above may yield or comprise quantities of nucleic acid (i.e. DNA or RNA) from one or more of the cSCC genes described herein as well as quantities of proteins or peptides (or fragments thereof) encoded thereby.

As stated, subjects diagnosed as suffering from cSCC or having a susceptibility or predisposition thereto, may yield samples which exhibit modulated and/or aberrant cSCC gene/protein expression, function or activity. The term "aberrant" or "modulated" expression, function and/or activity should be understood to encompass levels of gene/protein expression, function or activity that are either increased and/or decreased relative to the expression, function and/or activity of the same cSCC genes/proteins detected or identified in samples derived from healthy subjects or from subjects not suffering from cSCC. As such, all of the diagnostic methods described herein may further comprise the optional step of comparing the results with those obtained from reference or control samples (perhaps samples derived from healthy individuals), wherein aberrant or modulated function, expression and/or activity of one or more cSCC gene(s)/protein(s) in a sample tested, may exhibit as a level of expression, function and/or activity which is different from (i.e. higher or lower than) the level of expression, function and/or activity of the same gene(s)/protein(s) identified in the reference or control sample.

One of skill in the art will be familiar with the techniques that may be used to identify levels of cSCC genes and/or cSCC proteins, such as, for example, those levels of PLK1; c20orf20; GSG2; BDKRB1 and/or PRSS21, in samples such as those listed above.

For example, PCR based techniques may be used to detect levels of cSCC gene expression or gene quantity in a sample. Useful techniques may include, for example, polymerase chain reaction (PCR) using genomic DNA as template or reverse transcriptase (RT)-PCR (see below) based techniques in combination with real-time PCR (otherwise known as quantitative PCR). In the present case, real time-PCR may be used to determine the level of expression of the genes encoding any of the cSCC proteins described herein. Typically, and in order to quantify the level of expression of a particular nucleic acid sequence, RT-PCR may be used to reverse transcribe the relevant mRNA to complementary DNA (cDNA). Preferably, the reverse transcriptase protocol may use primers designed to specifically amplify an mRNA sequence of interest (in this case cSCC gene derived mRNA). Thereafter, PCR may be used to amplify the cDNA generated by reverse transcription. Typically, the cDNA is amplified using primers designed to specifically hybridise with a certain sequence and the nucleotides used for PCR may be labelled with fluorescent or radiolabelled compounds.

One of skill in the art will be familiar with the technique of using labelled nucleotides to allow quantification of the amount of DNA produced during a PCR. Briefly, and by way of example, the amount of labelled amplified nucleic acid may be determined by monitoring the amount of incorporated labelled nucleotide during the cycling of the PCR.

In one embodiment and to enable the quantification of c20orf20 mRNA, primers 5'-ATTCTTCCATTCCCGAATCC-3' and 5'-CCCAAACTCCCTGAAGATGA-3' may be used.

Further information regarding the PCR based techniques described herein may be found in, for example, PCR Primer: A Laboratory Manual, Second Edition Edited by Carl W. Dieffenbach & Gabriela S. Dveksler: Cold Spring Harbour Laboratory Press and Molecular Cloning: A Laboratory Manual by Joseph Sambrook & David Russell: Cold Spring Harbour Laboratory Press.

Other techniques that may be used to determine the level of cSCC gene expression in a sample include, for example, Northern and/or Southern Blot techniques. A Northern blot may be used to determine the amount of a particular mRNA present in a sample and as such, could be used to determine the amount or level of cSCC gene expression. Briefly and in one embodiment, mRNA may be extracted from, for example, a cell using techniques known to the skilled artisan, and subjected to electrophoresis. A nucleic acid probe, designed to hybridise (i.e. complementary to) an mRNA sequence of interest - in this case mRNA encoding one or more cSCC genes, may then be used to detect and quantify the amount of a particular mRNA present in a sample.

Additionally, or alternatively, a level of cSCC gene/protein expression may be identified by way of microarray analysis. Such a method would involve the use of a DNA micro-array which comprises nucleic acid derived from cSCC genes. To identify a level of cSCC gene expression, one of skill in the art may extract the nucleic acid, preferably the mRNA, from a sample and subject it to an amplification protocol such as, RT- PCR to generate cDNA. Preferably, primers specific for a certain mRNA sequence - in this case sequences encoding cSCC genes may be used.

The amplified cSCC cDNA may be subjected to a further amplification step, optionally in the presence of labelled nucleotides (as described above). Thereafter, the optionally labelled amplified cDNA may be contacted with the microarray under conditions which permit binding with the DNA of the microarray. In this way, it may be possible to identify a level of cSCC gene expression.

In addition, other techniques such as deep sequencing and/or pyrosequencing may be used to detect cSCC sequences in any of the samples described above. Further information on these techniques may be found in "Applications of next-generation sequencing technologies in functional genomics", Olena Morozovaa and Marco A. Marra, Genomics Volume 92, Issue 5, November 2008, Pages 255-264 and "Pyrosequencing sheds light on DNA sequencing", Ronaghi, Genome Research, Vol. 11, 2001, pages 3-11.

In addition to the molecular detection methods described above, one of skill will also appreciate that immunological detection techniques such as, for example, enzyme-linked immunosorbent assays (ELISAs) may be used to identify levels of cSCC proteins in samples. In other embodiments, ELISPOT, dot blot and/or Western blot techniques may also be used. In this way, samples provided by subjects suffering from cSCC or from outwardly healthy subjects to be tested or from subjects susceptible or predisposed to cSCC, may be probed for levels of one or more cSCC proteins so as to detect aberrant or modulated expression, function and/or activity which may indicate cSCC or a susceptibility or predisposition thereto.

Immunological detection techniques, may require the use of a substrate to which an antibody and/or antigen may be bound, conjugated or otherwise immobilised.

Suitable substrates may comprise, for example, glass, nitrocellulose, paper, agarose and/or plastic. A substrate which comprises, for example, a plastic material, may take the form of a microtitre plate.

The substrates provided by this invention and for use in the methods described herein may further comprise cSCC proteins (for example, the protein products of the cSCC genes (PLK1; c20orf20; GSG2; BDKRB1 and/or PRSS21) described herein) bound, conjugated and/or immobilised thereto. In other embodiments, the substrate may comprise an agent capable of binding a cSCC protein. It should be understood that references to agents capable of binding cSCC proteins, may include antibodies and in particular polyclonal and/or monoclonal antibodies with affinity for the cSCC proteins described herein. Techniques used to generate antibodies are well known in the art and may involve the use of cSCC antigens (such as those described herein) in animal immunisation protocols or as a basis for the generation of hybridomas. Further information on the preparation and use of polyclonal and/or monoclonal antibodies may be obtained from Using Antibodies: A Laboratory Manual by Harlow & Lane (CSHLP: 1999) and Antibodies: A Laboratory Manual by Harlow & Lane (CSHLP: 1988) -.

Immunological detection techniques such as, ELISA, may be classed as "indirect" assays or "direct" assays - both forms of ELISA are useful here. An indirect ELISA may exploit the use of a substrate coated with an agent capable of binding a cSCC protein whereas a direct ELISA may utilise substrates coated with one or more of the cSCC protein(s) described herein.

An ELISA may involve contacting a sample with a substrate (such as a substrate described above) under conditions which permit binding between antibodies and/or antigen present in the sample and the substrate and/or substances bound or immobilised to the substrate. One familiar with these techniques will appreciate that prior to contacting the sample to be analysed with the substrate, a blocking step may be used to reduce or prevent non-specific binding.

An ELISA may comprise the further step of contacting the substrate with a secondary antibody having specificity or affinity for antigen and/or antibodies bound thereto (via antigen or antibody immobilised, bound or conjugated to the substrate). Secondary antibodies for use in this invention may be rodent or ruminant antibodies (polyclonal or monoclonal) specific to particular forms of antibody present within the sample being tested.

Secondary antibodies for use in this invention may be conjugate to moieties which permit them to be detected - such moieties being referred to hereinafter as detectable moieties. By way of example, a secondary antibody may be conjugated to an enzyme capable of being detected via a colourmetric/chemiluminescent reaction. Such conjugated enzymes may include but are not limited to Horse radish Peroxidase (HRP) and alkaline phosphatase (AlkP). Additionally, or alternatively, the secondary antibodies may be conjugated to a fluorescent molecule such as, for example, a fluorophore, such as FITC, rhodamine or Texas Red. Other types of detectable moiety include radiolabelled moieties.

Further information regarding ELISA procedures and protocols relating to the other immunological techniques described herein may be found in Using Antibodies: A Laboratory Manual by Harlow & Lane (CSHLP: 1999) and Antibodies: A Laboratory Manual by Harlow & Lane (CSHLP: 1988).

One of skill will appreciate that the amount of secondary antibody detected as bound to the substrate (via other moieties which are themselves bound directly or indirectly to the substrate) may be representative of the amount of antigen and/or antibody present in the sample being tested.

Alternatively, in order to identify a level of cSCC protein in a sample, a substrate (optionally comprising an agent capable of binding a cSCC protein) may be contacted with a sample to be tested. Any cSCC protein bound to the substrate (perhaps via an agent capable of binding a cSCC protein) may be detected with the use of a further agent capable of binding a cSCC protein - referred to hereinafter as a primary antibody. The primary binding agent may be an antibody, optionally conjugated to a detectable moiety as described above.

One of skill will appreciate that many variations of the ELISA protocols described above may be used in order to detect a level of cSCC protein or anti-cSCC protein antibody present in a sample.

Further information regarding ELISA procedures and protocols relating to the other immunological techniques described herein may be found in Using Antibodies: A Laboratory Manual by Harlow & Lane (CSHLP: 1999) and Antibodies: A Laboratory Manual by Harlow & Lane (CSHLP: 1988).

In one part of the disclosure the methods for detecting cSCC genes and/or proteins, may take the form of an immunochromatographic test - otherwise known as a "dip-stick" or "pen" tests, where a substrate, or portion thereof, is contacted with a sample to be tested. Thereafter, the test sample flows through and/or along a substrate (perhaps guided by microfluidic channels) under capillary action and is brought into contact with an agent or agents which enables detection of any cSCC gene/proteins or fragments thereof in the sample. Such tests can offer rapid result and exemplary devices may include those known as lateral flow devices.

One of skill will appreciate that the results of a dip-stick or lateral flow test may be revealed in a "test line" where, for example, a change in appearance of the test line may indicate a positive result (i.e. presence of cSCC genes and/or cSCC proteins).

Agents capable of effecting detection of cSCC genes or cSCC proteins in a sample may include particles such as, for example latex or gold particles optionally coated with compounds capable of binding the target analyte in a sample. Other forms of particle such as, for example, fluorescent and/or magnetic particles may also be used.

A dip-stick or lateral flow may device operate a sandwich assay system where the sample is first brought into contact with a particle, perhaps a coloured particle, comprising a compound (for example an antibody) capable of binding a cSCC protein (or cSCC gene) to form a particle complex. Thereafter particle complexes may be contacted with further agents capable of binding cSCC proteins, wherein said agents are bound and/or immobilised to a test line region of the substrate. In this way the particles become localised at a particular region of the substrate and can be detected.

Other techniques which exploit the use of agents capable of binding the cSCC proteins (or fragments or portions thereof) include for example, techniques such as Western blot or dot blot. A Western blot may involve subjecting a sample to electrophoresis so as to separate or resolve the components, for example the proteinaceous components, of the sample. In other embodiments, electrophoresis techniques may be used to separate proteins purified from recombinant (perhaps microbial) systems. The resolved components/proteins may then be transferred to a substrate, such as nitrocellulose.

In order to identify any cSCC proteins in a sample, the substrate (for example nitrocellulose substrate) to which the resolved components and/or proteins have been transferred, may be contacted with a binding agent capable of cSCC proteins under conditions which permit binding between any cSCC protein in the sample (or transferred to the substrate) and the agents capable of binding the cSCC protein.

Advantageously, the agents capable of binding the cSCC protein may be conjugated to a detectable moiety.

Additionally, the substrate may be contacted with a further binding agent having affinity for the binding agent(s) capable of binding the cSCC protein(s). Advantageously, the further binding agent may be conjugated to a detectable moiety.

Other immunological techniques which may be used to identify a level of Pso o 2 antigen in a sample include, for example, immunohistochemistry wherein binding agents, such as antibodies capable of binding cSCC, are contacted with a sample such as those described above, under conditions which permit binding between any cSCC protein present in the sample and the cSCC protein binding agent. Typically, prior to contacting the sample with the binding agent, the sample is treated with, for example a detergent such as Triton X100. Such a technique may be referred to as "direct" immunohistochemical staining.

Alternatively, the sample to be tested may be subjected to an indirect immunohistochemical staining protocol wherein, after the sample has been contacted with a cSCC protein binding agent, a further binding agent (a secondary binding agent) which is specific for, has affinity for, or is capable of binding the cSCC binding agent, is used to detect cSCC proteins /binding agent complexes.

The skilled person will understand that in both direct and indirect immunohistochemical techniques, the binding agent or secondary binding agent may be conjugated to a detectable moiety. Preferably, the binding agent or secondary binding agent is conjugated to a moiety capable of reporting a level of bound binding agent or secondary binding agent, via a colourmetric chemiluminescent reaction.

In order to identify the levels of cSCC protein present in the sample, one may compare the results of an immunohistochemical stain with the results of an immunohistochemical stain conducted on a reference sample. By way of example, a sample revealing more or less bound cSCC protein binding agent (or secondary binding agent) than in a reference sample, may have been provided by a subject with cSCC.

The present invention also extends to the use of kits comprising reagents and compositions suitable for diagnosing, detecting or evaluating cSCC in subjects. Kits may be used to identify and/or detect aberrant or modulated levels of cSCC gene/cSCC protein expression, function or activity in samples. Depending on whether or not the kits are intended to be used to identify levels of cSCC genes and/or cSCC proteins in samples, the kits may comprise substrates having cSCC proteins or agents capable of binding cSCC proteins, bound thereto. In addition, the kits may comprise agents capable of binding cSCC proteins - particularly where the kit is to be used to identify levels of one or more cSCC proteins in samples. In other embodiments, the kit may comprise polyclonal antibodies or monoclonal antibodies which exhibit specificity and/or selectivity for one or more cSCC proteins. Antibodies for inclusion in the kits may be conjugated to detectable moieties. Kits for use in detecting the expression of genes encoding cSCC proteins (i.e. cSCC genes) may comprise one or more oligonucleotides/primers for detecting/amplifying/probing samples (particularly soamples comprising nucleic acid - for example keratinocyte derived nucleic acis) for cSCC protein encoding sequences. The kits may also comprise other reagents to facilitate, for example, sequencing, PCR and/or RFLP analysis. In one embodiment, the kits may comprise one or more oligonucleotides/primers for detecting/amplifying/probing nucleic acid samples (for example nucleic acid derived from keratinocytes) for aberrant or modulated c20orf20 expression, function and/or activity. All kits described herein may further comprise instructions for use.

In addition to the above, a further aspect provides a method of identifying or selecting genes associated with, or involved in the pathogenesis of, cSCC, said method comprising the steps of:
(a) identifying genes exhibiting modulated or aberrant expression, function or activity in cSCC keratinocytes, and/or cSCC tissue, wherein genes identified as exhibiting modulated or aberrant expression, function and/or activity, are selected for further study;
(b) identifying genes exhibiting modulated or aberrant expression in benign skin conditions, wherein genes identified as exhibiting modulated or aberrant expression, function and/or activity, are selected for further study
(c) comparing the information obtained in step (a) with the information obtained in step (b) and eliminating from further study, genes which exhibit modulated or aberrant function, activity and/or expression in both the cSCC analysed in step (a) and the benign skin conditions analysed in step (b) and selecting for further study those genes which exhibit modulated or aberrant function, expression and/or activity only in cSCC keratinocytes.
(d) analysing the genes selected in step (c) and selecting those genes which do not exhibit differential regulation in *in vitro* compared with *in vivo* systems;

The term "cSCC keratinocytes" should be understood to encompass keratinocytes which exhibit features and/or pathology associated with cSCC. Cutaneous SCC keratinocytes may be derived from biopsies of cSCC confirmed lesions. In other emobodiments, the cells for use in the method described above, may be obtained from cell lines held in culture collections. Suitable cells lines may be known to those skilled in this field and may include RDEBSCC3, RDEBSCC4, SCCIC1 and SCCRDEB2 cells.

In one embodiment,step (a) of the method described above comprises a first step of identifying genes exhibiting modulated or aberrant expression, function or activity in cSCC keratinocytes, and a second step in which genes exhibiting modulated or aberrant expression, function or activity are identified in cSCC tissue, wherein genes identified as exhibiting modulated or aberrant expression, function and/or activity compared with normal skin tissue or normal skin keratinocytes, are selected for further study.

Benign skin conditions may comprise conditions such as, for example psoriasis. As such, step (b) of the method described above may utilise cells derived from, or provided by, a subject known to be suffering from a benign skin condition. In some embodiments, the cells for use in step (b) may be obtained from lesions associated with a benign skin condition- or from tissue exhibiting symptoms of a benign condition.

As described in step (c), the data collected from steps (a) and (b) may be compared and only those genes which show modulated and/or aberrant expression, function and/or activity in step (a) - i.e. genes which exhibit modulated and/or aberrant expression, function and/or activity in cSCC keratinocytes only, are selected for further study. The cohort of genes selected in step (c) has been designated the "cSCC specific signature".

The *in vitro* systems described in step (d) may comprise a keratinocyte cell culture comprising, for example cSCC keratinocytes as described above. In certain embodiments, the *in vitro* system comprises cSCC keratinocytes derived from deposited cells lines. One of skill will appreciate that *in vivo* systems for use in the method described above, and in particular step (d), may comprise the use of animal models, particularly cSCC models and/or biopsies provided by human subjects. In one embodiment, the *in vivo* system is a murine cSCC model - in which SCID mice are administered matrigel/tumourigenic keratinocyte cell complexes.

Following execution of the protocol outlined in step (d) above, the inventors identified a "cSCC specific" cohort of genes - wherein said genes exhibit cSCC driver-like properties.

In order to determine whether or not any of the genes selected after execution of step (d) are critical for tumour cell survival, the inventors have devised a validation step in which siRNA molecule(s) designed to interfere with the regulation and/or expression of one or more of the gene(s) identified as being cSCC specific (i.e. the genes identified in step (d)) is/are introduced to a cSCC keratinocyte and thereafter the status of the keratinocyte is assessed. If following introduction and/or contact with an siRNA molecule, the status (for example viability) of the cSCC keratinocyte alters (for example the cell dies, goes into apoptosis or exhibits an increased or decreased rate of proliferation), it may be possible to conclude that the gene modulated by the siRNA molecule contacted with, or introduced into the cell, is critical to the survival of cSCC tumours and is associated with cSCC cells.

The validation step may be performed as an optional step (e) in the method described above.

Cell viability may be assessed by simple microscopic observation to detect changes (perhaps morphological) which represent apoptotic or dying cells. In other cases staining techniques such as trypan blue staining may be used. Other embodiments may utilise cell viability assays such as MTT and MTS colourmetric assays. Further information relating to these procedures may be found in Mosmann T (1983): "Rapid colorimetric assay for cellular growth and survival: application to proliferation and cytotoxicity assays"; Journal of immunological methods 65 (1-2): 55-63.

One of skill will appreciate that libraries of siRNA molecules - each designed to potentially inhibit or interfere with the expression of a gene identified in step (d) may be used in the validation step described above. It should be understood that a siRNA "library" may comprise two or more siRNA molecules. Additionally, or alternatively, two or more siRNA molecules (each directed to a separate cSCC gene) or two more siRNA libraries (again each directed to a separate cSCC library) may be added to a cell simultaneously. In this way rapid screening of a large number of genes may be achieved.

The methods by which modulated and/or aberrant gene function, expression and/or activity may be identified or detected are described in detail above and may be applied to the methods described herein. Furthermore, it should be understood that levels of cSCC gene expression, function and/or activity may be determined by comparing levels of expression, function and/or activity identified in, for example, step (a) with a level of function, expression and/or activity of the same cSCC genes identified in reference or control systems. For example, the results obtained in step (a) may be compared with the results obtained from non-cSCC keratinocytes and/or non-cSCC tissue (perhaps keratinocytes obtained from non-diseased (healthy) tissue). In one embodiment, step (a) may comprise determining differences in mRNA expression levels between cSCC and non-cSCC keratinocytes - wherein genes which are identified as differentially expressed in these two systems, are selected for further study.

### DETAILED DESCRIPTION

The present invention will now be described in detail with reference to the following figures which show:

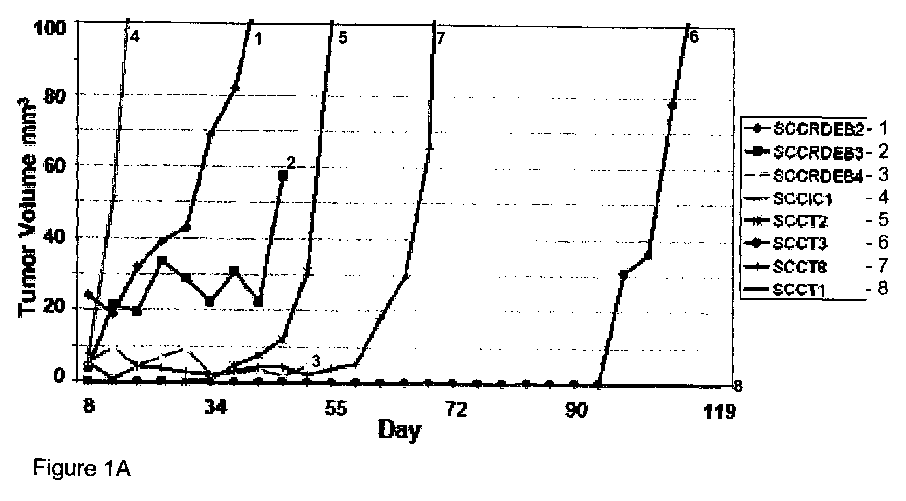

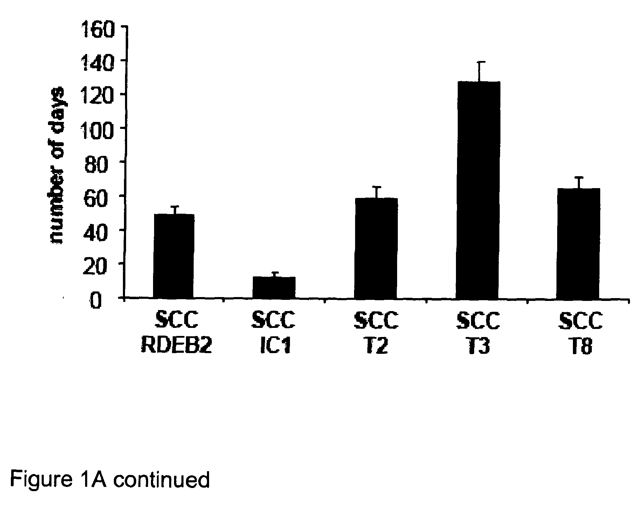

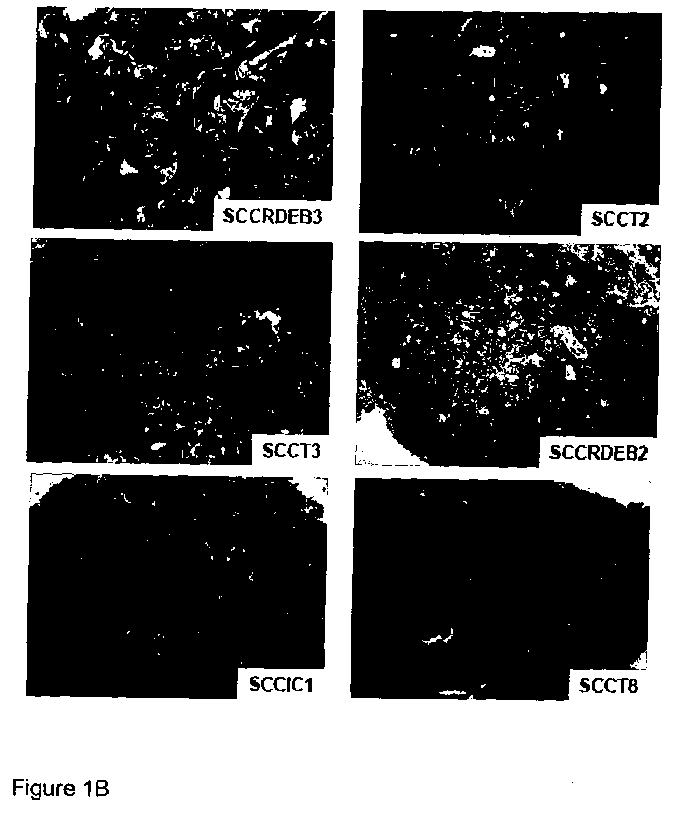

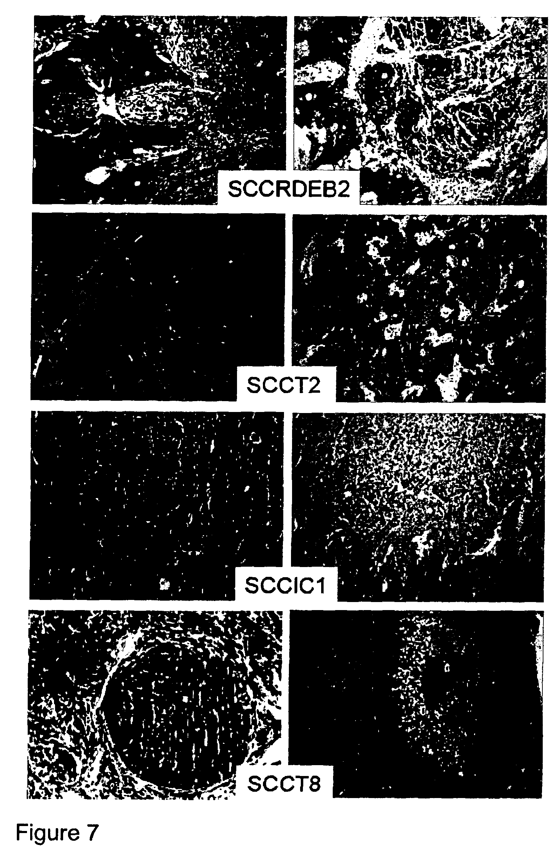

**Figure 1** **- cSCC keratinocytes readily form tumors in SCID mice with identical histology to human cSCC**. Female SCID Balb/c mice were subcutaneously injected in the right flank with 1-4×10⁶ tumor cells mixed with high-concentration Matrigel® (Becton-Dickinson, Oxford, UK). Tumor volumes were measured twice a week with callipers and calculated using the formula V = π4/3[(L + W)/4]3, were L is the length and W is the width. **(A)** Representative growth of 8 separate cSCC keratinocyte populations. **(B)** Number of days to reach a volume of 100mm³, data derived from 1-4 separate experiments n=2-6 in each case. **(C)** H&E stained sections of a representative xenograft tumor for each of the 6 cell populations that showed measurable growth in mice (100X magnification), see also Figure 7.

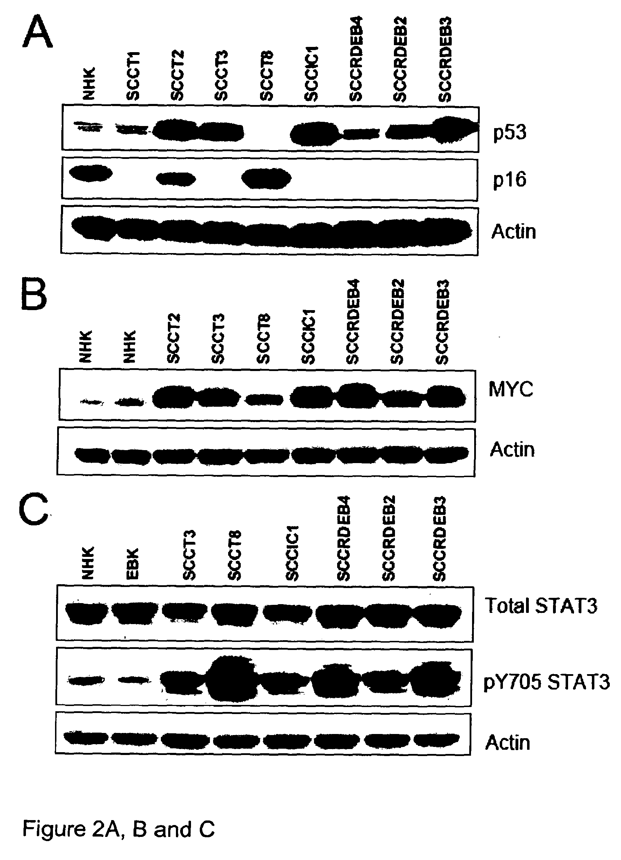

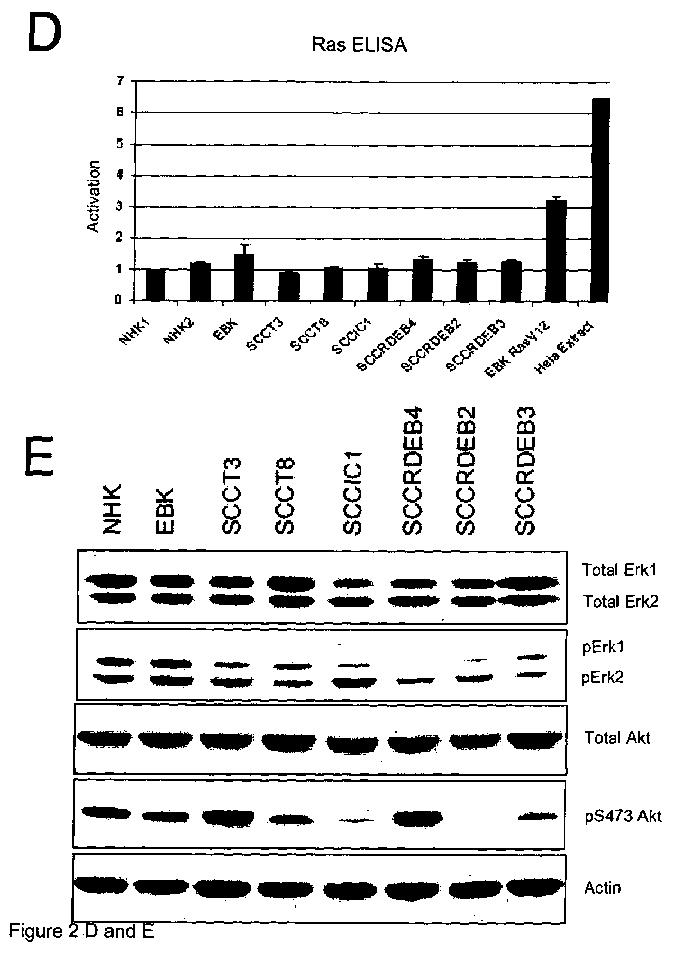

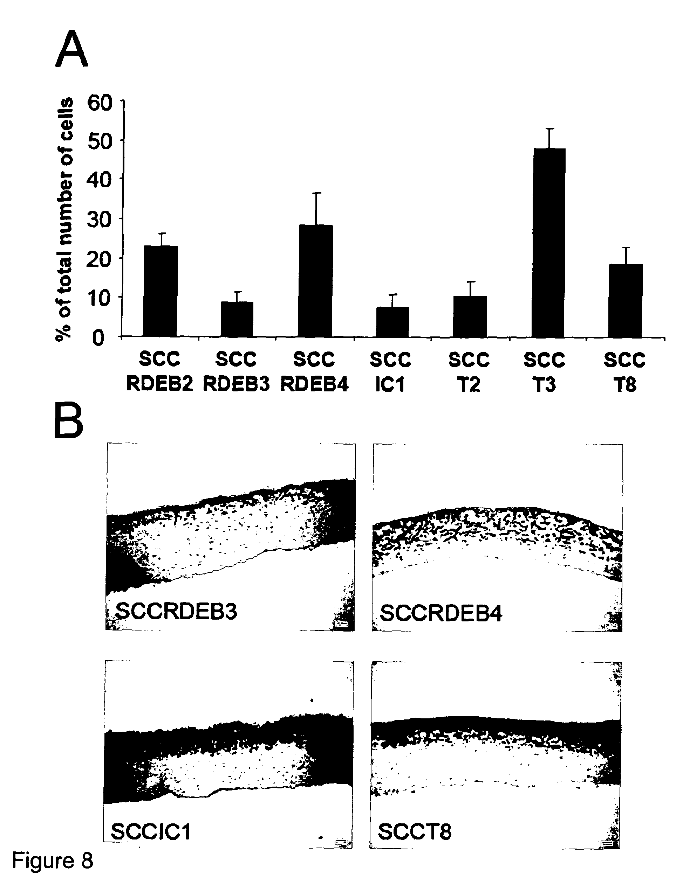

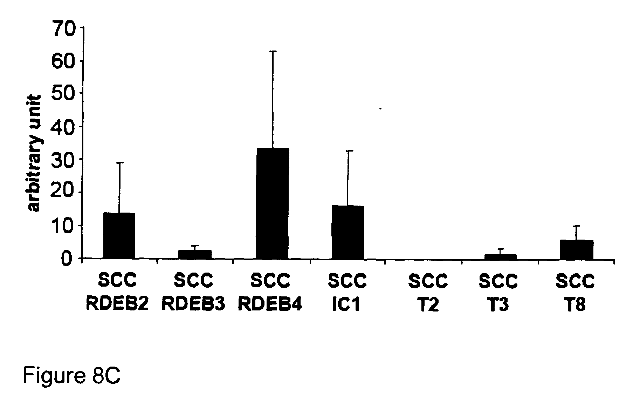

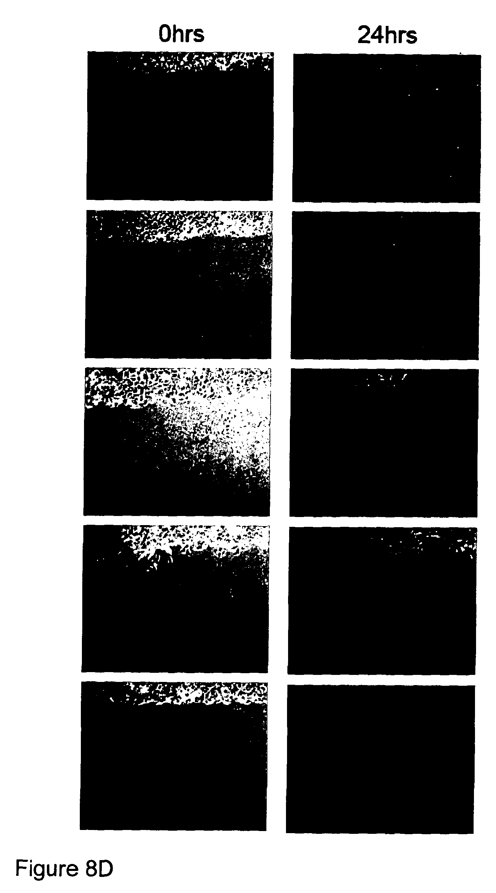

**Figure 2** **- cSCC tumor keratinocytes express altered p53, p16, increased myc, and increased phosphorylated STAT3 but do not display features of activated RAS. (A-C and E)** Western blotting of total cell lysates from cSCC cells and normal primary keratinocytes (NHK, separate donors in B) isolated from reduction surgery or RDEB skin (EBK). **(D)** Commercially available RAS ELISA showing no evidence of increased activated RAS in cultured cSCC populations compared with NHK or EBK. EBK Rasv12 are a population of RDEB skin keratinocytes transduced with a MML-V based vector expressing oncogenic RAS-V12. Hela Extract = positive control provided. No pattern from any of the antibody observations correlate with tumor forming ability of SCC keratinocytes, see also Figure 8.

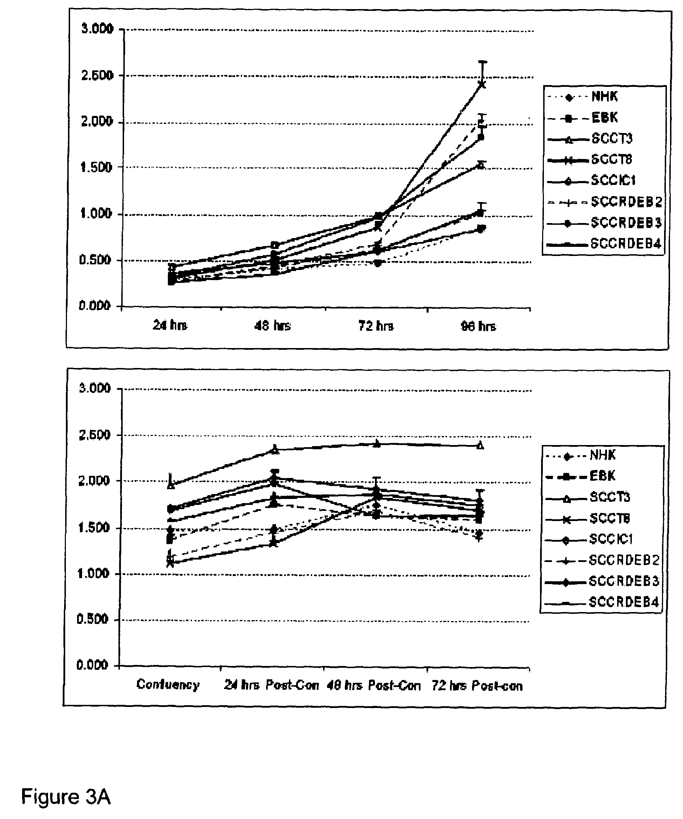

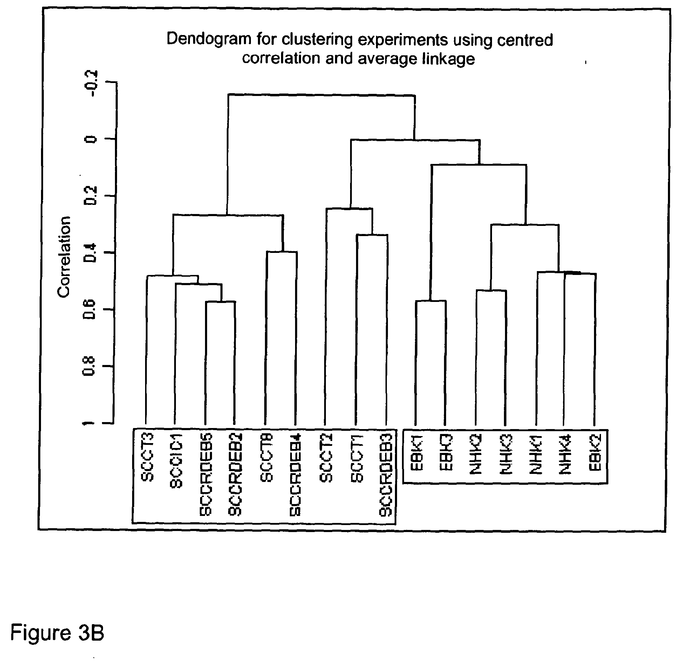

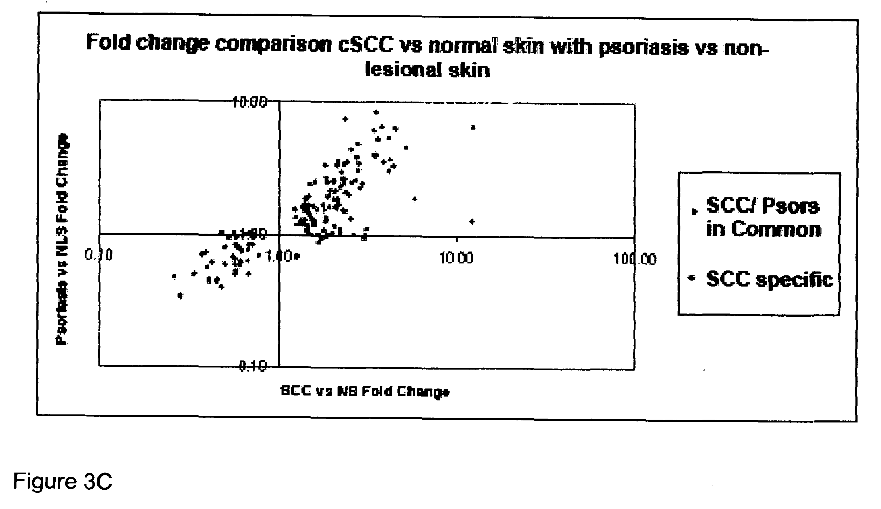

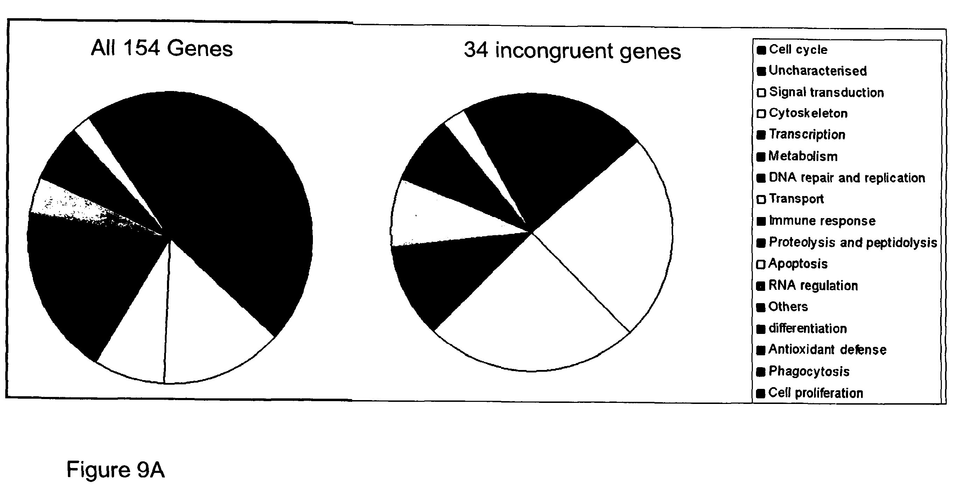

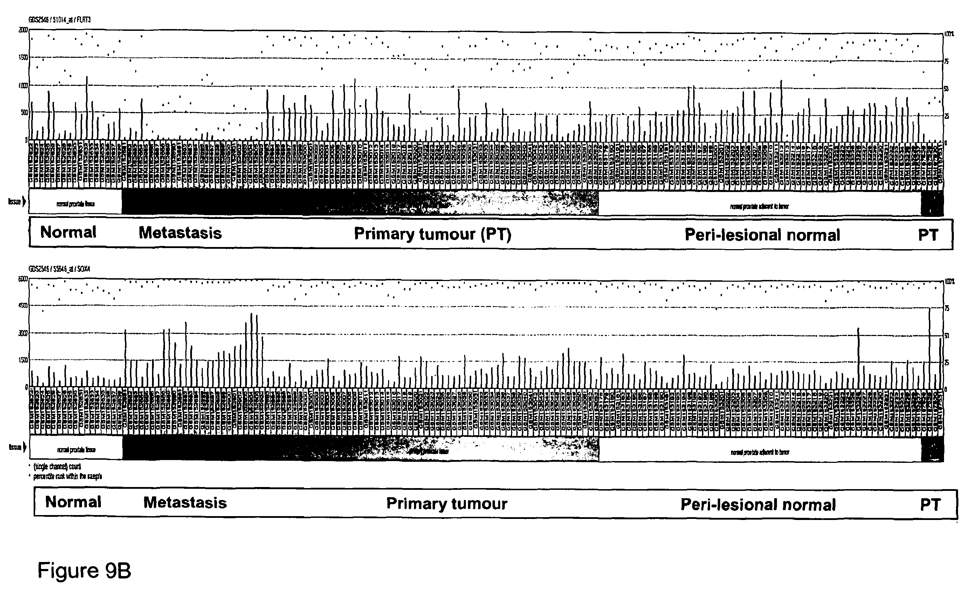

**Figure 3** **- Gene expression profiling can separate quiescent cSCC keratinocytes and normal keratinocytes in an unsupervised manner and identifies an *in vitro* cSCC signature consistently expressed in a range of *in vivo* data sets. (A)** Confluent keratinocytes are quiescent after 48 hours culture. Growth rates of keratinocytes used in this study as assessed by MTT assay seeded at low density (upper panel) and at confluence (lower panel). RNA was isolated between 48 and 56 hours post confluence. **(B)** Clustering dendrogram of *in vitro* gene expression data generated in BRB array tools v3.8.1. cSCC keratinocyte samples (red box) cluster independently of non-cSCC keratinocyte samples (blue box), see also Table S1-S2 for pairwise comparison of in vitro samples and full cSCC gene signature. **(C)** Average cSCC vs normal fold change for all 154 *in vivo* cSCC genes plotted against average psoriatic skin vs non-lesional skin fold change reveals a strong correlation (r²=0.84) for the majority of genes and identifies those genes specifically differentially regulated in cSCC. See also Tables S5-S6 for comprehensive gene lists and Figure 9 for GO and additional GEO analysis.

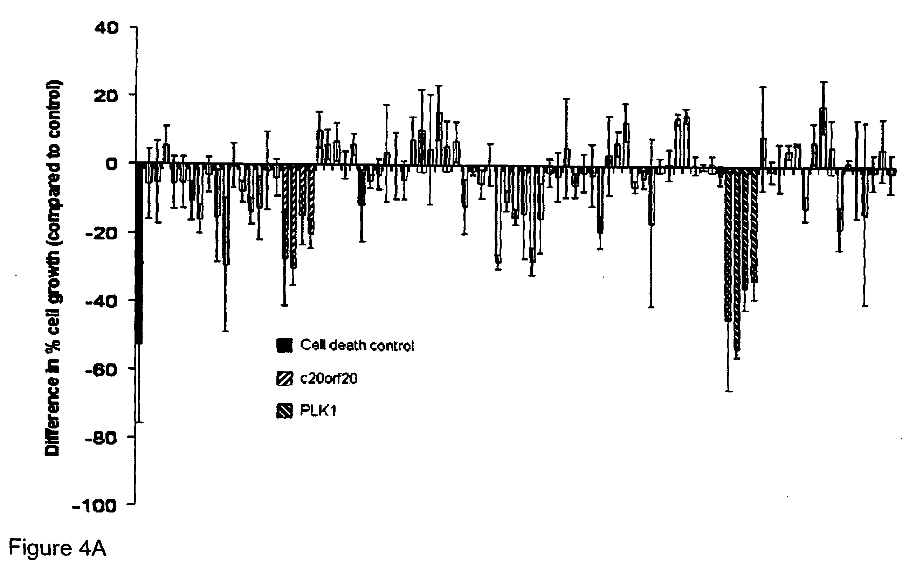

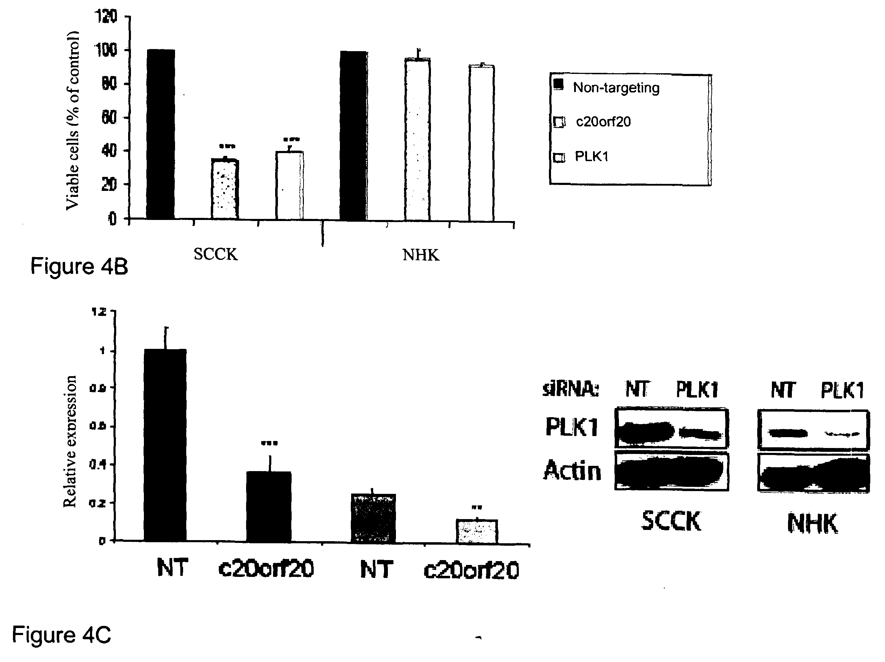

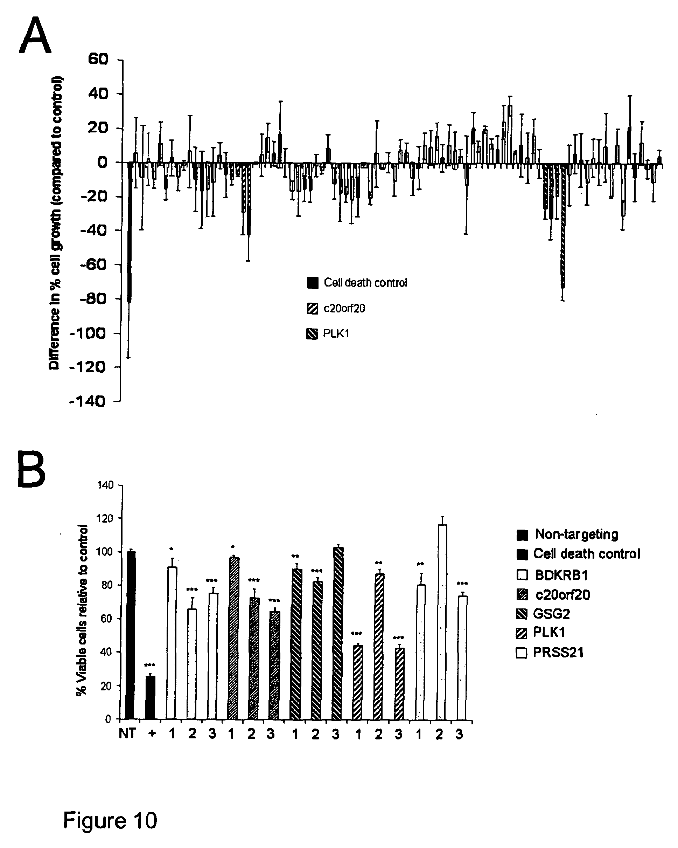

**Figure 4** **- *c20orf20* and *PLKl* knockdown inhibit cSCC growth with no effect on normal human primary keratinocytes. (A)** SCCIC1 keratinocytes were transfected with a siRNA library to the 21 cSCC specific up-regulated genes (3 siRNA duplexes individually and pooled) and cell viability assessed 72 hours post-transfection using the MTS assay. The percentage of viable cells was calculated relative to values at time zero (TO) and the difference in these values between treated samples and the non-targeting control siRNA is shown. (B) SCCRDEB2 (SCCK) keratinocytes and normal human keratinocytes (NHK) were transfected with siRNA to *c20orf20* and *PLK1* and cell viability determined by the MTS assay. The percentage of viable cells was calculated relative to values at T0 and data shown as a percentage of the non-targeting control siRNA value. (C) Total RNA (*c20orf20*) or whole cell lysate (*PLK1*) was extracted from SCCIC1 keratinocytes and mRNA or protein levels determined by qPCR or western blot respectively. All results shown represent mean ±SD. **p<0.01, ***p<0.001 compared with control (n=3). See also Figure 10 for SCCRDEB2 siRNA library screen and related data.

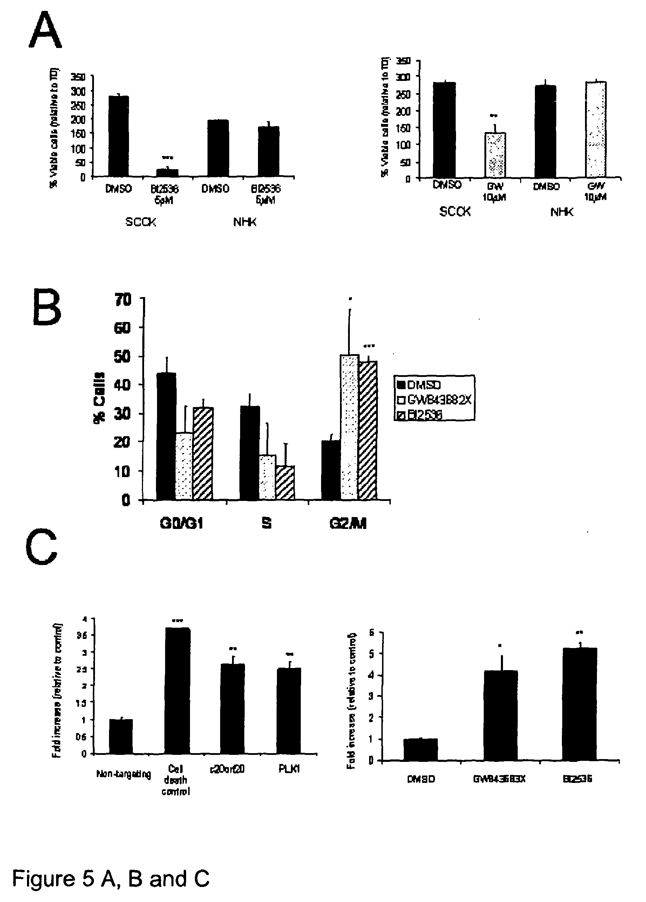

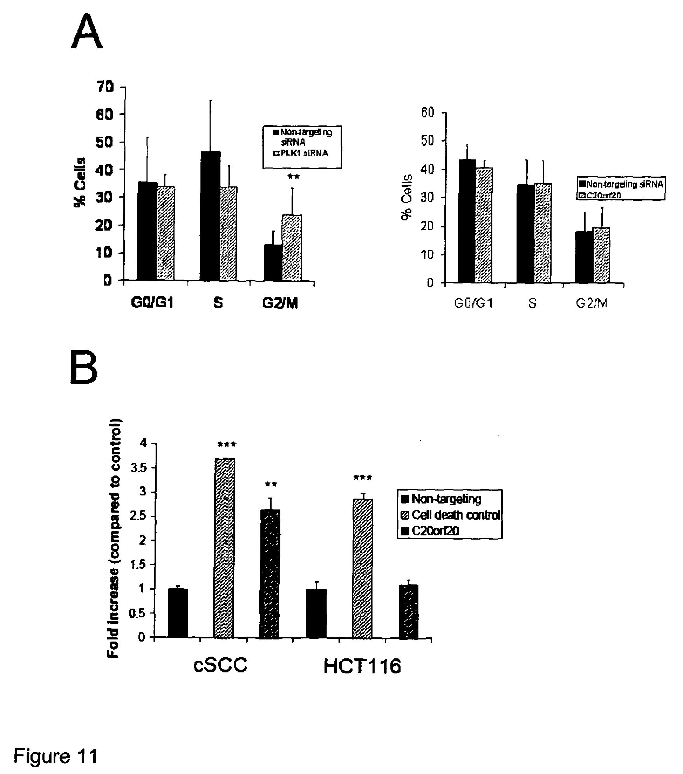

**Figure 5** **- PLK1 inhibition decreases cSCC growth, induces G2M arrest and apoptosis with no effect on normal primary human keratinocytes. (A)** cSCC keratinocytes and NHK were treated for 72 hours with the PLK1 inhibitors GW843682X (10µM) and BI2536 (5□µM) and cell viability assessed by the MTS assay. Percentage viable cell number was calculated relative to values at T0, where <100% represents a net decline in cell number and >100% represents a net increase in cell number. Representatives of a minimum of three experiments are shown. Results shown represent the mean ±SD, n=3. **(B)** Cell cycle analysis was performed on cSCC keratinocytes treated with the PLK1 inhibitors GW843682X (10µM) and BI2536 (5□µM) and stained for BrdU and propidium iodide. Results are expressed as the percentage of cells found at the G0/G1, S and G2/M phases of the cell cycle 20 hours following drug treatment. Results shown are the mean ±SD of 3 independent experiments. See also Figure 11 for cell cycle analysis after *PLK1* and *c20orf20* siRNA transfection (C) cSCC keratinocytes were treated with either the PLK1 inhibitors GW843682X (10µM) and BI2536 (5□µM), or transfected with siRNA to *c20orf20*, *PLK1* and a cell death positive control siRNA. Cells were lysed to release their cytoplasmic contents 24 or 28 hours post-treatment respectively and lysates run in an apoptosis detection ELISA. The number of cleaved nucleosomes released into the cytoplasm, indicative of apoptosis, was then quantitated. Results show the fold increase in absorbance associated with increased cytoplasmic nucleosomes relative to non-targeting siRNA or drug vehicle control respectively. Shown is a representative experiment, with each experiment performed a minimum of three times. Results are the mean ±SD, n=3. *p<0.05, **p<0.01, ***p<0.001 compared with control. See also Figure 11 for a comparison of *c20orf20* siRNA induced apoptosis in cSCC cells and the colon carcinoma line HCT116.

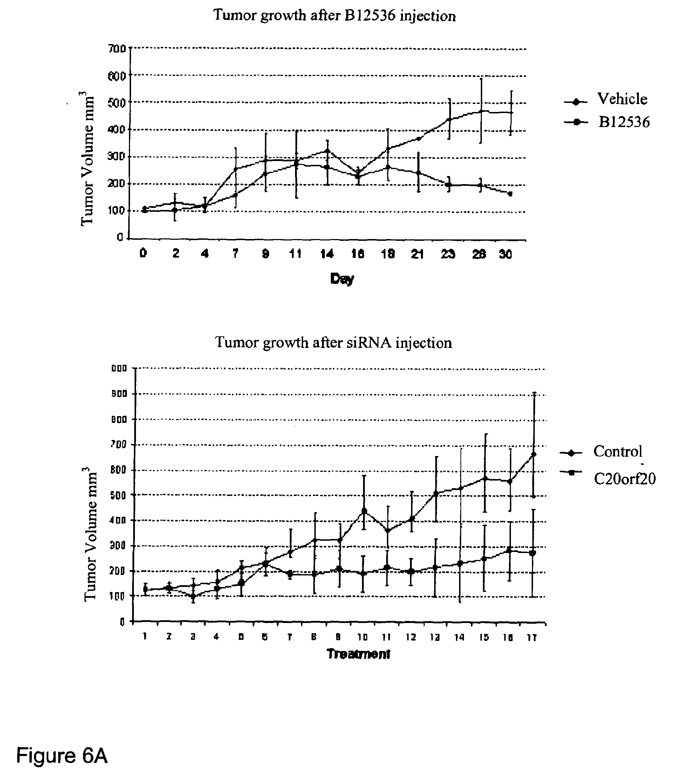

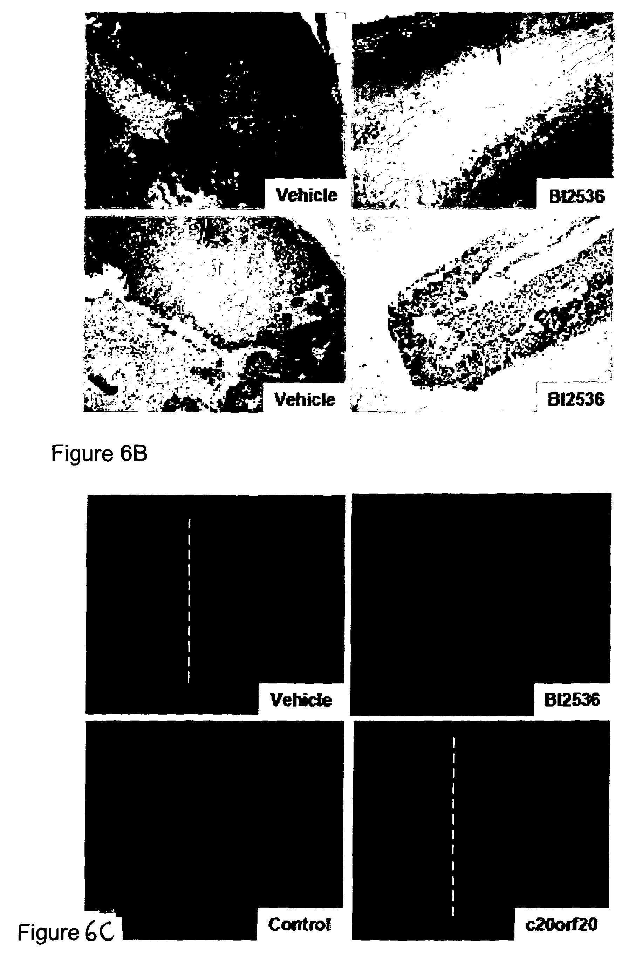

**Figure 6** **- PLK1 inhibition and *c20orf20* knockdown inhibits tumor growth *in vivo.*** Female SCID Balb/c mice were subcutaneously injected in the right flank with 4×10⁶ SCCIC1 cells mixed with high-concentration Matrigel®. When tumors reached a volume of 100mm³, animals were organised in treated and control groups. (A) Top panel: SCCIC1 tumors were treated with the PLK1 inhibitor, BI 2536. The treated group (n=3) was injected into the tumor with 100 µl of BI 2536 formulated in hydrochloric acid (0.1 N), diluted with 0.9% NaCl at a dose of 25mg/kg, 3 times a week during 2 weeks. The control group (n=2) was injected with the vehicle at the same schedule. Tumor volumes were measured 3 times a week with a calliper for a further 2 weeks after treatment. Bottom panel: SCCIC1 tumors were treated with *c20orf20* siRNA. The treated group (n=3) was injected into the tumor with 580pmol *c20orf20* targeting siRNA duplex in 100µl PBS. The control group was injected with 580pmol non-targeting siRNA duplex in approximately 100µl PBS. Each group was treated every other day for 16 cycles. The animals were sacrificed 2 days after the last treatment. **(B)** Representative H&E stained section (top) and keratin immunostained section (bottom) of control (left) and BI2536-treated (right) SCC IC1 tumors after 2 weeks of treatment. A subset of animals was sacrificed at the end of the 6 cycles of treatment without waiting 2 more weeks. Although no difference in tumor volume was seen at this time point between control and treated group, a histological study showed that in the BI2536 treated group, the lump consisted of mainly inert material (keratin) and mesenchymal infiltrating cells without any discernable SCCIC1 tumor cells. The control tumors contained numerous SCCIC1 cells (100X magnification). **(C)** Representative pictures from the experiment shown in (A) of vehicle (top left, tumor bisected showing both halves delineated by the dashed line) and BI2536-treated (top right) SCCIC1 tumors. The largest SCCIC1 tumor treated with *c20orf20* siRNA (bottom right, tumor bisected showing both halves delineated by the dashed line) display smaller size and are hollow in appearance compared with non-targeting siRNA treated tumor (bottom left). Both images are from the experiment shown in (A).
**Figure 7** **- Keratin staining of xenograft tumours identifies keratinocyte origin.** Left panel: H&E stained sections of xenograft tumors Right panel: immunostaining using the anti-keratin antibody MNF 116. 4 µm Sections were immunostained using Vectastain Elite kit and counterstained with hematoxylin.
**Figure 8** **- *In vitro* assays of migration vary greatly in isolated cSCC keratinocyte populations and do not predict xenograft growth (A)** Migration of the cSCC cell populations was assessed using the Transwell assay. After 18 hrs incubation, the non"-migrating cells were removed and the cells in the lower portion of the filter were stained with methylene blue and lysed with 1%SDS solution. Absorbance was measured using a multiplate reader at 630nm. The absorbance for the total number of cells seeded initially was also measured the same way but without removing the non-migrating cells. The results are expressed as a percentage of the total number of cells seeded: (absorbance of migrating cells/absorbance of total number of cells)×100. Results show the mean ±SD of 3 independent experiments performed in triplicate for each population. **(B)** H&E stained 4µm sections of organotypic culture. 5×10⁵ SCC cells were seeded onto a collagen/Matrigel gel containing 2×10⁵ human normal dermal fibroblasts. The organotypic cultures were maintained in keratinocytes medium at an air-liquid interface conditions. After 8 days, the gels were harvested and embedded in paraffin. **(C)** Organotypic invasion was calculated for the 7 populations of cSCC cells. Pictures from sections immunostained with a keratin antibody (MNF116) were processed with Image-Pro® Plus 5.1 software as described in Materials and Methods. Results show the mean ±SD of 3 independent experiments performed in triplicate. **(D)** *In vitro* scratch wound assays were performed to assess the motility of the SCC cells. Confluent monolayers of mitomycin C-treated SCC cells were scratched with a plastic pipette tip and cultured in keratinocyte medium. Pictures were taken at 0h (left panel) and 24 h (right panel).
**Figure 9** **- Gene Ontology and metastasis expression of an incongruent gene set. (A)** Gene ontology analysis of all 154 *in vivo* genes and a subset of 34 of these genes whose expression is oppositely regulated in relation to control, *in vitro* compared with *in vivo* (incongruent gene set). (B) GEO data shown for FLRT3 and SOX4 genes from a large dataset of normal, primary tumour, peri-lesional and metastatic, prostate cancer (GDS2546, http://www.ncbi.nlm.nih.gov/geo/ (Barrett et al., 2009).
**Figure 10** **- siRNA knockdown of *c20orf20, PLK1*, *BDKRB1, GSG2* and *PRSS21* inhibit growth of cSCC keratinocytes. (A)** SCCRDEB2 keratinocytes were transfected with a siRNA library to 21 cSCC specific up-regulated genes (3 siRNA duplexes individually and pooled) and cell viability assessed 48 hours post-transfection using the MTS assay. The percentage of viable cells was calculated relative to values at time zero (TO) and the difference in these values between treated samples and the non-targeting control siRNA are shown. (B) SCCIC1 keratinocytes were transfected with siRNA (3 duplexes each gene) to a set of genes, *BDKRB1*, *GSG2* and *PRSS21*, identified in the screens in addition to *c20orf20* and *PLK1.* The percentage of viable cells relative to values at T0 was calculated and these expressed in the graph as the percentage of control value. Results show the mean ±SD, n=3. *p<0.05, **p<0.01, ***p<0.001 compared with control.
**Figure 11** **- *c20orf20* siRNA induces apoptosis without cell cycle change in cSCC cells. (A)** Cell cycle analysis was performed on cSCC keratinocytes transfected with either *c20orf20* or *PLK1* siRNA (pool of 3 duplexes) and stained for BrdU and propidium iodide. Results are expressed as the percentage of cells found at the G0/G1, S and G2/M phases of the cell cycle 24 hours following transfection. Results shown are the mean ±SD of 3 independent experiments. **(B)** SCCIC1 keratinocytes and HCT116 colorectal carcinoma cells were transfected with *c20orf20* siRNA and a cell death positive control siRNA and apoptosis induction assessed 24 hours following transfection using a cell death detection ELISA. Results show the fold increase in absorbance associated with increased cytoplasmic nucleosomes relative to non-targeting siRNA. Shown is a representative experiment with the experiment performed twice. Results are the mean ±SD, n=3. (C) SCCIC1 keratinocytes were transfected with siRNA to either *c20orf20*, *tip60* or a double knockdown of *c20orf20* and *tip60.* Cell viability was assessed 48 hours post-transfection using the MTS assay. The percentage of viable cells relative to values at T0 was calculated. Results shown are the mean ±SD n=3.
   *p<0.05, **p<0.01, ***p<0.001 compared with control.

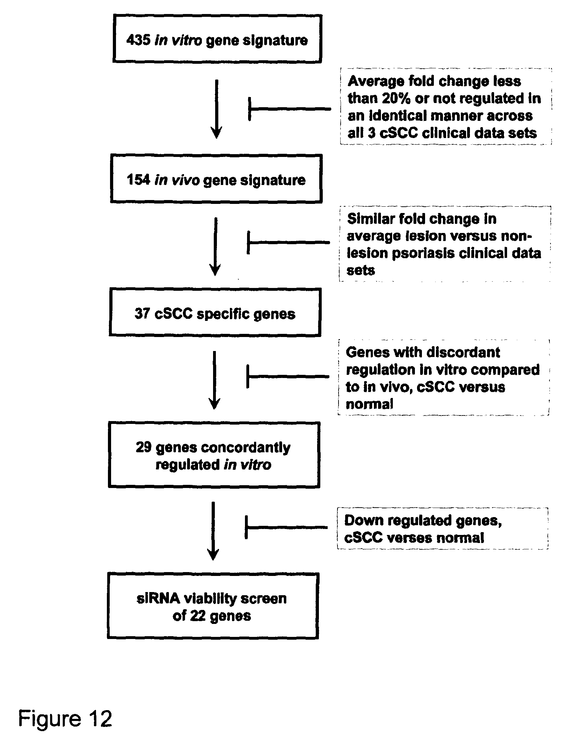

**Figure 12****: Strategy to identify 37 cSCC driver genes and subsequent siRNA screen.** Gene signatures/ groups indicated in blue are derived from the initial 435 *in vitro* signature (top) in a stepwise manner as shown. Subtraction criteria to derive each subsequent signature or grouping are summarized in red.

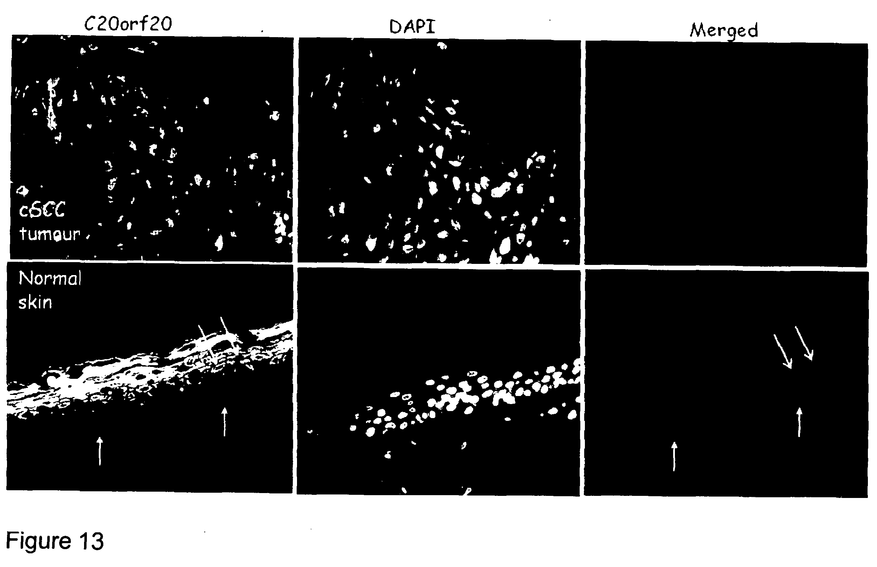

**Figure 13****: cSCC tumor and normal epidermis display contrasting C20orf20 localisation.** Immunofluorescence staining of paraffin embedded sections of cSCC tumor xenografts and normal human skin, using a novel N-terminal peptide polyclonal antibody, reveals abundant nuclear C20orf20 localisation in tumor cells, but a striking cytoplasmic distribution in suprabasal cells of the normal epidermis. C20orf20 levels in the basal compartment appear low with sporadic nuclear expression (indicated by arrows). Left panels sho C20orf20 staining, right panels show merged C20orf20 and DAPI counterstain. Magnification 600x

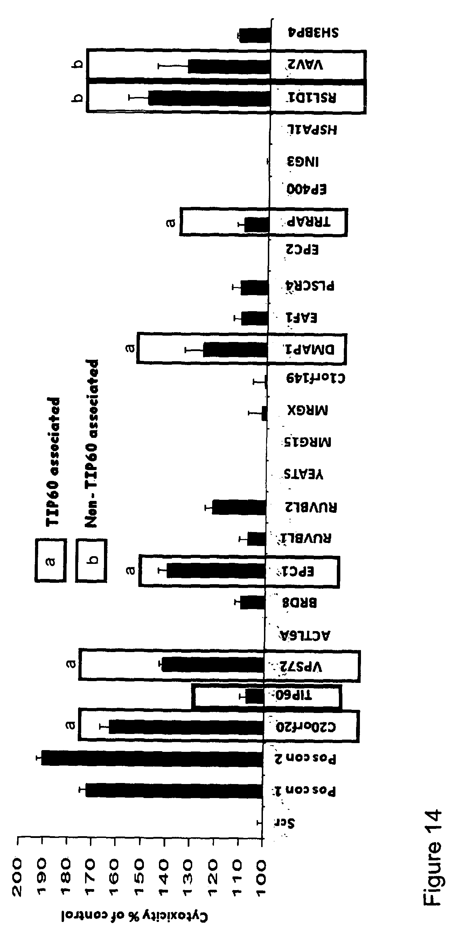

**Figure 14****: C20orf20 and four additional components of the TIP60 HAT complex, not including the catalytic subunit, are required for cSCC cell survival. (A)** To identify potential pro-tumorigenic C20orf20 functional interactions putative binding partners were determined using IntAct (EMBL-HDI) and a RNAi-cytotoxicity screen. Depletion of seven proteins, including C20orf20, induced significant increases in cytotoxicity compared to a non-targeting control siRNA (NT) in all experiments (n=3) across two cSCC cell populations. A cell death siRNA was used as a positive control. Five proteins are known components of the mammalian TIP60 HAT complex (highlighted red), while two are not associated with the TIP60 complex (blue). Interestingely, depletion of the catalytic subunit of TIP60 (green) had little effect. Data shown is 48 hours post-transfection and the mean ±SD n=3.

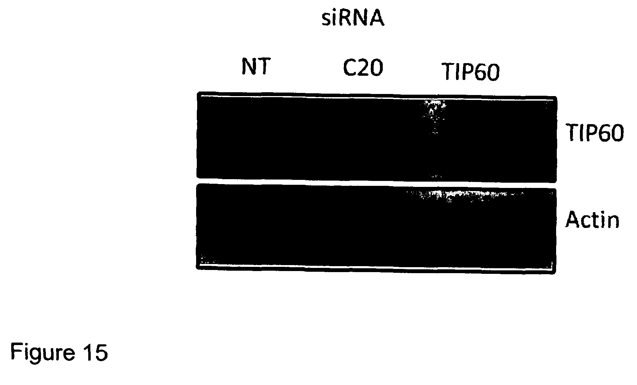

**Figure 15****:** Western blot showing reduction in TIP60 protein levels following C20orf20 siRNA treatment, indicating C20orf20 may regulate TIP60 expression.

### Materials & methods

All human samples were collected after informed consent and in accordance with Helsinki guidelines. All animals were used in accordance with UK Home Office regulations after approval from the University of Dundee ethics committee.

### Keratinocyte isolation, in vivo tumor growth and treatment

Primary keratinocytes were isolated and grown in the presence of a mitotically inactivated 3T3 feeder layer as described (Rheinwald and Beckett, 1981). Tumor populations were verified by SNP mapping (Purdie et al., 2007) or cytogenetic analysis (Cunningham et al., 2002) as described. For tumorigenicity assays, 1-4×10⁶ tumor cells were mixed with high concentration Matrigel® (Becton Dickinson, Oxford, UK) and injected sub-cutaneously into the flanks of SCID balb/c mice. For tumor treatment 4×10⁶ SCCIC1 cells were used. Tumors were measured by caliper and treatment began when volume reached 100mm³.

### Antibodies and Materials

Beta Actin - mAbcam 8226 (Abcam, Cambridge, UK); Akt - #9272, Phospho-Akt (Ser473) - #9271, ERK - #9102, Phospho ERK - #9101, STAT3 - #9139, Phospho-Stat3 (Tyr705) - #9131, PLK1 - #208G4 (Cell Signaling Technology, Inc, CA); Keratin 6 - Ks6.KA12 , Desmocollin 2 - #610120 (Progen, Heidelberg, Germany); c-Myc - 9E10 sc-40, p16 - C20 sc468, p53 - DO-1 sc126 (Santa Cruz Biotechnology, Inc, CA); anti-human cytokeratin antibody MNF116 (Dakocytomation, Glostrup, Denmark); Ras GTPase Cemi ELISA Kit - 52097 (Active Motif, Carlsbad, CA); Ras - #61001 18/Ras, BrdU - #347580 (Becton Dickinson, Franklin Lakes, NJ); BI2536 (Selleck Chemicals LLC, Houston, Tx), GW843682X (Sigma-Aldrich, Dorset, United Kingdom). All siRNA were purchased from Sigma except AllStars Hs Cell Death Control siRNA (Qiagen, Crawley, UK).

### p53 mutation analysis

The entire coding region of the p53 gene was RT-PCR amplified and sequenced as described (Bourdon et al, 2005).

### Proliferation assay

Proliferation was initially calculated using the Cell Proliferation Kit I (MTT) (Roche Diagnostics, West Sussex, UK). Subsequent viability was calculated using an MTS assay (described below).

### Gene expression and analysis

Total RNA was extracted from the cells (passage <7) or frozen tissue sections and purified using the RNeasy Kit (Qiagen, UK) according to the manufacturer's instructions, and hybridized to a Hybridize 6- Sample BeadChip (whole-genome gene expression for BeadStation), V1 arrays were used for the cell culture analysis, V2 arrays were used for the tissue analysis. Cubic-spline normalized signal intensities were generated for each probe using Illumina's BeadStudio Data Analysis Software. Data were analyzed using students T test in Excel (Microsoft). Fold change SCC versus normal for all public data sets analyzed were generated from normalize signal intensities available at the NCBI GEO database (http://www.ncbi.nlm.nih.gov/geo; Edgar et al., 2002) using Excel.

### RNAi screen

For the initial high-throughput RNAi screen we used a custom library to our 21 up regulated genes containing the top 3 siRNA oligonucleotides per gene as ranked by Sigma. In each experiment a negative control (MISSION® siRNA Universal Negative Control #1, Sigma) and positive control (AllStars Hs Cell Death Control siRNA, Qiagen) were used. Cells were seeded in 96-well plates at 5000 cells/well in 1001µl keratinocyte media and transfected 24 hours later with siRNA (40nM final concentration) using Lipofectamine™ 2000, Invitrogen, Carlsbad, CA) diluted in Opti-MEM® (Invitrogen) according to manufacturers instructions. Cell viability was assessed at 48, 72 and 96 hours post-transfection, and a reading taken at time zero (pre-transfection), using the MTS CellTitre 96 AQueous One Solution Cell Proliferation Assay (Promega, Madison, WI) according to manufacturers instructions. Absorbance readings were taken at 490nm using a VersaMax microplate reader (Molecular Devices, Sunnyvale, CA).

### Cell viability and death assays

For RNAi transfection, cells were seeded in 6-well plates at 2.5 x 10⁵ cells/well and 24 hours later transfected as described above. Cells were left for 16 hours then trypsinized, counted on a CASY counter (Roche Diagnostics Ltd, West Sussex, UK) and seeded in 96 well plates at 3000 cells/well in 100µl media. Cell viability was determined using the MTS assay as described above, with an absorbance reading at time of seeding (TO) and readings 48 and 72 hour post transfection. For small molecule inhibitor treatment cells were seeded in 96-well plates at 3000 cells/well and 24 hours later 100µl fresh media containing drug at the designated final concentrations added. Viability was assessed as before.

Apoptosis was detected using the Cell Death Detection ELISA^{PLUS} (Roche Diagnostics Ltd, West Sussex, UK). Cells were seeded in 24-well plates at 0.5 x 10⁵ cells/well for 24 hours and either transfected with siRNA or treated with small molecule inhibitors for 24 or 16 hours respectively before collecting lysates and performing ELISA as described by manufacturer.

### Cell cycle analysis

Cells were RNAi transfected or drug treated for designated times before BrdU (Sigma) was added at 30µM final volume for 20 min. Cells were collected and fixed by dropping 1ml cell suspension in PBS into 3ml ice cold ethanol while vortexing. Pepsin (Sigma) was added at 1mg/ml in 30mM HCL for 30 min and DNA denatured with 2N HCL for 20 min. Anti-BrdU antibody (Becton Dickinson) diluted in PBS/0.5% Tween/0.5% BSA was added for one hour followed by 30 min incubation with a FITC-sheep anti-mouse IgG (Sigma). Propidium iodide (Sigma) was added in the final wash step at a concentration of 25µg/ml and samples analyzed using a FACScan flow cytometer and CellQuest software (Becton Dickinson).

### RNA preparation and real-time quantitative PCR

Total RNA was extracted using RNA Bee (Amsbio, Abingdon, UK) and RNeasy columns (Qiagen) according to manufacturers' instructions. 5µg RNA was incubated with random primers and M-MLV reverse transcriptase (Promega) to generate cDNA. For quantitative measurement of *c20orf20* mRNA, SYBR Green Master Mix (Applied Biosystems, Warrington, UK) was used with the following primers: 5'-ATTCTTCCATTCCCGAATCC-3' and 5'-CCCAAACTCCCTGAAGATGA-3' (Eurofins MWG Operon, Germany). Primers 5'-GAGAGCTTCTCAGACTTATCC-3' and 5'-GTCCACTGCTTTGATGACAC-3' to *EF1α* were used as an internal control. PCR reactions were carried out on a MiniOpticon Real-Time PCR detection system (Bio-Rad, Hertfordshire, UK) and expression calculated by the ΔΔCT method (Livak and Schmittgen, 2001).

### Supplementary procedures

### Scratch wound migration assay

Cells were grown to confluency in a 6-well plate in keratinocyte medium. 2 h before wounding cells were treated with mitomycin C (10 µg/ml) to prevent proliferation. Cells were washed with PBS and a wound was made by applying a 1000 µl plastic pipette tip across the centre of the cell sheet. Cells were washed twice with PBS and incubated in keratinocyte medium.

### Transwell migration assay

A Transwell system that incorporated a polycarbonate filter membrane with a diameter of 6.5 mm and pore size of 8 11µm (Coming, Sigma-Aldrich, Poole, UK) was used to assess the rate of cell migration. Mitomycin C-treated cells (1×10⁵) were suspended in 100 µl of 0.1% BSA DMEM/HamF12 (3:1 V/V) and seeded in the upper chamber of the Transwell insert. The lower chamber was filled with 600 µl of DMEM/HamF12 (3:1 V/V) supplemented with 5% FBS, 0.4 µg/ml hydrocortisone, 5 µg/ml insulin, 10 ng/ml EGF, 5 µg/ml transferrin, 8.4 ng/ml cholera toxin and 13 ng/ml liothyronine. Following 18 h of incubation at 37°C, nonmigrating cells on the upper surface of the filter were removed with a cotton swab. Cells that migrated to the lower surface of the filter were stained with 1% Borax and 1% methylene blue before being lysed with a solution of 1% SDS. Absorbance was measured with a microplate spectrophotometer at 630 nm. Migration rate was calculated with the following equation (OD of the migrating cells/OD of total number of cells seeded) × 100%.

### 3-D organotypic culture

To prepare collagen/Matrigel® gels, 3.5 volumes of collagen type I (Marathon Laboratory Supplies, London, UK) were mixed on ice with 3.5 volumes of Matrigel® (Becton-Dickinson, Oxford, UK), 1 volume 10× DMEM, 1 volume FBS and 1 volume 10% FBS DMEM in which normal human fibroblasts (NHF) had been suspended at a concentration of 2 × 10⁶/ml. The solution was equilibrated with 1M NaOH and 1 ml of this solution (2 × 10⁵ NHF) was cast into wells of a 24-well plate and allowed to polymerise for 30 min at 37°C. After polymerization, the gels were detached from the well with a plastic pipette tip, 1ml of 10% FBS DMEM was added per well and gels were left overnight at 37°C. Next day, medium was aspirated and 5 × 10⁵ keratinocytes (suspended in keratinocyte medium) were added in a clonal cylinder (9.5mm×11mm, Sigma-Aldrich, Poole, UK) placed on the top of each gel. The following day, gels were lifted on steel grids. Sufficient keratinocyte medium was added to reach the undersurface of the gel allowing the epithelial layer to grow at an air-liquid interface. Medium was changed twice a week.

After 8 days, the gels were harvested, fixed in paraformaldehyde and embedded in paraffin. Sections of 4 µm were immunostained with the anti-human cytokeratin antibody MNF116 (Dakocytomation, Glostrup, Denmark) using Vectastain Elite kit (Vector Laboratories, Peterborough, UK).

### Quantitative analysis

An invasion index was calculated as previously reported (Nystrom et al., 2005). Briefly, digital images of keratin immunostained sections were analysed using Image-Pro® Plus 5.1 software (Media Cybernetics, Bethesda, MD, USA). The digital images were converted to greyscale and immunostained areas were converted to saturated red particles using the threshold function. This was subjected to two 'cleanup' procedures: the first to remove all particles less than ten pixels in size using the "select measurement" function. Next, any artifact was manually removed by comparing the processed image to the immunostained image. The main event representing the epidermis was removed. The saturated image was then virtually split in 4 zones of 500 pixels in its width and the lengths of invasion of the deepest event were measured in each of the zones and were used to determine the average length of invasion (A). The number of events (B) as well as the sum of the area of these events (C) were also calculated. The invasion index was determined by AxBxC.

### GO analysis

Gene ontology was curated manually based on literature searches using PubMed at the NCBI website.

### Results

Primary keratinocytes derived from cutaneous squamous cell carcinoma readily form *in vivo* tumors with histological features of cSCC

In order to model human cSCC without the need for genetic manipulation we isolated keratinocytes directly from fresh human tumor material as described (Rheinwald and Beckett, 1981). Because we wanted to study life threatening cSCC we isolated keratinocytes from tumors which presented with metastasis derived from immuno-competent and immuno-suppressed patients (UV induced SCC) and also tumors derived from patients with recessive dystrophic epidermolysis bullosa (RDEB), an inherited skin blistering disease where cSCC are aggressive and frequently lead to mortality (Fine et al., 2009). For comparison we used cSCC keratinocytes isolated from well differentiated tumors which did not present with metastasis and from non-SCC primary epidermal keratinocytes. Previous studies demonstrate that tumor keratinocytes can be identified through long term proliferative capacity *in vitro* with retention of primary tumor genetic alterations determined through SNP mapping (Purdie et al., 2007). All tumor keratinocytes used in this study showed clear genetic alterations as determined by 10K or 250K SNP mapping array hybridization and cytogenetic analysis (data not shown).

5/8 tumor keratinocyte populations readily formed tumors in SCID mice, 1/8 of the populations consistently formed squamous cysts which failed to reach 100mm³ volume during the experiment and 2/8 tumor populations tested did not grow (Figure 1A). Xenograft tumors were readily recognized as human cSCC except in the case of SCCT8 where the tumor population had pronounced spindle cell morphology (Figure 1B); carcinosarcoma was considered although the cells displayed immunolabelling with a keratin antibody thereby indicating a diagnosis of poorly differentiated spindle cell cSCC (Figure 7). Table 1 details the patient donors used for our *in vitro* studies. *In vivo* growth was not restricted to moderately or poorly differentiated tumors or those derived from patients with RDEB, as demonstrated by cSCC keratinocytes derived from a well differentiated tumor (SCCT2) and by an RDEB cSCC xenograft tumor displaying features of keratoancathoma (a variant of well differentiated cSCC, SCCRDEB3, Figure 1B and Table 1).

**Table 1:**

| **Table 1: Patient details and *p53* mutation**. **All mutations were homozygous with the exception of SCCIC1.** | | | | | | |
|---|---|---|---|---|---|---|
| Cells | Patient | Age | Sex | Primary Tumor Histology | Metastasis | *p53* mutation |
| SCCIC1 | Immuno-competent | 77 | M | Right temple. Mod. differentiated. | Yes | p.H179Y, p.R248Y |
| SCCT1 | Renal transplant. | 61 | M | Forearm. Well differentiated. | No | p.Y234S |
| SCCT2 | Cardiac transplant. | 66 | M | Hand. Well differentiated. | No | p.P278F |
| SCCT3 | Renal transplant. | 55 | | Hand. Mod. differentiated recurrence | Yes | p.V216M |
| SCCT8 | Renal transplant. | 67 | M | Ear. Poorly differentiated. | Yes | p.Y91G |
| SCCRDEB2 | **RDEB (COL7A1 c**.**3832**-1**G>A; unknown**) | 54 | M | Poorly differentiated. | No | p.V173L |
| SCCRDEB3 | **RDEB (*COL7A1* p**.**R525X; p**.**R578X**) | 36 | F | Left forearm. Moderately Differentiated | No | p.R273H |
| SCCRDEB4 | **RDEB (*COL7A1* c**.**8244insC; c**.**8244insC** | 32 | F | Shoulder. N/A | N/A | p.P152L |
| SCCRDEB5 | **RDEB (*COL7A1* p.R1632X; c**.**3551**-**3 T>G** | 28 | F | Moderately differentiated. | Yes | N/A |

### cSCC keratinocytes harbor p53 mutation, express increased levels of c-myc and phospho-STAT3 but do not display features of activated RAS

We investigated genes, proteins and pathways reported to be important in both human and mouse for the development of SCC and looked for patterns which might separate xenograft tumor forming capability or patient group. p53 and p16 expression varied amongst cSCC keratinocytes (Figure 2A) and we detected *p53* mutations in all 8 populations examined (Table 1). C-myc expression was consistently increased across all cSCC compared with primary human keratinocytes as was phospho-STAT3 expression (Figure 2B and C). As we and others have previously reported lack of activating RAS mutations in SCC (Clark et al., 1993; Pourreyron et al., 2007), we examined evidence for RAS activation using ELISA, phospho-ERK and phospho-AKT antibody staining. No consistent evidence of activated RAS was observed in tumorigenic cultured cSCC keratinocytes compared with normal primary or non-tumorigenic cSCC human keratinocytes (Figure 2D and E).

### In vitro assays of cSCC keratinocyte migration and invasion are unable to predict tumor forming ability or patient donor

In order to assess whether an *in vitro* assay could be a surrogate for tumor forming capacity or identify clinically aggressive cSCC we compared migration and invasion using transwell migration, scratch wound migration and 3-dimensional organotypic cultures. Transwell migration varied greatly among cSCC keratinocyte populations as did invasion into organotypic cultures (Figure 8). Ability to invade in organotypic assays or to migrate in Transwell and scratch wound assays did not correlate with tumor forming capacity as evident comparing SCCIC1, SCCRDEB3 and SCCT8 (Figure 1A and Figure 8).

### Comparison of gene expression with normal primary keratinocytes identifies a 435 cSCC keratinocyte gene signature in culture

To identify differences at the mRNA level between cSCC and non-SCC keratinocytes we performed gene expression analysis using quiescent cultures of early passage primary cells. We chose to use confluent cultures to best mimic close cell-cell proximity of keratinocytes *in vivo* (both cSCC and non-SCC) and to eliminate changes in gene expression caused by divergent proliferation rates. MTT assay confirmed that at the point of RNA isolation cultures were quiescent (Figure 3A). Unsupervised clustering of normalized signal intensities clearly segregated normal skin from cSCC (Figure 3B). Pairwise comparison of disease state, histology of primary tumor or xenograft, or tumor forming ability, revealed the highest significant number of differentially expressed genes in this assay were identified comparing cSCC with non-cSCC cultures (Table S1). This analysis defined a 435 gene *in vitro* cSCC signature (Table S2).

### 35% of in vitro cSCC genes are expressed concordantly across 3 independent in vivo mRNA expression data sets

To identify clinically relevant genes from our 435 *in vitro* cSCC signature we analyzed the expression of probes representing each of the 435 genes in 3 separate tissue expression data sets containing primary cSCC and normal skin samples. We performed our own experiment comparing RNA isolated from fresh frozen cSCC (n=9) and non-SCC (n=5) skin samples and interrogated data from two publicly available experiments containing cSCC and normal skin samples using separate array platforms (GDS2200 (Nindl et al., 2006) and GSE7553 (Riker et al., 2008) respectively). In agreement with recent observations that little overlap exists between gene expression profiling of cutaneous or HNSCC when stringent filtering criteria are applied (Braakhuis et al., 2010; Van Haren et al., 2009), probes representing only 6 of our 435 gene signature were returned as differentially expressed genes across all three *in vivo* data sets based on fold change> 2 and p<0.005. However, when using a fold change of 20% increase or decrease in expression, probes representing 154 of the 435 genes were concordantly expressed across all datasets in the three separate array platforms (Table S3(data not shown)) suggesting that a large proportion of the genes identified in culture are relevant to cSCC pathology. We designated this 154 gene set as an *"in vivo* cSCC signature" and noted that 37 of these were recognized as differentially expressed genes based on fold change > 2 and p <0.005 in at least one of the three data sets (Table S4 (data not shown)).

### Subtraction comparison with the benign hyper-proliferation disorder psoriasis identifies 37/154 in vivo genes as potential drivers of cSCC

Those genes which are differentially expressed comparing cSCC and normal skin can be either a driver or a consequence of disease state. One approach to identifying tumor specific changes is to compare expression profiles with comparable benign conditions. Psoriasis offers such a point of comparison as this disease, though completely benign, harbors massive hyper-proliferation, along with concomitant cell cycle activation, up-regulation of signaling pathways driving keratinocyte migration, as well as a reactive inflammatory response (Haider et al., 2006). Indeed, comparison of fold change psoriasis versus normal (average of data sets GSE13355 and GSE14905, (Romanowska et al., 2010) with fold change cSCC versus normal (average of the three *in vivo* data sets in Table S3 (data not shown)) revealed a striking relationship between expression (r²=0.8) indicating that the majority of our *in vivo* cSCC signature were dysregulated analogously in psoriasis (Figure 3C and Table S5). Of the 37 differentially expressed genes (fold change> 2 and p<0.005) within the *in vivo* cSCC signature there was an even greater correlation with psoriasis (r²=0.94, data not shown) indicating that highly significant markers of cSCC are shared with psoriasis. Intriguingly, however, a separate 37 from the 154 *in vivo* cSCC genes did not show similar fold change in psoriasis (Figure 3C, Table S6) and were designated "cSCC specific" with potential driver-like properties.

### 22% of in vivo cSCC genes are differentially regulated in vitro compared to normal

Of the 37 potential drivers of cSCC only 29 were similarly differentially regulated *in vitro* and *in vivo*; 8 genes were differentially expressed (SCC versus normal) in an opposite manner in cultured keratinocytes compared with tissue (Table S6). This percentage was similar when we examined the expression of all 154 *in vivo* cSCC genes: 34 (22%) were discordantly regulated *in vitro* compared with *in vivo* (Table S5). Gene ontology analysis demonstrated that these discordantly expressed genes were disproportionately involved in cytoskeleton or signal transduction compared with all 154 genes, suggesting that cellular context is important for their expression. We predicted that if these genes did respond to cellular context then the 8 cSCC specific discordantly expressed genes may be differentially regulated in metastasis. To investigate this possibility we analyzed their expression in a dataset comparing prostate primary, peri-lesional, normal and matched metastasis (GDS 2546, (Yu et al., 2004)). We noted that 4 out of 5 of the cSCC specific discordantly expressed genes present in this data set demonstrated a clear difference of expression in metastatic tissue (Figure 9). However, because of discordant expression we chose not to pursue those cSCC specific genes whose *in vitro* expression, compared with control, did not represent the same regulation in tissue.

### RNAi screen in cultured keratinocytes identifies PLK1 and c20orf20 as genes critical for tumor cell survival

As 21 of the remaining 29 cSCC specific genes were up-regulated in tumor keratinocytes and cSCC tissue, we screened two cSCC keratinocyte populations, SCCIC1 and SCCRDEB2, by siRNA knockdown of each gene individually with 3 separate duplex sequences and assessing cell viability by the colorometric MTS assay. All 3 duplexes, individually and pooled, targeting *PLK1* and *c20orf20* consistently reduced cell viability compared with controls in a high-throughput format (Figure 4A and Figure 10). Further to this, 3 additional potential targets (*GSG2, BDKRB1* and *PRSS21*) were identified for follow-on studies by demonstrating 'hits' consistently with 2/3 siRNAs (Figure 10).

### PLK1 knockdown and inhibition induces G2/M arrest and apoptosis in cSCC keratinocytes with no effect on normal keratinocytes

The ser/thr kinase Polo-like kinase 1 (PLK1) is an important regulator of mitosis which is overexpressed in a number of cancers (Takai et al., 2005). Targeted depletion or inhibition of PLK1 has been shown to cause G2/M arrest and induction of apoptosis in tumor cells without affecting normal cells (Liu et al., 2006; Schmit and Ahmad, 2007). Here, both RNAi-mediated depletion of PLK1 and activity inhibition with the small molecule inhibitors BI2536 and GW843682X resulted in a potent reduction of cell viability in cSCC cells with no effect on the growth of normal primary keratinocytes (Figures 4B and 5A). Cell cycle analysis revealed an accumulation of cells at the G2/M phase following PLK1 inhibition and depletion (Figures 5B and 11A) and a cell death detection ELISA demonstrated a substantial induction of apoptosis, through an increase in cleaved nucleosomes in the cytoplasm, following both PLK1 inhibition and depletion in cSCC cells (Figure 5C). Together these data correlate with results in other cancers and show that reduction or inhibition of PLK1 results in the induction of apoptosis in cSCC keratinocytes whilst having little effect on normal keratinocytes.

### C20orf20 knockdown induces apoptosis in cSCC cells with no obvious cell cycle arrest and does not effect normal keratinocyte growth

The chromosomal segment harboring *c20orf20* has been identified as frequently amplified in both colorectal cancer (Carvalho et al., 2009) and cervical cancer (Scotto et al., 2008) and most recently, in parallel to our work, *c20orf20* was identified as being over-expressed in colorectal cancer (Yamaguchi et al., 2010). This study also showed that a reduction of *c20orf20* expression through stable shRNA inhibited proliferation in the colon carcinoma lines HCT116 and SW480 (demonstrated by a 10% decrease in S phase replicating cells). No evidence of apoptosis in response to *c20orf20* depletion was observed (Yamaguchi et al., 2010). We sought to investigate whether *c20orf20* knockdown yielded similar results in cSCC and whether it had any effect on normal keratinocytes. *c20orf20* knockdown in cSCC resulted in reduced cell viability assessed by MTS assay (Figure 4B). Further to this, no effects on cell proliferation were seen in normal keratinocytes (Figure 4B), but depletion in cSCC induced a significant apoptotic response in the absence of any change in cell cycle parameters (Figure 5C and 11A), the opposite to that seen in colorectal cancer cell lines. In our hands, and using siRNA, knockdown of *c20orf20* in HCT116 cells did not demonstrate a significant increase in apoptosis (Figure 11B), in agreement with the previous study (Yamaguchi et al., 2010), indicating either a more potent effect in cSCC or a different mode of action in different tumor types. As c20orf20 forms part of the TIP60 complex we tested whether the effects seen with *c20orf20* knockdown were mediated through the TIP60 HAT complex itself. To do this the TIP60 catalytic subunit (KAT5) was depleted by siRNA and cell viability assessed by MTS assay. Although Tip60 knockdown moderately reduced the proliferation of SCCIC1 and SCCRDEB2 it was far less potent than the depletion of *c20orf20* alone. In addition, double knockdown of *c20orf20* and Tip60 neither positively nor negatively influenced the effect of *c20orf20* (Figure 11C). Together this suggests that the increased cell death/decreased proliferation seen upon *c20orf20* depletion is not mediated simply through its effect on the TIP60 HAT complex.

### PLK1 inhibition and c20orf20 siRNA knockdown target cSCC in vivo

In order to assess the *in vivo* action of PLK1 inhibition and *c20orf20* depletion we injected either the PLK1 inhibitor BI2536 (Steegmaier et al., 2007) or *c20orf20* targeting siRNA into established SCCIC1 xenograft tumors. In each case we saw direct evidence of effective tumor targeting (Figure 6). In as little as two weeks, tumors harvested from animals treated with BI2536 showed marked reduction in the presence of tumor keratinocytes compared with vehicle controls (Figure 6B). Treatment with *c20orf20* siRNA reduced tumor volume over time compared with a non-targeting siRNA control (Figure 6A). The largest *c20orf20* siRNA treated tumors showed a marked reduction in the number of tumor keratinocytes present and were hollow in appearance (Figure 6C).

### Discussion

Our approach to target identification has not only yielded definite therapeutic targets for cSCC in the form of *PLK1* and *c20orf20* (Figure 6) but also suggests *BDKRB1, GSG2,* and *PRSS21* may hold similar potential (Figure 10B). The discovery that PLK1 is overexpressed and required for survival in cSCC cells is encouraging since similar observations in a number of different tumor types are linked to poor prognosis (Takai et al., 2005). In agreement with our findings a recent study has shown PLK1 overexpression in cSCC using immunohistochemical staining of skin tissue arrays (Schmit et al., 2009). Here, we have demonstrated that cSCC keratinocytes undergo the established hallmarks of PLK1 inhibition and depletion; mitotic arrest, inhibition of proliferation and apoptosis, and as reported previously, cell death occurs preferentially in cancer cells compared to normal cells, thus providing a potential therapeutic window (Liu et al., 2006; Schmit and Ahmad, 2007). Normal cells require the knockdown of p53 in addition to PLK1 to invoke cell death (Liu et al., 2006), and various reports suggest increased sensitivity to PLK1 inhibition when p53 is defective (Degenhardt and Lampkin, 2010; Guan et al., 2005). This is especially pertinent in the case of cSCC as both our data (Table 1) and that of others show that the majority of cSCC harbor p53 mutation (Giglia-Mari and Sarasin, 2003), making this disease a prime candidate for PLK1 targeting. The potential of PLK1 as a therapeutic target that could be fast-tracked into human trials for cSCC is enhanced by the fact that a number of small molecule inhibitors are already in clinical development (Degenhardt and Lampkin, 2010; Schoffski, 2009). Among these, the inhibitor BI2536 has progressed to phase II trials for both hematological and solid tumor malignancies (http://www.clinicaltrials.gov) and is shown here to have dramatic efficacy in treating cSCC *in vivo.* Together, these data suggest that targeting PLK1 has great promise for an effective cSCC therapy.

Our observation that knockdown of *c20orf20* can induce apoptosis and reduce tumor growth in cSCC highlights this gene, and the histone acetyltransferase complex (TIP60) which it associates with, as potential targets for therapeutic development. The evidence presented here provides a much stronger argument for their targeting in cSCC than in colon cancer, as advocated by Yamaguchi and colleagues (Yamaguchi et al., 2010). *c20orf20* depletion in cSCC resulted in reduced cell viability attributable to induction of apoptosis at levels comparable with PLK1 knockdown, and without any effect on the cell cycle (Figures 4B, 5C and 11A). This is in contrast to the data in colorectal cancer cells where a reduction in proliferation was observed without engagement of apoptosis. In addition, normal keratinocytes, which express this gene at very low levels (Figure 4C), remain unaffected by *c20orf20* knockdown (Figure 4B).

*c20orf20* was first identified as a protein capable of binding to two components of the TIP60 HAT complex, MRG15 and MRGX (Bertram and Pereira-Smith, 2001; Cai et al., 2003), and although little functional data exists on c20orf20, potential for a role in transcriptional control and/or the DNA damage response exists. MRG15 and MRGX are stable components of both HAT and HDAC complexes, and overexpression of *c20orf20,* which specifically associates with TIP60 HAT, has been shown to increase their protein levels, indicating regulation of stability and/or synthesis (Hayakawa et al., 2007). As a result it has been suggested that *c20orf20* influences the acetylation of histones and potentially transcription factors such as p53 (Gu and Roeder, 1997) and cMYC (Patel et al., 2004), by controlling the balance of MRG proteins associated with either TIP60 HAT or HDAC complexes (Hayakawa et al, 2007). In the study by Yamaguchi and colleagues knockdown of another c20orf20 binding partner, *BRD8,* also a component of the TIP60 HAT complex, produced effects similar to *c20orf20* depletion (Yamaguchi et al., 2010). It is possible that the effects of c20orf20 are mediated through interaction with other HAT complex proteins and it will be necessary to systematically knockdown these components to identify functional partners specific to cSCC. Because of the increased potency seen in cSCC compared to colon cancer it is tempting to speculate that *c20orf20* is 'wired' differently in different tumor types, leading to varied responses upon reduced expression. It should also be noted that expression at the mRNA level was around 2-fold higher in cSCC cells compared to HCT116 (data not shown), perhaps indicating a greater *c20orf20*-dependent pro-survival drive in cSCC than in colon carcinoma. Our results also suggest that simply reducing the expression of the catalytic subunit of the TIP60 complex, *KAT5,* does not impair the effect of *c20orf20* depletion, nor does it reduce cell viability as markedly. It will be important to investigate the mode of action for *c20orf20-*specific apoptotic induction to clarify the potential as a cancer target.

By identifying both *PLK1* and *c20orf20* as demonstrable cancer targets we re-enforce the notion that although cultured tumor cells may fall short of faithfully replicating the complex nature of human cancers they are nevertheless invaluable in our goal to understand and ultimately treat this disease (Masters, 2000; Sharma et al., 2010). The majority of arguments against the use of cultured cells to investigate tumor biology are based on the marked differences seen in the expression profiling of cultured cancer cells compared directly with tumor tissue (Dairkee et al., 2004; Perou et al., 1999; Ross et al., 2000; Welsh et al., 2001). Few examples exist where normal cells are included in such analysis and in these cases, expression profiles in culture, as would be expected from disparate environments, cluster separately from tissue (Perou et al., 1999). Analysis has been restricted to comparing all profiling, cultured and tissue samples, in the search for significant differentially expressed genes and will have also overlooked differential expression relative to controls *in vitro* compared with *in vivo* (Figure 9 and Table S5).

The use of quiescent, confluent cultures represents a departure from traditional *in vitro* mRNA expression experiments which utilize log phase growing cultures, typically considered "healthy" (Perou et al., 1999; Welsh et al., 2001). This was prompted by observations that junctional complexes in keratinocytes can take 48 hours to mature in culture (South et al., 2003; Wallis et al., 2000) and that varying cell-cell adhesion can modulate numerous signaling cascades (Wu and Bonavida, 2009). By using cultured material we have been able to assess gene expression in the absence of surrounding microenvironment and supported on plastic by the cells own matrix. Although it is well documented that this does not reflect the situation *in vivo* (Creighton et al., 2003; Weaver et al., 1997), it has enabled us to compare tumor with normal in the absence of variation resulting from tumor heterogeneity. In doing so we make the following observations: RDEB cSCC keratinocytes possess similar expression profiles to other cSCC in this assay, indicating common initiation and maintenance pathways and, even after using a quiescent *in vitro* model, many of the "cSCC specific" genes identified are involved in cell cycle and proliferation (*BUB1*, *PLK1 , CDC25C, WDHD1*; Table S6), thus in keeping with features common to all cancers (Hanahan and Weinberg, 2000) and suggesting that dysregulation of the cell cycle is apparent even in the absence of proliferation.

Subtracting similar changes in psoriatic lesional skin versus normal is a method previously used (Haider et al., 2006), but here we collate data from five independent experiments and define consistent changes rather than those based on stringent selection criteria. Such an approach has been identified as useful for integrating data sets (Shi et al., 2008). Out of the 154 *in vivo* cSCC genes, 118 were similarly regulated in psoriasis with a strong correlation (r²=0.8), suggesting that our *in vivo* gene signature is not derived by chance, that the 118 genes in common play important roles in both proliferative conditions, and points to the 37 "cSCC driver" genes as being specific to tumor phenotype. Of these 37 cSCC specific genes, we were unable to identify commonality with pathways previously shown to be important in cSCC, data which is in agreement with our biochemical analysis (Figure 2).

In summary, we have used an integrative approach including expression profiling and *in vivo* assays to identify novel targets in cSCC. We hope this work will lead to the use of PLK1 inhibitors in the treatment of cSCC and to the development of targeted therapies based around the biology of c20orf20.
Tables S1, S2, S5 and S6

**Table S1: Numbers of probes returned from pairwise comparisons of averaged cubic-spline normalised signal intensities**

| | | |
|---|---|---|
| p<0.005 indicates Student T test significance between comparison groups. Mod/Poor vs Well Differentiated excludes SCCRDEB3 (no histology available) and tumor vs non-tumor comparison excludes RDEBSCC4 (tumourgenicity not tested). All other comparisons are inclusive. | | |
| Flitered probes returned meeting the following 3 criteria: | | |
| A = p<0.001, FC>2.5 expression above detection threshold of 10 | | |
| B = p<0.001, FC>1.5 with intermediate expression (signal intensity >50) | | |
| C = p<0.005, FC>2.5 with high expression (signal intensity >100) | | |

| **Comparison** | **p<0.005** | **Filtered** |
|---|---|---|
| All cSCC vs All Control | 1045 | 446 |
| Mod/Poor vs Well Differentiated | 471 | 233 |
| RDEBSCC vs non-RDEB SCC | 239 | 18 |
| NHK vs RDEBK | 428 | 82 |
| Tumor vs non-tumor forming | 220 | 12 |

**Table S2: "cSCC in vitro gene siganture" of 446 probes representing 435 genes meeting criteria A, B and C given in Supplementary Table 1.**

| Illuimina probe ID, average signal intensities for all cSCC and all controls, Student T-TEST p value, relative fold change and gene symbol as described for the array at the time are shown. | | | | | |
|---|---|---|---|---|---|
| **Probe ID** | **SCC** | **Control** | **p value** | **Fold Change** | **GENE** |
| GI_42741647-S | 721 | 436 | 2.69E-05 | 1.7 | *SEP15* |
| GI_30795236-A | 76 | 359 | 1.00E-03 | 0.2 | *ABCA12* |
| GI_33469972-S | 51 | 112 | 2.13E-04 | 0.5 | *ABHD5* |
| GI_5174428-S | 11 | 47 | 3.44E-03 | 0.2 | *ACAA2* |
| GI_6138970-S | 64 | 298 | 1.09E-04 | 0.2 | *ACP5* |
| GI_11496989-S | 355 | 142 | 3.49E-04 | 2.5 | *ADPRT* |
| GI_4557302-S | 38 | 96 | 4.80E-04 | 0.4 | *ALDH3A2* |
| GI_42716312-S | 27 | 68 | 4.60E-03 | 0.4 | *ANG* |
| GI_31657093-S | 171 | 52 | 2.61E-03 | 3.3 | *ANLN* |
| GI_5454087-S | 1001 | 598 | 2.12E-04 | 1.7 | *ANP32B* |
| GI_18375500-I | 44 | 16 | 2.37E-03 | 2.8 | *APEX1* |
| GI_31541940-S | 43 | 108 | 2.33E-04 | 0.4 | *APG-1* |
| GI_4557324-S | 26 | 103 | 3.46E-03 | 0.2 | *APOE* |
| GI_11038652-S | 2 | 83 | 1.96E-03 | 0.0 | *AQP9* |
| GI_10835001-S | 849 | 326 | 4.02E-03 | 2.6 | *ARHGDIB* |
| GI_21327678-S | 2217 | 1447 | 6.57E-04 | 1.5 | *ATP5E* |
| GI_19913427-S | 240 | 495 | 3.07E-04 | 0.5 | *ATP6V1B2* |
| GI_4759177-S | 326 | 106 | 3.59E-03 | 3.1 | *AURKB* |
| GI_11038645-S | 107 | 42 | 1.26E-03 | 2.5 | *BANF1* |
| GI_4502368-S | 31 | 228 | 1.29E-03 | 0.1 | *BBOX1* |
| GI_17402869-I | 48 | 19 | 1.49E-03 | 2.5 | *BCCIP* |
| GI_20336304-I | 26 | 82 | 3.02E-03 | 0.3 | *BCL11A* |
| GI_34147602-S | 32 | 13 | 4.11E-04 | 2.5 | *BCS1L* |
| GI_20544171-S | 37 | 4 | 6.58E-04 | 8.4 | *BDKRB1* |
| GI_4503298-S | 283 | 741 | 9.71E-06 | 0.4 | *BHLHB2* |
| GI_4502144-S | 188 | 47 | 3.53E-03 | 4.0 | *BIRC5* |
| GI_42734437-S | 222 | 102 | 3.87E-05 | 2.2 | *BM-009* |
| GI_39725678-S | 140 | 30 | 9.90E-04 | 4.7 | *BM039* |
| GI_19923079-I | 66 | 414 | 3.59E-04 | 0.2 | *BNIPL* |
| GI_19923712-A | 6 | 27 | 9.80E-04 | 0.2 | *BNIPL* |
| GI_8923147-S | 26 | 93 | 3.42E-04 | 0.3 | *BSPRY* |
| GI_4502472-S | 405 | 1136 | 2.97E-04 | 0.4 | *BTG1* |
| GI_28872718-S | 60 | 193 | 2.91E-03 | 0.3 | *BTG2* |
| GI_4757877-S | 88 | 26 | 4.82E-03 | 3.3 | *BUB1* |
| GI_7661743-S | 274 | 154 | 4.44E-04 | 1.8 | *BZW2* |
| GI_34147683-S | 232 | 72 | 1.60E-03 | 3.2 | *C10orf3* |
| GI_27734692-S | 107 | 32 | 1.32E-03 | 3.3 | *C14orf80* |
| GI_42516575-S | 765 | 455 | 3.91E-04 | 1.7 | *C14orf87* |
| GI_7019336-S | 8 | 48 | 7.43E-05 | 0.2 | *C16orf5* |
| GI_42655770-S | 10 | 45 | 3.02E-05 | 0.2 | *C1orf34* |
| GI_14249635-S | 30 | 2 | 5.22E-04 | 13.6 | *C20orf100* |
| GI_18201877-S | 37 | 109 | 1.75E-07 | 0.3 | *C20orf108* |
| GI_24308304-S | 90 | 18 | 9.15E-04 | 5.0 | *C20orf129* |
| GI_31542256-S | 60 | 20 | 2.52E-03 | 3.0 | *C20orf172* |
| GI_40353206-S | 280 | 121 | 9.40E-04 | 2.3 | *C20orf20* |
| GI_7705768-S | 81 | 50 | 5.57E-04 | 1.6 | *C20orf9* |
| GI_9506436-S | 54 | 17 | 2.50E-04 | 3.1 | *C21orf45* |
| GI_31581597-S | 12 | 0 | 8.00E-05 | 19.2 | *C6orf150* |
| GI_22325369-A | 55 | 13 | 1.83E-03 | 4.2 | *C9orf23* |
| GI_9951924-S | 150 | 737 | 2.55E-03 | 0.2 | *CA12* |
| GI_23943849-I | 5 | 54 | 7.13E-06 | 0.1 | *CAMK1D* |
| GI_14161691-S | 64 | 290 | 2.26E-03 | 0.2 | *CAPNS2* |
| GI_20544150-I | 197 | 73 | 7.07E-04 | 2.7 | *CBX3* |
| GI_20544152-A | 366 | 161 | 9.74E-04 | 2.3 | *CBX3* |
| GI_16950653-S | 110 | 29 | 4.00E-03 | 3.8 | *CCNA2* |
| GI_34304372-S | 513 | 170 | 1.45E-03 | 3.0 | *CCNB1* |
| GI_38176157-S | 194 | 106 | 6.37E-04 | 1.8 | *CCNK* |
| GI_16306490-I | 105 | 34 | 4.48E-03 | 3.1 | *CDC2* |
| GI_27886643-A | 272 | 84 | 2.60E-03 | 3.2 | *CDC2* |
| GI_4557436-S | 1628 | 437 | 1.57E-04 | 3.7 | *CDC20* |
| GI_12408659-I | 18 | 3 | 6.30E-04 | 5.4 | *CDC25C* |
| GI_38683841-S | 148 | 66 | 5.93E-04 | 2.2 | *CDC26* |
| GI_34147481-S | 44 | 8 | 4.92E-03 | 5.5 | *CDCA5* |
| GI_8922437-S | 213 | 64 | 1.79E-03 | 3.3 | *CDCA8* |
| GI_16936531-I | 173 | 74 | 9.72E-04 | 2.4 | *CDK4* |
| GI_17981703-S | 364 | 93 | 3.02E-04 | 3.9 | *CDKN3* |
| GI_22035623-S | 156 | 385 | 4.43E-06 | 0.4 | *CDS1* |
| GI_7705317-S | 5 | 18 | 7.20E-05 | 0.3 | *GULP1* |
| GI_13325063-S | 63 | 198 | 2.30E-03 | 0.3 | *CELSR2* |
| GI_8923762-S | 13 | 92 | 3.18E-04 | 0.1 | *CENTA2* |
| GI_34147675-S | 110 | 40 | 7.42E-04 | 2.8 | *CGI-12* |
| GI_27477096-I | 45 | 92 | 1.40E-06 | 0.5 | *CGI-85* |
| GI_10092611-A | 64 | 18 | 3.13E-03 | 3.5 | *CKLF* |
| GI_4502856-S | 1107 | 477 | 1.79E-04 | 2.3 | *CKS1B* |
| GI_31563536-S | 71 | 123 | 1.74E-04 | 0.6 | *CLASP*1 |
| GI_21536297-S | 222 | 777 | 2.38E-03 | 0.3 | *CLDN1* |
| GI_7661555-S | 80 | 44 | 5.38E-05 | 1.8 | *TRUB2* |
| GI_31581523-S | 5 | 41 | 1.93E-04 | 0.1 | *COBL* |
| GI_18641355-A | 987 | 1901 | 4.69E-04 | 0.5 | *COL17A1* |
| GI_16554581-S | 18 | 66 | 4.11E-04 | 0.3 | *COL5A3* |
| GI_32171224-S | 70 | 25 | 1.45E-03 | 2.8 | *COQ3* |
| GI_10047105-S | 37 | 219 | 1.56E-05 | 0.2 | *CPA4* |
| GI_16418454-S | 9 | 43 | 2.34E-03 | 0.2 | *RBP7* |
| GI_4503056-S | 60 | 433 | 9.76E-04 | 0.1 | *CRYAB* |
| GI_5031774-S | 22 | 72 | 1.09E-03 | 0.3 | *CTDSPL* |
| GI_9910389-S | 89 | 176 | 7.09E-04 | 0.5 | *CTNNBIP1* |
| GI_20149514-S | 153 | 462 | 6.15E-04 | 0.3 | *CXADR* |
| GI_23199988-S | 166 | 1144 | 3.54E-03 | 0.1 | *CXCL14* |
| GI_4503182-S | 72 | 207 | 1.14E-03 | 0.3 | *CYB5* |
| GI_7706442-S | 50 | 430 | 2.85E-05 | 0.1 | *CYB5R2* |
| GI_21359866-S | 754 | 346 | 3.07E-05 | 2.2 | *CYC1* |
| GI_31542479-S | 10 | 3 | 8.43E-04 | 3.7 | *D4ST1* |
| GI_33667026-S | 2249 | 1099 | 7.06E-05 | 2.0 | *DC50* |
| GI_41327774-S | 365 | 214 | 2.02E-04 | 1.7 | *DDX47* |
| GI_13124884-S | 68 | 786 | 1.92E-04 | 0.1 | *DEFB1* |
| GI_21614500-A | 395 | 887 | 1.10E-04 | 0.4 | *DEGS* |
| GI_44662829-A | 32 | 11 | 8.26E-04 | 2.8 | *DERP6* |
| GI_13375617-S | 326 | 680 | 4.53E-04 | 0.5 | *DHCR24* |
| GI_20336301-S | 77 | 31 | 2.51E-03 | 2.5 | *DHX33* |
| GI_37542859-S | 103 | 176 | 1.75E-04 | 0.6 | *DKFZp313M0720* |
| GI_13899331-S | 10 | 50 | 6.69E-04 | 0.2 | *DKFZP434B044* |
| GI_14149980-S | 19 | 56 | 6.56E-04 | 0.3 | *DKFZp434K2435* |
| GI_37552665-S | 97 | 202 | 8.40E-04 | 0.5 | *DKFZp761P0423* |
| GI_21361644-S | 116 | 36 | 9.38E-04 | 3.2 | *DLG7* |
| GI_31342420-S | 31 | 8 | 7.66E-04 | 3.8 | *DLX1* |
| GI_38505265-S | 77 | 40 | 6.42E-04 | 1.9 | *DNTTIP1* |
| GI_7657036-A | 15 | 58 | 5.67E-04 | 0.3 | *DOC1* |
| GI_20070301-S | 27 | 69 | 4.49E-03 | 0.4 | *DOK4* |
| GI_22035582-I | 11 | 1 | 9.92E-04 | 11.2 | *DONSON* |
| GI_39540511-S | 21 | 8 | 2.29E-05 | 2.8 | *DNAJC14* |
| GI_40806177-A | 143 | 496 | 1.49E-03 | 0.3 | *DSC2* |
| GI_4503400-S | 17 | 313 | 4.11E-03 | 0.1 | *DSG1* |
| GI_4503404-S | 199 | 719 | 2.69E-03 | 0.3 | *DSG3* |
| GI_7657045-S | 338 | 78 | 3.68E-03 | 4.3 | *UBE2S* |
| GI_4503554-S | 79 | 31 | 1.94E-04 | 2.6 | *ELF4* |
| GI_21362099-S | 53 | 227 | 1.58E-04 | 0.2 | *ELOVL4* |
| GI_32490571-S | 3 | 76 | 3.58E-03 | 0.0 | *EPB41L3* |
| GI_21264609-A | 19 | 83 | 3.30E-03 | 0.2 | *EPS8L1* |
| GI_21264615-S | 134 | 342 | 1.85E-03 | 0.4 | *EPS8L2* |
| GI_4758311-S | 34 | 103 | 4.75E-04 | 0.3 | *ETFDH* |
| GI_39995068-A | 64 | 16 | 3.04E-03 | 4.0 | *EXO1* |
| GI_12669906-S | 182 | 487 | 4.52E-04 | 0.4 | *FACL2* |
| GI_4759335-S | 50 | 19 | 3.15E-03 | 2.6 | *FANCG* |
| GI_21536438-A | 135 | 240 | 1.07E-04 | 0.6 | *FBXL5* |
| GI_15812190-S | 79 | 17 | 3.65E-03 | 4.7 | *FBXO5* |
| GI_16117778-A | 66 | 156 | 3.32E-04 | 0.4 | *FBXW7* |
| GI_36030993-S | 51 | 80 | 2.39E-04 | 0.6 | *FEM1C* |
| GI_37546229-S | 31 | 119 | 3.65E-04 | 0.3 | *FLJ10097* |
| GI_8922242-S | 110 | 972 | 1.64E-04 | 0.1 | *FLJ10134* |
| GI_9506604-S | 61 | 19 | 2.74E-03 | 3.2 | *FLJ10156* |
| GI_8922460-S | 42 | 8 | 2.85E-03 | 5.2 | *C9orf87* |
| GI_8922580-S | 2 | 15 | 2.97E-04 | 0.1 | *FLJ10665* |
| GI_8922600-S | 248 | 476 | 6.92E-05 | 0.5 | *ARL10C* |
| GI_31542666-S | 33 | 118 | 1.21E-04 | 0.3 | *FLJ11036* |
| GI_8922930-S | 36 | 14 | 2.43E-04 | 2.6 | *FLJ11193* |
| GI_31377840-S | 76 | 194 | 5.13E-04 | 0.4 | *FLJ11280* |
| GI_13375741-S | 119 | 50 | 5.10E-04 | 2.4 | *FLJ11712* |
| GI_40255030-S | 38 | 8 | 7.94E-04 | 4.9 | *FLJ12150* |
| GI_13375990-S | 52 | 193 | 1.15E-03 | 0.3 | *FLJ13841* |
| GI_34915997-S | 11 | 35 | 2.73E-05 | 0.3 | *FLJ20209* |
| GI_40254903-S | 35 | 122 | 9.88E-06 | 0.3 | *FLJ20321* |
| GI_21361603-S | 19 | 4 | 9.05E-04 | 4.3 | *SH3TC1* |
| GI_31982880-S | 20 | 55 | 3.21E-03 | 0.4 | *FLJ21069* |
| GI_13376643-S | 78 | 657 | 3.27E-05 | 0.1 | *FLJ21511* |
| GI_13375808-S | 73 | 46 | 9.73E-04 | 1.6 | *FLJ21908* |
| GI_13376163-S | 12 | 117 | 6.45E-05 | 0.1 | *ABHD9* |
| GI_34147690-S | 222 | 47 | 2.69E-03 | 4.7 | *FLJ22582* |
| GI_34303916-S | 30 | 3 | 8.16E-04 | 8.7 | *FLJ23322* |
| GI_22749326-S | 108 | 214 | 1.64E-04 | 0.5 | *UBE2E2* |
| GI_32698975-S | 10 | 82 | 8.40E-04 | 0.1 | *FLJ30469* |
| GI_21389358-S | 84 | 15 | 2.13E-03 | 5.6 | *FLJ30525* |
| GI_34594658-S | 44 | 16 | 2.82E-03 | 2.8 | *FLJ39616* |
| GI_32526889-S | 46 | 15 | 4.39E-03 | 3.0 | *FLJ40629* |
| GI_4503746-S | 199 | 508 | 2.11E-03 | 0.4 | *FLNB* |
| GI_38202220-A | 99 | 238 | 2.68E-04 | 0.4 | *FLRT3* |
| GI_38455415-S | 8 | 2 | 2.12E-04 | 3.8 | *FLT3LG* |
| GI_4503770-S | 211 | 357 | 8.40E-04 | 0.6 | *FNTA* |
| GI_11968026-S | 97 | 196 | 5.01E-04 | 0.5 | *FTS* |
| GI_40068511-S | 100 | 24 | 3.89E-05 | 4.2 | *FUCA2* |
| GI_13470091-S | 19 | 55 | 5.38E-04 | 0.3 | *FYCO1* |
| GI_18105041-A | 14 | 76 | 1.50E-03 | 0.2 | *GAB2* |
| GI_4503928-S | 35 | 162 | 2.28E-03 | 0.2 | *GATA3* |
| GI_22035688-S | 21 | 0 | 2.75E-05 | 15.9 | *GCAT* |
| GI_7705820-S | 93 | 161 | 2.29E-05 | 0.6 | *GOLGA7* |
| GI_21614532-A | 20 | 3 | 4.06E-04 | 7.4 | *PRSS21* |
| GI_34222184-S | 41 | 16 | 1.48E-03 | 2.5 | *LOC133957* |
| GI_42476192-S | 472 | 757 | 3.24E-04 | 0.6 | *MGC8721* |
| GI_4758199-S | 30 | 132 | 7.87E-06 | 0.2 | *DSP* |
| GI_4755136-S | 162 | 411 | 6.67E-04 | 0.4 | *GJA1* |
| GI_45359854-S | 77 | 164 | 3.58E-04 | 0.5 | *GOLGA4* |
| GI_41584199-S | 254 | 620 | 1.99E-04 | 0.4 | *GPR56* |
| GI_19263339-S | 102 | 289 | 1.06E-03 | 0.4 | *GPT2* |
| GI_13994373-S | 11 | 2 | 6.62E-04 | 5.8 | *GSG2* |
| GI_38044287-A | 151 | 532 | 2.39E-05 | 0.3 | *GSN* |
| GI_20357598-I | 46 | 14 | 2.92E-03 | 3.3 | *H2AV* |
| GI_41406065-I | 662 | 278 | 4.59E-04 | 2.4 | *H2AV* |
| GI_29728513-S | 101 | 317 | 5.20E-04 | 0.3 | *HES2* |
| GI_41393565-S | 28 | 10 | 3.50E-04 | 2.8 | *HIBADH* |
| GI_5031748-S | 1410 | 525 | 6.11E-05 | 2.7 | *HMGN2* |
| hmm7207-S | 81 | 492 | 6.65E-05 | 0.2 | *hmm7207* |
| GI_14110430-A | 665 | 406 | 4.20E-04 | 1.6 | *HNRPC* |
| GI_4758547-S | 24 | 68 | 2.74E-03 | 0.3 | *HOMER2* |
| GI_25121962-S | 63 | 0 | 1.23E-05 | 45.6 | *HOXB7* |
| GI_4758559-S | 93 | 37 | 7.92E-04 | 2.5 | *HPRP8BP* |
| Hs.118342-S | 6 | 26 | 1.85E-04 | 0.2 | *Hs.118342* |
| Hs.425023-S | 12 | 44 | 7.10E-04 | 0.3 | *Hs.425023* |
| GI_40538783-S | 58 | 154 | 4.58E-04 | 0.4 | *IBRDC2* |
| GI_38683856-S | 8 | 240 | 8.34E-04 | 0.0 | *ICEBERG* |
| GI_19923138-S | 6 | 25 | 8.20E-04 | 0.2 | *ID4* |
| GI_5360207-I | 18 | 53 | 4.75E-03 | 0.3 | *IDS* |
| GI_40354208-S | 2 | 11 | 6.29E-04 | 0.2 | *IDUA* |
| GI_21361309-S | 95 | 29 | 6.58E-04 | 3.3 | *IFI44* |
| GI_24430200-A | 19 | 74 | 1.98E-05 | 0.3 | *IL17RC* |
| GI_27894309-A | 23 | 170 | 2.74E-04 | 0.1 | *IL1F5* |
| GI_40254822-S | 1 | 34 | 6.13E-05 | 0.0 | *INPP5D* |
| GI_38327532-S | 49 | 81 | 6.60E-04 | 0.6 | *INSIG2* |
| GI_33589836-S | 5 | 154 | 1.14E-03 | 0.0 | *ITM2A* |
| GI_44890058-S | 56 | 331 | 6.02E-04 | 0.2 | *IVL* |
| GI_24475846-S | 76 | 177 | 1.21E-05 | 0.4 | *IVNS1ABP* |
| GI_4557678-S | 46 | 132 | 4.96E-03 | 0.3 | *JAG1* |
| GI_20357521-S | 83 | 163 | 2.43E-05 | 0.5 | *JMJD1* |
| GI_4758623-S | 44 | 101 | 1.21E-05 | 0.4 | *KCNK6* |
| GI_29746781-S | 52 | 96 | 4.71E-04 | 0.5 | *KIAA0350* |
| GI_41281419-S | 19 | 49 | 2.84E-03 | 0.4 | *KIAA0377* |
| GI_42655845-S | 85 | 168 | 5.02E-04 | 0.5 | *KIAA0450* |
| GI_29745993-S | 99 | 283 | 2.30E-04 | 0.4 | *KIAA0830* |
| GI_37552339-S | 105 | 189 | 6.69E-06 | 0.6 | *KIAA0876* |
| GI_35038563-S | 20 | 67 | 3.92E-04 | 0.3 | *KIAA0922* |
| GI_37564256-S | 117 | 286 | 2.66E-04 | 0.4 | *KIAA0930* |
| GI_21361584-S | 176 | 486 | 7.73E-04 | 0.4 | *KIAA0992* |
| GI_32698731-S | 64 | 10 | 1.89E-03 | 6.5 | *KIAA1363* |
| GI_27479430-S | 458 | 135 | 9.90E-05 | 3.4 | *KIAA1393* |
| GI_41147326-S | 21 | 59 | 1.56E-03 | 0.4 | *KIAA1411* |
| GI_37221178-S | 25 | 8 | 2.51E-04 | 3.2 | *ANKRD25* |
| GI_13699823-S | 65 | 13 | 4.46E-03 | 4.8 | *KIF11* |
| GI_13699832-S | 207 | 54 | 4.86E-04 | 3.8 | *KIF2C* |
| GI_7305204-S | 56 | 14 | 1.19E-03 | 4.1 | *KIF4A* |
| GI_22208983-A | 53 | 182 | 3.79E-03 | 0.3 | *KLK10* |
| GI_21618355-A | 228 | 1125 | 3.25E-05 | 0.2 | *KLK11* |
| GI_22208993-S | 330 | 1642 | 1.18E-04 | 0.2 | *KLK5* |
| GI_21327704-A | 284 | 1420 | 2.92E-05 | 0.2 | *KLK7* |
| GI_15431309-S | 2850 | 5659 | 5.35E-04 | 0.5 | *KRT14* |
| GI_40354193-A | 402 | 98 | 9.25E-04 | 4.1 | *KRT18* |
| GI_27894340-A | 9 | 125 | 1.92E-03 | 0.1 | *KRT23* |
| GI_17505187-S | 3299 | 7808 | 5.46E-04 | 0.4 | *KRT6B* |
| GI_30409981-S | 306 | 149 | 7.89E-04 | 2.1 | *LAPTM4B* |
| GI_7705579-A | 180 | 398 | 1.73E-04 | 0.5 | *LCMT1* |
| GI_7662509-S | 42 | 90 | 3.56E-05 | 0.5 | *LEPROTL1* |
| GI_4504984-S | 599 | 4419 | 1.69E-04 | 0.1 | *LGALS7* |
| GI_5174496-S | 56 | 163 | 2.67E-03 | 0.3 | *LIPG* |
| GI_22050438-S | 15 | 44 | 2.16E-03 | 0.3 | *LOC116412* |
| GI_17158004-S | 13 | 429 | 3.23E-03 | 0.0 | *LOC118430* |
| GI_37550830-S | 96 | 287 | 1.23E-03 | 0.3 | *LOC119548* |
| GI_41152082-S | 114 | 56 | 5.04E-04 | 2.0 | *TIM14* |
| GI_37551965-S | 8 | 181 | 3.63E-04 | 0.0 | *LOC147920* |
| GI_37563604-S | 133 | 68 | 5.00E-04 | 2.0 | *LOC150223* |
| GI_37549413-S | 74 | 298 | 1.95E-03 | 0.2 | *LOC150739* |
| GI_18552555-S | 4 | 59 | 2.17E-04 | 0.1 | *LOC151283* |
| GI_24308449-S | 67 | 26 | 1.98E-04 | 2.6 | *LOC152217* |
| GI_40255093-S | 69 | 26 | 2.62E-03 | 2.6 | *LOC159090* |
| GI_37551029-S | 9 | 70 | 1.32E-03 | 0.1 | *LOC221061* |
| GI_37542191-S | 9 | 3 | 9.40E-04 | 2.7 | *LOC283871* |
| GI_27498427-S | 17 | 56 | 4.01E-03 | 0.3 | *LOC285812* |
| GI_39930540-S | 160 | 59 | 2.98E-03 | 2.7 | *LOC286257* |
| GI_38348349-S | 48 | 5 | 1.02E-03 | 9.8 | *LOC374654* |
| GI_37549959-S | 409 | 195 | 5.08E-04 | 2.1 | *LOC375459* |
| GI_37540494-S | 153 | 75 | 4.97E-05 | 2.0 | *LOC375757* |
| GI_42659556-S | 21 | 53 | 1.43E-03 | 0.4 | *LOC387648* |
| GI_41204905-S | 15 | 75 | 3.97E-05 | 0.2 | *LOC388121* |
| GI_42661631-S | 15 | 5 | 7.89E-04 | 2.7 | *LOC388540* |
| GI_42656857-S | 818 | 455 | 3.54E-04 | 1.8 | *LOC389168* |
| GI_41146994-S | 49 | 132 | 4.41E-03 | 0.4 | *LOC389337* |
| GI_42658766-S | 20 | 6 | 8.73E-04 | 3.4 | *LOC389641* |
| GI_42656706-S | 7 | 29 | 9.65E-04 | 0.2 | *LOC401059* |
| GI_32307179-S | 1546 | 1018 | 9.78E-04 | 1.5 | *CHCHD2* |
| GI_13124772-S | 54 | 14 | 3.82E-03 | 3.8 | *LOC51236* |
| GI_31543081-S | 36 | 86 | 5.92E-05 | 0.4 | *LOC51257* |
| GI_7706556-A | 327 | 172 | 3.23E-05 | 1.9 | *C9orf78* |
| GI_8923856-S | 135 | 213 | 1.75E-04 | 0.6 | *LOC55831* |
| GI_20149710-S | 76 | 23 | 1.01E-05 | 3.3 | *LOC93349* |
| GI_38195079-I | 4 | 109 | 6.72E-04 | 0.0 | *LOH11CR2A* |
| GI_38195081-A | 6 | 28 | 7.23E-05 | 0.2 | *LOH11CR2A* |
| GI_38195081-I | 11 | 65 | 8.65E-07 | 0.2 | *LOH11CR2A* |
| GI_6912463-S | 20 | 108 | 6.82E-04 | 0.2 | *LPHN2* |
| GI_8923223-A | 59 | 122 | 4.67E-04 | 0.5 | *LRRFIP2* |
| GI_8923223-I | 70 | 167 | 1.22E-05 | 0.4 | *LRRFIP2* |
| GI_6912487-S | 457 | 204 | 1.03E-04 | 2.2 | *LSM5* |
| GI_6466452-S | 122 | 34 | 2.65E-03 | 3.6 | *MAD2L1* |
| GI_6006019-S | 224 | 66 | 1.37E-03 | 3.4 | *MAD2L2* |
| GI_5453735-S | 31 | 115 | 1.94E-03 | 0.3 | *MAF* |
| GI_34335240-S | 62 | 21 | 4.61E-03 | 3.0 | *MAGEF1* |
| GI_6006021-S | 217 | 86 | 4.51E-03 | 2.5 | *MAGOH* |
| GI_31563517-A | 50 | 132 | 3.24E-03 | 0.4 | *MAP1LC3A* |
| GI_14195617-A | 2 | 39 | 6.08E-04 | 0.1 | *MAP2* |
| GI_34335241-S | 166 | 346 | 1.60E-04 | 0.5 | *MAPKAPK3* |
| GI_3356546-S | 406 | 125 | 1.28E-03 | 3.2 | *MCM2* |
| GI_33469916-A | 168 | 62 | 1.99E-03 | 2.7 | *MCM4* |
| GI_27544938-S | 143 | 290 | 2.06E-04 | 0.5 | *C3orf10* |
| GI_41281490-S | 127 | 38 | 4.05E-04 | 3.3 | *MELK* |
| GI_5174556-S | 243 | 934 | 2.96E-04 | 0.3 | *MFGE8* |
| GI_28376644-S | 36 | 102 | 1.42E-03 | 0.4 | *MGC10500* |
| GI_14150059-S | 137 | 58 | 8.41E-05 | 2.4 | *MGC10911* |
| GI_38488726-S | 62 | 25 | 2.58E-03 | 2.5 | *MGC12197* |
| GI_45505158-S | 156 | 966 | 2.74E-03 | 0.2 | *MGC21394* |
| GI_21717806-S | 49 | 19 | 1.80E-03 | 2.5 | *MGC21654* |
| GI_44921601-S | 38 | 95 | 4.36E-05 | 0.4 | *ZNF524* |
| GI_13128989-S | 47 | 15 | 1.54E-04 | 3.1 | *MGC2603* |
| GI_34222172-S | 272 | 38 | 4.51E-05 | 7.2 | *MGC33630* |
| GI_22748880-S | 44 | 13 | 4.39E-03 | 3.3 | *MGC40214* |
| GI_42734435-S | 412 | 186 | 2.08E-04 | 2.2 | *EFHD2* |
| GI_34147362-S | 102 | 342 | 4.93E-03 | 0.3 | *MGC4504* |
| GI_21450827-S | 44 | 11 | 6.54E-04 | 3.9 | *MGC7036* |
| GI_42661554-S | 4 | 87 | 4.59E-03 | 0.0 | *MGC9913* |
| GI_4826835-S | 20 | 306 | 2.26E-03 | 0.1 | *MMP9* |
| GI_5174484-S | 4 | 50 | 1.21E-05 | 0.1 | *MRC2* |
| GI_21265095-S | 168 | 87 | 4.76E-04 | 1.9 | *MRPL50* |
| GI_22035596-S | 95 | 47 | 5.07E-04 | 2.0 | *MRPL9* |
| GI_16554613-S | 377 | 165 | 1.96E-04 | 2.3 | *MRPS17* |
| GI_13027603-S | 170 | 96 | 2.68E-04 | 1.8 | *MRPS34* |
| GI_40317613-A | 147 | 89 | 7.94E-04 | 1.7 | *MRRF* |
| GI_5453745-S | 53 | 21 | 2.32E-03 | 2.6 | *MTHFS* |
| GI_4505278-A | 126 | 52 | 5.61E-04 | 2.4 | *MTRR* |
| GI_42658866-S | 11 | 41 | 1.09E-04 | 0.3 | *MTSG1* |
| GI_10947033-S | 75 | 228 | 3.86E-04 | 0.3 | *MXD4* |
| GI_37202122-S | 33 | 13 | 7.54E-04 | 2.6 | *NARG2* |
| GI_13430863-A | 8 | 53 | 2.29E-04 | 0.1 | *NDRG4* |
| GI_33519467-S | 386 | 202 | 2.05E-05 | 1.9 | *NDUFB5* |
| GI_6274549-S | 972 | 544 | 1.29E-04 | 1.8 | *NDUFB9* |
| GI_4758787-S | 223 | 132 | 6.53E-04 | 1.7 | *NDUFS3* |
| GI_5453757-S | 34 | 167 | 1.96E-03 | 0.2 | *NEBL* |
| GI_4505372-S | 10 | 2 | 7.32E-04 | 5.5 | *NEK2* |
| GI_20127615-S | 36 | 88 | 9.64E-04 | 0.4 | *NICAL* |
| GI_9506922-S | 160 | 972 | 1.72E-03 | 0.2 | *NICE-1* |
| GI_6005787-S | 26 | 102 | 1.58E-04 | 0.3 | *NISCH* |
| GI_25777609-A | 61 | 188 | 3.78E-03 | 0.3 | *NOD9* |
| GI_27894367-S | 56 | 210 | 2.61E-04 | 0.3 | *NOTCH1* |
| GI_38455392-S | 32 | 11 | 4.90E-04 | 2.9 | *NTHL1* |
| GI_12232386-S | 948 | 541 | 1.42E-05 | 1.8 | *NUCKS* |
| GI_37594460-A | 14 | 4 | 6.80E-04 | 3.5 | *NUDT6* |
| GI_24430147-A | 121 | 32 | 1.42E-03 | 3.8 | *NUP155* |
| GI_34222120-S | 363 | 167 | 1.68E-04 | 2.2 | *Nup37* |
| GI_7705950-I | 180 | 65 | 2.57E-03 | 2.8 | *NUSAP1* |
| GI_5031984-S | 205 | 126 | 8.90E-04 | 1.6 | *NUTF2* |
| GI_24430182-A | 19 | 1 | 3.27E-04 | 14.5 | *ODF2* |
| GI_7662457-S | 15 | 44 | 1.67E-03 | 0.3 | *OIP106* |
| GI_24307928-S | 82 | 20 | 2.90E-03 | 4.1 | *OIP5* |
| GI_34147604-S | 233 | 562 | 3.49E-04 | 0.4 | *OPTN* |
| GI_32483368-A | 59 | 21 | 3.09E-03 | 2.8 | *ORC3L* |
| GI_22035611-A | 24 | 64 | 1.36E-03 | 0.4 | *OSBPL6* |
| GI_33695118-S | 121 | 187 | 8.10E-05 | 0.6 | *PAPSS1* |
| GI_14670372-A | 17 | 48 | 1.62E-04 | 0.4 | *PCBP4* |
| GI_34304340-A | 77 | 217 | 3.18E-03 | 0.4 | *PDCD4* |
| GI_5453915-S | 166 | 285 | 1.80E-04 | 0.6 | *PGRMC2* |
| GI_23308576-S | 276 | 793 | 2.43E-03 | 0.3 | *PHGDH* |
| GI_19923778-S | 84 | 16 | 1.99E-03 | 5.4 | *PIR51* |
| GI_6005829-S | 321 | 599 | 7.28E-04 | 0.5 | *PKP3* |
| GI_5453909-S | 27 | 101 | 4.11E-04 | 0.3 | *PLCD1* |
| GI_4505872-S | 14 | 47 | 1.10E-03 | 0.3 | *PLD1* |
| GI_34147632-S | 58 | 16 | 4.13E-03 | 3.7 | *PLK1* |
| GI_40255004-S | 13 | 122 | 1.61E-05 | 0.1 | *PLXDC2* |
| GI_32189368-S | 45 | 11 | 1.71E-03 | 4.1 | *POLE2* |
| GI_4505946-S | 397 | 255 | 4.95E-04 | 1.6 | *POLR2G* |
| GI_14589955-S | 365 | 179 | 2.84E-04 | 2.0 | *POLR2K* |
| GI_14589957-S | 57 | 16 | 4.84E-04 | 3.6 | *POLR3K* |
| GI_13775195-S | 77 | 183 | 8.20E-04 | 0.4 | *PP1057* |
| GI_29570797-S | 70 | 24 | 3.23E-03 | 2.9 | *PPAT* |
| GI_27499434-S | 26 | 171 | 5.53E-05 | 0.2 | *PPFIBP2* |
| GI_45439315-I | 5 | 21 | 5.95E-04 | 0.2 | *PPlE* |
| GI_45439341-A | 67 | 22 | 6.14E-04 | 3.0 | *PPlL5* |
| GI_19311005-S | 81 | 213 | 2.33E-04 | 0.4 | *PPP1R14C* |
| GI_42476161-S | 41 | 157 | 8.65E-05 | 0.3 | *PPP1R3C* |
| GI_32967585-A | 33 | 72 | 3.55E-05 | 0.5 | *PPP2R3A* |
| GI_40807441-S | 269 | 107 | 4.30E-03 | 2.5 | *PRC1* |
| GI_24497617-S | 100 | 25 | 7.17E-04 | 3.9 | *PRO2000* |
| GI_21536454-I | 7 | 23 | 4.04E-05 | 0.3 | *PSEN1* |
| GI_23110945-A | 2556 | 1680 | 3.44E-05 | 1.5 | *PSMA7* |
| GI_34335278-I | 39 | 11 | 8.84E-05 | 3.5 | *PSMB8* |
| GI_30410791-S | 370 | 153 | 2.94E-04 | 2.4 | *PSME2* |
| GI_38505195-S | 297 | 72 | 4.95E-03 | 4.2 | *PTGES* |
| GI_18104977-S | 271 | 169 | 5.55E-04 | 1.6 | *PTPN1* |
| GI_18426910-S | 220 | 489 | 3.53E-04 | 0.4 | *PTPNS1* |
| GI_9257235-S | 13 | 48 | 2.16E-03 | 0.3 | *QPCT* |
| GI_34485712-S | 311 | 623 | 1.33E-05 | 0.5 | *RA811A* |
| GI_18640747-S | 57 | 135 | 2.67E-05 | 0.4 | *RAB24* |
| GI_31543538-S | 156 | 285 | 2.01E-06 | 0.5 | *RAB5A* |
| GI_21361396-S | 130 | 49 | 2.12E-03 | 2.7 | *RACGAP1* |
| GI_19924136-S | 42 | 9 | 4.95E-03 | 4.7 | *RAD54L* |
| GI_18702328-S | 5 | 43 | 5.70E-04 | 0.1 | *RAET1L* |
| GI_31542536-S | 194 | 50 | 3.74E-03 | 3.9 | *RAM2* |
| GI_6382077-S | 666 | 262 | 3.76E-03 | 2.5 | *RANBP1* |
| GI_4506424-S | 10 | 238 | 4.29E-03 | 0.0 | *RARRES1* |
| GI_5803140-S | 26 | 113 | 3.30E-05 | 0.2 | *RBPMS* |
| GI_31881686-A | 187 | 60 | 9.82E-04 | 3.1 | *RFC4* |
| GI_37550356-S | 33 | 79 | 4.48E-04 | 0.4 | *RGNEF* |
| GI_38327597-I | 93 | 200 | 1.51E-04 | 0.5 | *RGS12* |
| GI_37577171-A | 21 | 69 | 7.73E-04 | 0.3 | *RNASE4* |
| GI_38045930-S | 5 | 16 | 5.60E-04 | 0.3 | *RNF122* |
| GI_37588870-A | 2 | 38 | 2.30E-06 | 0.1 | *RNF128* |
| GI_7705605-S | 337 | 157 | 5.95E-04 | 2.1 | *RRP40* |
| GI_34147329-S | 92 | 26 | 4.73E-03 | 3.5 | *RRS1* |
| GI_5730022-S | 97 | 47 | 2.24E-04 | 2.1 | *RUVBL2* |
| GI_9845514-I | 26 | 157 | 1.34E-03 | 0.2 | *S100A4* |
| GI_9845515-A | 30 | 123 | 1.11E-03 | 0.2 | *S100A4* |
| GI_16306547-S | 549 | 1059 | 1.68E-04 | 0.5 | *SARS* |
| GI_33598918-A | 164 | 301 | 6.09E-04 | 0.5 | *SCAMP1* |
| GI_45006985-S | 190 | 122 | 6.26E-05 | 1.6 | *SCYE1* |
| GI_21735576-S | 4 | 28 | 3.49E-05 | 0.2 | *SDK2* |
| GI_31377801-S | 102 | 301 | 1.58E-03 | 0.3 | *SEMA3F* |
| GI_4505148-S | 145 | 737 | 2.88E-05 | 0.2 | *SERPINB7* |
| GI_7657436-S | 7 | 19 | 3.91E-05 | 0.3 | *SESN1* |
| GI_32454742-S | 38 | 108 | 2.34E-03 | 0.4 | *SESN2* |
| GI_4506890-S | 866 | 442 | 6.70E-05 | 2.0 | *SET* |
| GI_13899316-S | 15 | 41 | 4.09E-03 | 0.4 | *SH3BGRL2* |
| GI_34222154-S | 52 | 141 | 9.77E-06 | 0.4 | *SLC20A2* |
| GI_39777593-S | 101 | 21 | 5.56E-04 | 4.8 | *SLCO4A1* |
| GI_5730050-S | 188 | 486 | 3.14E-03 | 0.4 | *SLC2A1* |
| GI_5453590-S | 42 | 15 | 2.33E-03 | 2.8 | *SMC2L1* |
| GI_5730054-S | 93 | 283 | 4.03E-06 | 0.3 | *PLK2* |
| GI_38149917-I | 75 | 30 | 3.62E-03 | 2.5 | *SNRPB2* |
| GI_4507126-S | 100 | 44 | 2.51E-04 | 2.3 | *SNRPC* |
| GI_4507128-S | 627 | 297 | 3.95E-05 | 2.1 | *SNRPE* |
| GI_30179901-S | 45 | 126 | 3.02E-03 | 0.4 | *SOX4* |
| GI_37704387-S | 65 | 191 | 3.35E-04 | 0.3 | *SOX9* |
| GI_5803218-S | 35 | 554 | 3.99E-04 | 0.1 | *SPINK5* |
| GI_40787998-S | 25 | 82 | 2.80E-03 | 0.3 | *SSBP2* |
| GI_6552341-S | 747 | 489 | 6.79E-05 | 1.5 | *SSR2* |
| GI_5803180-S | 125 | 44 | 3.20E-03 | 2.9 | *STIP1* |
| GI_4759179-A | 43 | 66 | 7.29E-04 | 0.7 | *STK19* |
| GI_38327571-A | 225 | 53 | 1.28E-03 | 4.3 | *STK6* |
| GI_7305502-S | 162 | 72 | 5.46E-04 | 2.2 | *STOML2* |
| GI_38327656-I | 4 | 46 | 4.64E-07 | 0.1 | *SULF2* |
| GI_38327657-A | 364 | 1509 | 1.18E-05 | 0.2 | *SULF2* |
| GI_38202249-S | 34 | 96 | 1.54E-04 | 0.4 | *SUMF1* |
| GI_19924176-S | 227 | 101 | 4.50E-05 | 2.3 | *SUPT16H* |
| GI_18152766-S | 0 | 16 | 4.82E-04 | 0.0 | *SYTL4* |
| GI_20357590-S | 77 | 35 | 2.44E-04 | 2.2 | *TAF2* |
| GI_19743568-I | 71 | 137 | 5.80E-04 | 0.5 | *TANK* |
| GI_21071007-S | 46 | 813 | 4.43E-05 | 0.1 | *TCN1* |
| GI_23308578-S | 950 | 504 | 4.61E-04 | 1.9 | *TEBP* |
| GI_6912699-A | 28 | 9 | 2.91E-04 | 3.3 | *TFAM* |
| GI_4507506-S | 88 | 40 | 7.26E-04 | 2.2 | *TIMELESS* |
| GI_7706574-S | 121 | 341 | 6.02E-06 | 0.4 | *TM7SF3* |
| GI_20270211-S | 97 | 190 | 1.87E-04 | 0.5 | *TNKS1BP1* |
| GI_5174722-S | 188 | 54 | 3.80E-03 | 3.5 | *TOMM40* |
| GI_20127661-S | 33 | 98 | 1.33E-04 | 0.3 | *TP531NP1* |
| GI_40354199-S | 85 | 24 | 7.07E-04 | 3.6 | *TPX2* |
| GI_18087810-S | 128 | 40 | 5.18E-07 | 3.2 | *THRAP6* |
| GI_15011942-S | 27 | 162 | 1.68E-03 | 0.2 | *TRIM2* |
| GI_15208661-S | 20 | 68 | 1.24E-03 | 0.3 | *TRIM22* |
| GI_17402908-I | 87 | 219 | 2.08E-03 | 0.4 | *TRIM29* |
| GI_4507728-S | 173 | 763 | 8.03E-05 | 0.2 | *TUBB* |
| GI_4507728-S | 356 | 1359 | 1.71E-05 | 0.3 | *TUBB* |
| GI_9910595-S | 120 | 343 | 9.57E-05 | 0.3 | *TUFT1* |
| GI_32967290-A | 271 | 80 | 1.16E-03 | 3.4 | *UBE2C* |
| GI_16507203-S | 107 | 37 | 3.97E-03 | 2.9 | *UHRF1* |
| GI_38348365-S | 402 | 1890 | 2.34E-03 | 0.2 | *UNQ698* |
| GI_40254472-S | 20 | 74 | 1.28E-03 | 0.3 | *VLDLR* |
| GI_4507902-S | 40 | 12 | 3.83E-03 | 3.3 | *VRK1* |
| GI_34222152-S | 27 | 92 | 6.94E-05 | 0.3 | *VSNL1* |
| GI_23199997-A | 84 | 31 | 3.83E-03 | 2.7 | *WBSCR20A* |
| GI_5901891-S | 69 | 25 | 2.10E-03 | 2.8 | *WDHD1* |
| GI_20127459-S | 29 | 73 | 4.59E-04 | 0.4 | *XPC* |
| GI_33504488-S | 489 | 2890 | 7.89E-04 | 0.2 | *ZD52F10* |
| GI_7019582-S | 4 | 12 | 6.90E-04 | 0.3 | *ZNF215* |
| GI_21687251-S | 23 | 7 | 2.00E-04 | 3.1 | *ZNF342* |
| GI_14602428-A | 61 | 16 | 4.91E-03 | 3.7 | *ZWINT* |

**Table S5: Fold change comparison of 154 cSCC genes between in vitro cSCC, in vivo SCC and in vivo psoriatic mRNA expression analysis.**

| GENE | *In vitro* SCC FC | *In vivo* SCC FC | *In vivo* Psoriasis FC |
|---|---|---|---|
| *ABHD5* | -2.2 | -1.8 | 1.0 |
| *ACSL1* | -2.7 | -2.0 | -1.3 |
| *ALDH3A2* | -2.5 | -2.3 | -1.7 |
| *ANG* | -2.6 | -3.0 | -2.0 |
| *ANKRD25* | 3.2 | -1.7 | -1.7 |
| *ANLN* | 3.3 | 2.0 | 2.1 |
| *APOE* | -4.0 | -2.5 | -1.8 |
| *AQP9* | -51.6 | -2.2 | -2.3 |
| *ARHGDIB* | 2.6 | 1.4 | 1.5 |
| *ATAD2* | 3.9 | 1.9 | 1.6 |
| *AURKA* | 4.3 | 2.4 | 3.5 |
| *AURKB* | 3.1 | 1.9 | 2.7 |
| *BCL11A* | -3.1 | -1.4 | -1.0 |
| *BDKRB1* | 8.4 | 1.3 | -1.5 |
| *BEXL1* | -3.9 | -1.8 | -1.5 |
| *BIRC5* | 4.0 | 2.3 | 3.4 |
| *BUB1* | 3.3 | 2.7 | 1.0 |
| *C1orf59* | 5.6 | 2.1 | 1.6 |
| *C20orf20* | 2.3 | 1.3 | 1.2 |
| *C6orf150* | 19.2 | 3.1 | 1.1 |
| *PSMG3* | 2.4 | 1.4 | 1.2 |
| *CCNA2* | 3.8 | 2.8 | 3.5 |
| *CCNB1* | 3.0 | 4.2 | 5.3 |
| *CDC2* | 3.1 | 2.2 | 3.6 |
| *CDC20* | 3.7 | 3.5 | 4.0 |
| *CDC25C* | 5.4 | 1.5 | 1.0 |
| *CDCA5* | 5.5 | 3.0 | 2.5 |
| *CDCA8* | 3.3 | 5.8 | 1.9 |
| *CDKN3* | 3.9 | 2.6 | 4.4 |
| *CENPN* | 4.7 | 2.2 | 1.0 |
| *CENTA2* | -7.3 | 2.8 | 3.9 |
| *CEP55* | 3.2 | 3.9 | 3.5 |
| *CHAC1* | -3.3 | 4.5 | 6.4 |
| *CKAP2L* | 3.0 | 2.3 | 1.5 |
| *CKS1B* | 2.3 | 1.6 | 1.3 |
| *CLDN1* | -3.5 | -1.5 | -2.0 |
| *COBL* | -7.8 | -2.4 | -2.3 |
| *CRYAB* | -7.2 | -2.1 | -2.5 |
| *CTNNBIP1* | -2.0 | -1.8 | -2.0 |
| *CXCL14* | -6.9 | -1.6 | -1.4 |
| *CYB5A* | -2.9 | -2.1 | 1.0 |
| *DEGS1* | -2.2 | -1.7 | -1.3 |
| *DKFZp761 P0423* | -2.1 | 1.9 | 1.3 |
| *DLG7* | 3.2 | 3.6 | 5.3 |
| *DONSON* | 11.2 | 1.4 | 1.5 |
| *DSC2* | -3.5 | 12.4 | 6.6 |
| *DSG3* | -3.6 | 4.2 | 3.0 |
| *DSN1* | 3.0 | 1.6 | 1.3 |
| *EFHD2* | 2.2 | 1.4 | 1.8 |
| *ELF4* | 2.6 | 2.1 | 1.2 |
| *EP841L3* | -29.7 | 1.7 | -1.0 |
| *EXO1* | 4.0 | 1.8 | 1.5 |
| *FAM122B* | 2.6 | 1.2 | 1.5 |
| *FAM64A* | 3.2 | 1.5 | 1.4 |
| *FAM83D* | 5.0 | 2.2 | 3.0 |
| *FBXO5* | 4.7 | 2.2 | 1.7 |
| *FILIP1L* | -3.7 | -1.5 | -1.3 |
| *FLNB* | -2.5 | 1.8 | -1.1 |
| *FLRT3* | -2.4 | 2.2 | 1.1 |
| *FLT3LG* | 3.8 | 1.4 | 1.1 |
| *FUCA2* | 4.2 | 1.6 | 1.2 |
| *GATA3* | -4.6 | -2.4 | -2.2 |
| *GCAT* | 15.9 | 1.4 | 1.3 |
| *GSG2* | 5.8 | 1.3 | 1.2 |
| *GSN* | -3.5 | -1.6 | -1.6 |
| *GULP1* | -3.3 | -1.9 | -1.1 |
| *HES2* | -3.2 | 1.9 | 1.4 |
| *HIBADH* | 2.8 | -1.8 | -1.5 |
| *ID4* | -4.0 | -3.5 | -3.0 |
| *IFI44* | 3.3 | 2.8 | 4.9 |
| *IL1F5* | -7.5 | 2.4 | 7.5 |
| *INSIG2* | -1.7 | -1.3 | -1.4 |
| *ITM2A* | -28.0 | -2.2 | -2.2 |
| *IVL* | -5.9 | 4.2 | 3.7 |
| *KCNK6* | -2.3 | 1.5 | 2.4 |
| *KIF11* | 4.8 | 2.2 | 2.3 |
| *KI F2C* | 3.8 | 2.3 | 3.0 |
| *KI F4A* | 4.1 | 2.3 | 2.6 |
| *KLK10* | -3.4 | 3.4 | 6.1 |
| *KRT6B* | -2.4 | 3.5 | 8.4 |
| *LIPG* | -2.9 | 1.8 | 3.3 |
| *LOC152217* | 2.6 | 1.3 | 1.2 |
| *LOH11CR2A* | -24.9 | -1.5 | -1.2 |
| *MAD2L2* | 3.4 | 1.5 | 1.4 |
| *MAP1LC3A* | -2.6 | -1.4 | 1.0 |
| *MCM2* | 3.2 | 2.2 | 1.8 |
| *MCM4* | 2.7 | 2.1 | 1.9 |
| *MEL K* | 3.3 | 3.5 | 4.0 |
| *MMP9* | -15.0 | 5.2 | 4.6 |
| *NDRG4* | -6.7 | 2.9 | 2.2 |
| *NEBL* | -4.9 | -1.7 | -1.1 |
| *NEK2* | 5.5 | 2.5 | 2.0 |
| *NISCH* | -3.8 | -1.4 | -1.2 |
| *NUP155* | 3.8 | 1.5 | 1.3 |
| *NUP37* | 2.2 | 1.4 | 1.6 |
| *NUSAP1* | 2.8 | 2.7 | 2.6 |
| *NUTF2* | 1.6 | 1.5 | 1.1 |
| *OIP5* | 4.1 | 2.3 | 2.7 |
| *PALLD* | -2.8 | 2.0 | 1.0 |
| *PARP1* | 2.5 | 1.5 | 1.1 |
| *PDCD4* | -2.8 | -1.8 | -1.7 |
| *PGRMC2* | -1.7 | -1.6 | -1.6 |
| *PLCD1* | -3.7 | 1.4 | 1.7 |
| *PLCH2* | -2.0 | -1.5 | 1.0 |
| *PLK1* | 3.7 | 3.1 | -1.1 |
| *POLE2* | 4.1 | 1.9 | 2.5 |
| *PPIL5* | 3.0 | 2.3 | 2.4 |
| *PPP1R3C* | -3.9 | -2.0 | -1.7 |
| *PRC1* | 2.5 | 2.1 | 2.1 |
| *PRSS21* | 7.4 | 1.5 | 1.2 |
| *PSMB8* | 3.5 | 1.5 | 1.9 |
| *PSME2* | 2.1 | 1.6 | 2.5 |
| *RACGAP1* | 2.7 | 1.8 | 1.7 |
| *RAD51AP1* | 5.4 | 2.4 | 2.1 |
| *RAD54L* | 4.7 | 2.6 | 1.3 |
| *RANBP1* | 2.5 | 1.4 | 1.5 |
| *RARRES1* | -23.0 | -1.4 | 1.0 |
| *RBP7* | -4.8 | -2.5 | -1.4 |
| *RFC4* | 3.1 | 1.7 | 1.5 |
| *RNASE4* | -3.3 | -3.8 | -2.1 |
| *RSRC1* | 2.5 | 1.8 | 1.1 |
| *S100A4* | -5.9 | -1.7 | -1.2 |
| *SBEM* | -34.1 | -2.7 | -1.4 |
| *SDK2* | -6.7 | 2.4 | 1.5 |
| *SESN2* | -2.8 | 1.5 | 1.7 |
| *SH3BGRL2* | -2.8 | -1.7 | -1.9 |
| *SH3TC1* | 4.3 | 1.5 | 1.0 |
| *SIRPA* | -2.2 | 1.6 | 1.2 |
| *SLC2A1* | -2.6 | 2.1 | 1.8 |
| *SMC2* | 2.8 | 1.6 | 1.7 |
| *SNRPC* | 2.3 | 1.4 | 1.3 |
| *SOX4* | -2.8 | 1.7 | -1.2 |
| *SPINK6* | -6.2 | 12.3 | 1.3 |
| *SSBP2* | -3.3 | -1.6 | -1.3 |
| STIP1 | 2.9 | 1.4 | 1.6 |
| *SULF2* | -11.6 | 3.1 | -1.0 |
| *SUPT16H* | 2.3 | 1.5 | -1.0 |
| *TCN1* | -17.8 | 16.3 | 193.3 |
| *TIMELESS* | 2.2 | 2.1 | 1.6 |
| *TMEM117* | -2.9 | 1.9 | 2.0 |
| *TP531NP1* | -3.0 | 1.9 | -1.0 |
| *TPX2* | 3.6 | 2.8 | 3.1 |
| *TRIM22* | -3.4 | 2.1 | 3.3 |
| *TRUB2* | 1.8 | 1.5 | 1.5 |
| *UBE2C* | 3.4 | 2.3 | 2.6 |
| *UBE2E2* | -2.0 | 1.3 | 1.4 |
| *UBE2S* | 4.3 | 2.1 | 2.5 |
| *UHRF1* | 2.9 | 4.5 | 3.3 |
| *VSNL1* | -3.4 | 2.7 | 3.8 |
| *WDHD1* | 2.8 | 2.0 | 1.0 |
| *WDR66* | 7.2 | 3.8 | 6.6 |
| *WDR67* | 2.5 | 1.5 | 1.0 |
| *XPC* | -2.5 | -1.5 | -1.6 |
| *ZWINT* | 3.7 | 2.6 | 2.5 |

**Table S6: Genes specifically differentially regulated in cSCC and not Psoriasis.**

| GENE | *In vitro* SCC FC | *In vivo* SCC FC | *In vivo* Psoriasis FC |
|---|---|---|---|
| *ABHD5* | -2.2 | -1.8 | 1.0 |
| *BCL11A* | -3.1 | -1.4 | -1.0 |
| *BDKRB1* | 8.4 | 1.3 | -1.5 |
| *BUB1* | 3.3 | 2.7 | 1.0 |
| *C20orf20* | 2.3 | 1.3 | 1.2 |
| *C6orf150* | 19.2 | 3.1 | 1.1 |
| *PSMG3* | 2.4 | 1.4 | 1.2 |
| *CDC25C* | 5.4 | 1.5 | 1.0 |
| *CENPN* | 4.7 | 2.2 | 1.0 |
| *CYB5A* | -2.9 | -2.1 | 1.0 |
| *ELF4* | 2.6 | 2.1 | 1.2 |
| *EPB41L3* | -29.7 | 1.7 | -1.0 |
| *FLNB* | -2.5 | 1.8 | -1.1 |
| *FLRT3* | -2.4 | 2.2 | 1.1 |
| *FLT3LG* | 3.8 | 1.4 | 1.1 |
| *FUCA2* | 4.2 | 1.6 | 1.2 |
| *GSG2* | 5.8 | 1.3 | 1.2 |
| *GULP1* | -3.3 | -1.9 | -1.1 |
| *LOC152217* | 2.6 | 1.3 | 1.2 |
| *MAP1LC3A* | -2.6 | -1.4 | 1.0 |
| *NEBL* | -4.9 | -1.7 | -1.1 |
| *NUTF2* | 1.6 | 1.5 | 1.1 |
| *PALLD* | -2.8 | 2.0 | 1.0 |
| *PARP1* | 2.5 | 1.5 | 1.1 |
| *PLCH2* | -2.0 | -1.5 | 1.0 |
| *PLK1* | 3.7 | 3.1 | -1.1 |
| *PRSS21* | 7.4 | 1.5 | 1.2 |
| *RARRES1* | -23.0 | -1.4 | 1.0 |
| *RSRC1* | 2.5 | 1.8 | 1.1 |
| *SH3TC1* | 4.3 | 1.5 | 1.0 |
| *SIRPA* | -2.2 | 1.6 | 1.2 |
| *SOX4* | -2.8 | 1.7 | -1.2 |
| *SULF2* | -11.6 | 3.1 | -1.0 |
| *SUPT16H* | 2.3 | 1.5 | -1.0 |
| *TP531NP1* | -3.0 | 1.9 | -1.0 |
| *WDHD1* | 2.8 | 2.0 | 1.0 |
| *WDR67* | 2.5 | 1.5 | 1.0 |

### References

Bourdon, J. C., Fernandes, K., Mumay-Zmijewski, F., Liu, G., Diot, A., Xirodimas, D. P., Saville, M. K., and Lane, D. P. (2005). p53 isoforms can regulate p53 transcriptional activity. Genes Dev 19, 2122-2137.
Braakhuis, B. J., Brakenhoff, R. H., and Leemans, C. R. (2010). Gene expression profiling in head and neck squamous cell carcinoma. Curr Opin Otolaryngol Head Neck Surg 18, 67-71.
Carvalho, B., Postma, C., Mongera, S., Hopmans, E., Diskin, S., van de Wiel, M. A., van Criekinge, W., Thas, O., Matthai, A., Cuesta, M. A., et al. (2009). Multiple putative oncogenes at the chromosome 20q amplicon contribute to colorectal adenoma to carcinoma progression. Gut 58, 79-89.
Cheng, H., and Force, T. (2010). Molecular mechanisms of cardiovascular toxicity of targeted cancer therapeutics. Circ Res 106, 21-34.
Clark, L. J., Edington, K., Swan, I. R., McLay, K. A., Newlands, W. J., Wills, L. C., Young, H. A., Johnston, P. W., Mitchell, R., Robertson, G., and et al. (1993). The absence of Harvey ras mutations during development and progression of squamous cell carcinomas of the head and neck. Br J Cancer 68, 617-620.
Creighton, C., Kuick, R., Misek, D. E., Rickman, D. S., Brichory, F. M., Rouillard, J. M., Omenn, G. S., and Hanash, S. (2003). Profiling of pathway-specific changes in gene expression following growth of human cancer cell lines transplanted into mice. Genome Biol 4, R46.
Cunningham, J., Sales, M., Pearce, A., Howard, J., Stallings, R., Telford, N., Wilkie, R., Huntly, B., Thomas, A., O'Marcaigh, A., et al. (2002). Does isochromosome 7q mandate bone marrow transplant in children with Shwachman-Diamond syndrome? Br J Haematol 119, 1062-1069.
Dairkee, S. H., Ji, Y., Ben, Y., Moore, D. H., Meng, Z., and Jeffrey, S. S. (2004). A molecular 'signature' of primary breast cancer cultures; patterns resembling tumor tissue. BMC Genomics 5, 47.
Degenhardt, Y., and Lampkin, T. (2010). Targeting Polo-like Kinase in Cancer Therapy. Clin Cancer Res 16, 384-9.
Druker, B. J., Talpaz, M., Resta, D. J., Peng, B., Buchdunger, E., Ford, J. M., Lydon, N. B., Kantarjian, H., Capdeville, R., Ohno-Jones, S., and Sawyers, C. L. (2001). Efficacy and safety of a specific inhibitor of the BCR-ABL tyrosine kinase in chronic myeloid leukemia. N Engl J Med 344, 1031-1037.
Edgar, R., Domrachev, M., and Lash, A. E. (2002). Gene Expression Omnibus: NCBI gene expression and hybridization array data repository. Nucleic Acids Res 30, 207-210.
Fine, J. D., Johnson, L. B., Weiner, M., Li, K. P., and Suchindran, C. (2009). Epidermolysis bullosa and the risk of life-threatening cancers: the National EB Registry experience, 1986-2006. J Am Acad Dermatol 60, 203-211.
Fong, P. C., Boss, D. S., Yap, T. A., Tutt, A., Wu, P., Mergui-Roelvink, M., Mortimer, P., Swaisland, H., Lau, A., O'Connor, M. J., et al. (2009). Inhibition of poly(ADP-ribose) polymerase in tumors from BRCA mutation carriers. N Engl J Med 361, 123-134.
Gallegos Ruiz, M. I., Floor, K., Roepman, P., Rodriguez, J. A., Meijer, G. A., Mooi, W. J., Jassem, E., Niklinski, J., Muley, T., van Zandwijk, N., et al. (2008). Integration of gene dosage and gene expression in non-small cell lung cancer, identification of HSP90 as potential target. PLoS One 3, e0001722.
Giglia-Mari, G., and Sarasin, A. (2003). TP53 mutations in human skin cancers. Hum Mutat 21, 217-228.
Green, J., Ikram, M., Vyas, J., Patel, N., Proby, C. M., Ghali, L., Leigh, I. M., O'Toole, E. A., and Storey, A. (2006). Overexpression of the Axl tyrosine kinase receptor in cutaneous SCC-derived cell lines and tumors. Br J Cancer 94, 1446-1451.
Guan, R., Tapang, P., Leverson, J. D., Albert, D., Giranda, V. L., and Luo, Y. (2005). Small interfering RNA-mediated Polo-like kinase 1 depletion preferentially reduces the survival of p53-defective, oncogenic transformed cells and inhibits tumor growth in animals. Cancer Res 65, 2698-2704.
Haider, A. S., Peters, S. B., Kaporis, H., Cardinale, I., Fei, J., Ott, J., Blumenberg, M., Bowcock, A. M., Krueger, J. G., and Carucci, J. A. (2006). Genomic analysis defines a cancer-specific gene expression signature for human squamous cell carcinoma and distinguishes malignant hypeiproliferation from benign hyperplasia. J Invest Dermatol 126, 869-881.
Hanahan, D., and Weinberg, R. A. (2000). The hallmarks of cancer. Cell 100, 57-70.
Hayakawa, T., Ohtani, Y., Hayakawa, N., Shinmyozu, K., Saito, M., Ishikawa, F., and Nakayama, J. (2007). RBP2 is an MRG15 complex component and down-regulates intragenic histone H3 lysine 4 methylation. Genes Cells 12, 811-826.
Liu, X., Lei, M., and Erikson, R. L. (2006). Normal cells, but not cancer cells, survive severe Plk1 depletion. Mol Cell Biol 26, 2093-2108.
Livak, K. J., and Schmittgen, T. D. (2001). Analysis of relative gene expression data using real-time quantitative PCR and the 2(-Delta Delta C(T)) Method. Methods 25, 402-408.
Masters, J. R. (2000). Human cancer cell lines: fact and fantasy. Nat Rev Mol Cell Biol 1, 233-236.
Merlo, L. M., Pepper, J. W., Reid, B. J., and Maley, C. C. (2006). Cancer as an evolutionary and ecological process. Nat Rev Cancer 6, 924-935.
Michiels, S., Koscielny, S., and Hill, C. (2007). Interpretation of microarray data in cancer. Br J Cancer 96, 1155-1158.
Nindl, I., Dang, C., Forschner, T., Kuban, R. J., Meyer, T., Sterry, W., and Stockfleth, E. (2006). Identification of differentially expressed genes in cutaneous squamous cell carcinoma by microarray expression profiling. Mol Cancer 5, 30.
Perou, C. M., Jeffrey, S. S., van de Rijn, M., Rees, C. A., Eisen, M. B., Ross, D. T., Pergamenschikov, A., Williams, C. F., Zhu, S. X., Lee, J. C., et al. (1999). Distinctive gene expression patterns in human mammary epithelial cells and breast cancers. Proc Natl Acad Sci U S A 96, 9212-9217.
Piccart-Gebhart, M. J., Procter, M., Leyland-Jones, B., Goldhirsch, A., Untch, M., Smith, I., Gianni, L., Baselga, J., Bell, R., Jackisch, C., et al. (2005). Trastuzumab after adjuvant chemotherapy in HER2-positive breast cancer. N Engl J Med 353, 1659-1672.
Pourreyron, C., Cox, G., Mao, X., Volz, A., Baksh, N., Wong, T., Fassihi, H., Arita, K., O'Toole, E. A., Ocampo-Candiani, J., et al. (2007). Patients with recessive dystrophic epidermolysis bullosa develop squamous-cell carcinoma regardless of type VII collagen expression. J Invest Dermatol 127, 2438-2444.
Purdie, K. J., Lambert, S. R., Teh, M. T., Chaplin, T., Molloy, G., Raghavan, M., Kelsell, D. P., Leigh, I. M., Harwood, C. A., Proby, C. M., and Young, B. D. (2007). Allelic imbalances and microdeletions affecting the PTPRD gene in cutaneous squamous cell carcinomas detected using single nucleotide polymorphism microarray analysis. Genes Chromosomes Cancer 46, 661-669.
Rheinwald, J. G., and Beckett, M. A. (1981). Tumorigenic keratinocyte lines requiring anchorage and fibroblast support cultures from human squamous cell carcinomas. Cancer Res 41, 1657-1663.
Riker, A. I., Enkemann, S. A., Fodstad, O., Liu, S., Ren, S., Morris, C., Xi, Y., Howell, P., Metge, B., Samant, R. S., et al. (2008). The gene expression profiles of primary and metastatic melanoma yields a transition point of tumor progression and metastasis. BMC Med Genomics 1, 13.
Romanowska, M., Reilly, L., Palmer, C. N., Gustafsson, M. C., and Foerster, J. (2010). Activation of PPARbeta/delta causes a psoriasis-like skin disease in vivo. PLoS One 5, e9701.
Ross, D. T., Scherf, U., Eisen, M. B., Perou, C. M., Rees, C., Spellman, P., Iyer, V., Jeffrey, S. S., Van de Rijn, M., Waltham, M., et al. (2000). Systematic variation in gene expression patterns in human cancer cell lines. Nat Genet 24, 227-235.
Schmit, T. L., and Ahmad, N. (2007). Regulation of mitosis via mitotic kinases: new opportunities for cancer management. Mol Cancer Ther 6, 1920-1931.
Schmit, T. L., Zhong, W., Nihal, M., and Ahmad, N. (2009). Polo-like kinase 1 (Plkl) in non-melanoma skin cancers. Cell Cycle 8, 2697-2702.
Schoffski, P. (2009). Polo-like kinase (PLK) inhibitors in preclinical and early clinical development in oncology. Oncologist 14, 559-570.
Scotto, L., Narayan, G., Nandula, S. V., Arias-Pulido, H., Subramaniyam, S., Schneider, A., Kaufmann, A. M., Wright, J. D., Pothuri, B., Mansukhani, M., and Murty, V. V. (2008). Identification of copy number gain and overexpressed genes on chromosome arm 20q by an integrative genomic approach in cervical cancer: potential role in progression. Genes Chromosomes Cancer 47, 755-765.
Sharma, S. V., Haber, D. A., and Settleman, J. (2010). Cell line-based platforms to evaluate the therapeutic efficacy of candidate anticancer agents. Nat Rev Cancer 10, 241-253.
Shi, L., Jones, W. D., Jensen, R. V., Harris, S. C., Perkins, R. G., Goodsaid, F. M., Guo, L., Croner, L. J., Boysen, C., Fang, H., et al. (2008). The balance of reproducibility, sensitivity, and specificity of lists of differentially expressed genes in microarray studies. BMC Bioinformatics 9 Suppl 9, S10.
Shi, L., Reid, L. H., Jones, W. D., Shippy, R., Warrington, J. A., Baker, S. C., Collins, P. J., de Longueville, F., Kawasaki, E. S., Lee, K. Y., et al. (2006). The MicroArray Quality Control (MAQC) project shows inter- and intraplatform reproducibility of gene expression measurements. Nat Biotechnol 24, 1151-1161.
South, A. P., Wan, H., Stone, M. G., Dopping-Hepenstal, P. J., Purkis, P. E., Marshall, J. F., Leigh, I. M., Eady, R. A., Hart, I. R., and McGrath, J. A. (2003). Lack of plakophilin 1 increases keratinocyte migration and reduces desmosome stability. J Cell Sci 116, 3303-3314.
Steegmaier, M., Hoffmann, M., Baum, A., Lenart, P., Petronczki, M., Krssak, M., Gurtler, U., Garin-Chesa, P., Lieb, S., Quant, J., et al. (2007). BI 2536, a potent and selective inhibitor of polo-like kinase 1, inhibits tumor growth in vivo. Curr Biol 17, 316-322.
Takai, N., Hamanaka, R., Yoshimatsu, J., and Miyakawa, I. (2005). Polo-like kinases (Plks) and cancer. Oncogene 24, 287-291.
van de Vijver, M. J., He, Y. D., van't Veer, L. J., Dai, H., Hart, A. A., Voskuil, D. W., Schreiber, G. J., Peterse, J. L., Roberts, C., Marton, M. J., et al. (2002). A gene-expression signature as a predictor of survival in breast cancer. N Engl J Med 347, 1999-2009.
Van Haren, R., Feldman, D., and Sinha, A. A. (2009). Systematic comparison of nonmelanoma skin cancer microarray datasets reveals lack of consensus genes. Br J Dermatol 161, 1278-1287.
Wallis, S., Lloyd, S., Wise, I., Ireland, G., Fleming, T. P., and Garrod, D. (2000). The alpha isoform of protein kinase C is involved in signaling the response of desmosomes to wounding in cultured epithelial cells. Mol Biol Cell 11, 1077-1092.
Weaver, V. M., Petersen, O. W., Wang, F., Larabell, C. A., Briand, P., Damsky, C., and Bissell, M. J. (1997). Reversion of the malignant phenotype of human breast cells in three-dimensional culture and in vivo by integrin blocking antibodies. J Cell Biol 137, 231-245.
Welsh, J. B., Sapinoso, L. M., Su, A. I., Kern, S. G., Wang-Rodriguez, J., Moskaluk, C. A., Frierson, H. F., Jr., and Hampton, G. M. (2001). Analysis of gene expression identifies candidate markers and pharmacological targets in prostate cancer. Cancer Res 61, 5974-5978.
Wu, K., and Bonavida, B. (2009). The activated NF-kappaB-Snail-RKIP circuitry in cancer regulates both the metastatic cascade and resistance to apoptosis by cytotoxic drugs. Crit Rev Immunol 29, 241-254.
Yamaguchi, K., Sakai, M., Shimokawa, T., Yamada, Y., Nakamura, Y., and Furukawa, Y. (2010). C20orf20 (MRG-binding protein) as a potential therapeutic target for colorectal cancer. Br J Cancer 102, 325-331.

### Supplementary references

Nystrom, M. L., Thomas, G. J., Stone, M., Mackenzie, I. C., Hart, I. R., and Marshall, J. F. (2005). Development of a quantitative method to analyse tumour cell invasion in organotypic culture. J Pathol 205, 468-475.
Barrett, T., Troup, D. B., Wilhite, S. E., Ledoux, P., Rudnev, D., Evangelista, C., Kim, I. F., Soboleva, A., Tomashevsky, M., Marshall, K. A., et al. (2009). NCBI GEO: archive for high-throughput functional genomic data. Nucleic Acids Res 37, D885-890.

## Claims

1. A polynucleotide for use in the treatment and/or prevention of cutaneous squamous cell carcinoma (cSCC), wherein the polynucleotide encodes a sequence at least 65% identical to a sequence encoding Chromosome 20 open reading frame 20 (*c20orf20*) or a fragment thereof

2. The polynucleotide of claim 1 for the use of claim 1, wherein the polynucleotide is at least 65% identical to SEQ ID NO: 2 or a fragment thereof.

3. A polypeptide for use in the treatment and/or prevention of cutaneous squamous cell carcinoma (cSCC), wherein the polypeptide is a sequence at least 65% identical to a sequence encoding Chromosome 20 open reading frame 20 *(c20orf20)* or a fragment thereof

4. The polypeptide of claim 3 for the use of claim 3, wherein the polypeptide is at least 65% identical to SEQ ID NO: 7 or a fragment thereof.

5. A compound for use in treating cSCC, wherein said compound modulates the expression, function and/or activity of the chromosome 20 open reading frame 20 (*c20orf20*) gene and/or its protein product wherein the compound is an antisense, silencing and/or interfering nucleic acid; or wherein the compound is an antibody or an antigen/epitope binding fragment thereof, capable of binding to the chromosome 20 open reading frame 20 (*c20oif20*) protein or-a fragment thereof.

6. The compound of claim 5 for the use of claim 5, wherein the antisense silencing and/or interfering nucleic acid is one or more selected from the group consisting of:
(i) CUCAGAUAUUGAGGGCUCUdTdT;
(ii) AGAGCCCUCAAUAUCUGAGdTdT;
(iii) GGGACAAGUUCAGCCAGAAdTdT; and
(iv) UUCUGGCUGAACUUGUCCCdTdT.
said antisense nucleic acids being effective in modulating c20orf20 expression.

7. The compound of claim 5 for the use of claim 5, wherein the antibody or antigen binding fragment thereof, is specific or selective for one or more epitopes contained within a C20orf20 peptide selected from the group consisting of:
(a) CNPSSPSAAKRRRT
(b) GEAEVGGGGAAGDKGC
(c) CGKASEKSSKDKEKNSSD

8. A pharmaceutical composition comprising a polynucleotide for use according to claims 1 and 2 and/or polypeptide for use according to claims 3 and 4 and/or a compound for use according to any one of claims 5-7 together or in association with, a pharmaceutically acceptable excipient, carrier or diluent.

9. Use of oligonucleotide/polypeptide probes and/or primers capable of hybridising to all or part of SEQ ID NOS: 2 and/or 7 in the detection and/or diagnosis of cSCC.

10. A method of diagnosing cSCC or a predisposition or susceptibility thereto, said method comprising the steps of:
(a) providing a sample from a subject; and
(b) identifying a level of expression or activity in the sample, of the chromosome 20 open reading frame 20 (*c20orf20*) gene or its protein product or a fragment thereof;
wherein the detection of aberrant levels of expression/activity of the *c20orf20* gene its protein product or a fragment thereof, indicates that the subject is suffering from and/or susceptible/predisposed to cSCC.

11. The method of claim 10, wherein an oligonucleotide/polypeptide probe or primer capable of hybridising to all or part of SEQ ID NOS: 2 and/or 7 is used to identify a level of expression or activity of the genes and/or proteins in the sample.

12. Use of a kit comprising a substrate having (i) the chromosome 20 open reading frame 20 *(c20orf20)* protein or a fragment thereof bound thereto; or (ii) an antibody capable of binding to a chromosome 20 open reading frame 20 *(c20orf20)* protein or fragment thereof bound thereto; and
one or more components selected from the group consisting of:
(a) an antibody according to claim 7;
(b) one or more oligonucleotides/primers for detecting/amplifying/probing nucleic acid samples for aberrant or modulated c20orf20 expression, function and/or activity; and
(c) instructions for use;
in the diagnosis, detection or evaluation cSCC in a subject.

## Patentansprüche

1. Polynukleotid zur Verwendung in der Behandlung und/oder Verhütung von kutanem Plattenepithelkarzinom (kPEK), wobei das Polynukleotid für eine Sequenz codiert, die zu mindestens 65% identisch ist mit einer Sequenz, codierend für Chromosom-20-offener-Leserahmen-20 (*c20orf20*), oder einem Fragment davon.

2. Polynukleotid nach Anspruch 1 zur Verwendung nach Anspruch 1, wobei das Polynukleotid zu mindestens 65% identisch ist mit SEQ ID NO: 2 oder einem Fragment davon.

3. Polypeptid zur Verwendung in der Behandlung und/oder Verhütung von kutanem Plattenepithelkarzinom (kPEK), wobei das Polypeptid eine Sequenz ist, die zu mindestens 65% identisch ist mit einer Sequenz, codierend für Chromosom-20-offener-Leserahmen-*20* (*c20orf20*), oder einem Fragment davon.

4. Polypeptid nach Anspruch 3 zur Verwendung nach Anspruch 3, wobei das Polypeptid zu mindestens 65% identisch ist mit SEQ ID NO: 7 oder einem Fragment davon.

5. Verbindung zur Verwendung bei Behandlung von kPEK, wobei die Verbindung die Expression, Funktion und/oder Aktivität des Chromosom-20-offener-Leserahmen-20-(*c20orf20*-) Gens und/oder seines Proteinprodukts moduliert, wobei die Verbindung eine Antisense-, Silencing- und/oder Interferenz-Nukleinsäure ist; oder wobei die Verbindung ein Antikörper oder ein Antigen/Epitop bindendes Fragment davon ist, in der Lage zum Binden an das Chromosom-20-offener-Leserahmen-20-(*c20orf20*-) Protein oder ein Fragment davon.

6. Verbindung nach Anspruch 5 zur Verwendung nach Anspruch 5, wobei die Antisense-Silencing- und/oder -Interferenz-Nukleinsäure eine oder mehrere ist bzw. sind, ausgewählt aus der Gruppe, bestehend aus:
(i) CUCAGAUAUUGAGGGCUCUdTdT;
(ii) AGAGCCCUCAAUAUCUGAGdTdT;
(iii) GGGACAAGUUCAGCCAGAAdTdT; und
(iv) UUCUGGCUGAACUUGUCCCdTdT.
wobei die Antisense-Nukleinsäuren beim Modulieren von *c20orf20*-Expression effektiv sind.

7. Verbindung nach Anspruch 5 zur Verwendung nach Anspruch 5, wobei der Antikörper, oder das Antigen bindende Fragment davon, spezifisch oder selektiv ist für ein oder mehrere Epitope, enthalten innerhalb eines *c20orf20*-Peptids, ausgewählt aus der Gruppe, bestehend aus:
(a) CNPSSPSAAKRRRT
(b) GEAEVGGGGAAGDKGC
(c) CGKASEKSSKDKEKNSSD

8. Pharmazeutische Zusammensetzung, umfassend ein Polynukleotid zur Verwendung nach Ansprüchen 1 und 2 und/oder ein Polypeptid zur Verwendung nach Ansprüchen 3 und 4 und/oder eine Verbindung zur Verwendung nach einem der Ansprüche 5-7 gemeinsam, oder in Verbindung mit, einem pharmazeutisch verträglichen Exzipienten, Träger oder Verdünnungsmittel.

9. Verwendung von Oligonukleotid/Polypeptid-Sonden und/oder -Primern, die zum Hybridisieren an alles oder einen Teil von SEQ ID NOS: 2 und/oder 7 in der Lage sind, bei dem Nachweis und/oder der Diagnose von kPEK.

10. Verfahren zur Diagnose von kPEK oder einer Veranlagung oder Anfälligkeit dafür, wobei das Verfahren die Schritte umfasst:
(a) Bereitstellen einer Probe aus einem Individuum; und
(b) Identifizieren eines Niveaus der Expression oder Aktivität in der Probe, des Chromosom-20-offener-Leserahmen-20-(*c20orf20*-) Gens oder seines Proteinprodukts oder eines Fragments davon;
wobei der Nachweis aberranter Niveaus der Expression/Aktivität des *c20orf20-*Gens, seines Proteinprodukts oder eines Fragments davon anzeigt, dass das Individuum an kPEK leidet und/oder dafür anfällig/veranlagt ist.

11. Verfahren nach Anspruch 10, wobei ein(e) Oligonukleotid/Polypeptid-Sonde oder -Primer, in der Lage zum Hybridisieren an alles oder einen Teil von SEQ ID NOS: 2 und/oder 7, verwendet wird, um ein Niveau der Expression oder Aktivität der Gene und/oder Proteine in der Probe zu identifizieren.

12. Verwendung eines Kits, umfassend ein Substrat mit (i) dem Chromosom-20-offener-Leserahmen-20- (*c20orf20*-) Protein oder einem Fragment davon an sich gebunden; oder (ii) einem Antikörper, der zum Binden an ein Chromosom-20-offener-Leserahmen-20- (*c20orf20-*) Protein oder ein Fragment davon in der Lage ist, an sich gebunden; und
eine oder mehrere Komponenten, ausgewählt aus der Gruppe, bestehend aus:
(a) einem Antikörper nach Anspruch 7;
(b) einem oder mehreren Oligonukleotiden/Primern zum Nachweisen/Amplifizieren/Sondieren von Nukleinsäuresonden auf aberrante oder modulierte *c20orf20*-Expression, -Funktion und/oder -Aktivität; und
(c) Anweisungen zur Anwendung;
bei der Diagnose, dem Nachweis oder der Evaluierung von kPEK bei einem Individuum.

## Revendications

1. Polynucléotide à utiliser dans le traitement et/ou la prévention de carcinome cutané à cellules squameuses (cSCC), où le polynucléotide code une séquence identique à 65 % au moins à une séquence codant le cadre de lecture ouvert 20 du chromosome 20 (*c20orf20*) ou un fragment de celui-ci.

2. Polynucléotide selon la revendication 1 à utiliser selon la revendication 1, où le polynucléotide est identique à 65 % au moins à la SEQ ID NO: 2 ou à un fragment de celle-ci.

3. Polypeptide à utiliser dans le traitement et/ou la prévention de carcinome cutané à cellules squameuses (cSCC), où le polypeptide est une séquence identique à 65 % au moins à une séquence codant le cadre de lecture ouvert 20 du chromosome 20 (*c20orf20*) ou un fragment de celui-ci.

4. Polypeptide selon la revendication 3 à utiliser selon la revendication 3, où le polypeptide est identique à 65 % au moins à la SEQ ID NO: 7 ou à un fragment de celle-ci.

5. Composé à utiliser dans le traitement de cSCC, où ledit composé module l'expression, la fonction et/ou l'activité du gène du cadre de lecture 20 du chromosome 20 (*c20orf20*) et/ou de son produit protéique, où le composé est un acide nucléique anti-sens, de blocage et/ou interférent; ou bien où le composé est un anticorps ou un fragment de liaison à un antigène/épitope de celui-ci, capable de se lier à la protéine du cadre de lecture 20 du chromosome 20 (*c20orf20*) ou à un fragment de celle-ci.

6. Composé selon la revendication 5 à utiliser selon la revendication 5, où l'acide nucléique anti-sens, de blocage et/ou interférent en est un ou plusieurs sélectionnés parmi le groupe consistant en:
(i) CUCAGAUAUUGAGGGCUCUdTdT;
(ii) AGAGCCCUCAAUAUCUGAGdTdT;
(iii) GGGACAAGUUCAGCCAGAAdTdT; et
(iv) UUCUGGCUGAACUUGUCCCdTdT.
lesdits acides nucléiques anti-sens étant efficaces pour moduler l'expression de c20orf20.

7. Composé selon la revendication 5 à utiliser selon la revendication 5, dans lequel l'anticorps ou le fragment de liaison à un antigène de celui-ci, est spécifique ou sélectif pour un ou plusieurs épitopes contenus dans un peptide c20orf20 sélectionné parmi le groupe consistant en:
(a) CNPSSPSAAKRRRT
(b) GEAEVGGGGAAGDKGC
(c) CGKASEKSSKDKEKNSSD

8. Composition pharmaceutique comprenant un polynucléotide à utiliser selon les revendications 1 et 2 et/ou un polypeptide à utiliser selon les revendications 3 et 4 et/ou un composé à utiliser selon l'une quelconque des revendications 5-7 avec ou en association avec un excipient, un véhicule ou un diluant pharmaceutiquement acceptable.

9. Utilisation de sondes et/ou d'amorces oligonucléotidiques/polypeptidiques capables de s'hybrider à toutes ou à une partie des SEQ ID NO: 2 et/ou 7 dans la détection et/ou le diagnostic de cSCC.

10. Procédé de diagnostic de cSCC ou d'une prédisposition ou susceptibilité à celui-ci, ledit procédé comprenant les étapes consistant à:
(a) fournir un échantillon d'un sujet; et
(b) identifier dans l'échantillon un niveau d'expression ou d'activité du gène du cadre de lecture 20 du chromosome 20 (*c20orf20*) et/ou de son produit protéique ou d'un fragment de celui-ci;
dans lequel la détection de niveaux aberrants d'expression/d'activité du gène *c20orf20,* de son produit protéique ou d'un fragment de celui-ci, indique que le sujet souffre et/ou est susceptible de souffrir de/est prédisposé au cSCC.

11. Procédé selon la revendication 10, dans lequel une sonde ou une amorce oligonucléotidique/polypeptidique capable de s'hybrider à toutes ou à une partie des SEQ ID NO: 2 et/ou 7, est utilisée pour identifier un niveau d'expression ou d'activité des gènes et/ou des protéines dans l'échantillon.

12. Utilisation d'un kit comprenant un substrat ayant (i) une protéine du cadre de lecture ouvert 20 du chromosome 20 (*c20orf20*) ou un fragment de celle-ci lié à celui-ci; ou bien (ii) un anticorps capable de se lier à une protéine du cadre de lecture ouvert 20 du chromosome 20 (*c20orf20*) ou à un fragment de celle-ci lié à celui-ci; et
un ou plusieurs composants sélectionnés parmi le groupe consistant en:
(a) un anticorps selon la revendication 7;
(b) un ou plusieurs oligonucléotides/amorces pour détecter/amplifier/sonder des échantillons d'acide nucléiques pour l'expression, la fonction et/ou l'activité aberrante ou modulée de c20orf20; et
(c) des instructions d'emploi;
dans le diagnostic, la détection ou l'évaluation de cSCC chez un sujet.
